# EUROPEAN PATENT APPLICATION

(11) **EP 4 851 176 A1**
(43) Date of publication of application: **09.12.2026**
(21) Application number: 25747673.9
(22) Date of filing: 22.01.2025
(51) Int. Cl.: C07J 63/00, A61K 31/58, A61K 31/56, A61P 25/08, A61P 25/14, A61P 9/04, A61P 13/12, A61P 29/00, A61P 25/00, A61P 9/10, A61P 25/18, A61P 21/00, A61P 25/16, A61P 25/28

(54) **C17-SITE NITROGEN-SUBSTITUTED AND METHYLENE-SUBSTITUTED OLEANANE TRITERPENE DERIVATIVE, PREPARATION METHOD THEREFOR AND USE THEREOF**

(30) Priority: 29.01.2024 CN 202410123583; 09.01.2025 CN 202510036037
(71) Applicant: Yantai University, Yantai, Shandong 264005 (CN)
(72) Inventor: TIAN, Jingwei, Yantai, Shandong 264005 (CN); FU, Fenghua, Yantai, Shandong 264005 (CN); WANG, Hongbo, Yantai, Shandong 264005 (CN); ZOU, Fangxia, Yantai, Shandong 264005 (CN); LI, Chunmei, Yantai, Shandong 264005 (CN)
(74) Representative: Valet Patent Services Limited
(86) International application number: PCT/CN2025/073801
(87) International publication number: WO 2025/162101

(57) **Abstract**

A C17-site nitrogen-substituted and methylene-substituted oleanane triterpene derivative, a preparation method therefor, and a use thereof. Provided are a C17-site nitrogen-substituted and methylene-substituted oleanane triterpene derivative, a use of the compound in the preparation of an NRF2-Leap1 decoupling agent, and a use of the compound in the preparation of a medicament for preventing and/or treating diseases in a patient, the diseases comprising cerebral small vessel disease, mitochondrial encephalomyopathy, autism spectrum disorder, Rett syndrome, Friedreich ataxia, stroke, hemorrhagic cerebral apoplexy, ischemic cerebral apoplexy, multiple sclerosis, amyotrophic lateral sclerosis, schizophrenia, cognitive impairment in schizophrenia, Parkinson's disease, cognitive impairment in Parkinson's disease, Alzheimer's disease, vascular dementia, epilepsy, Huntington's disease, heart failure, myocardial infarction, renal failure, kidney ischemia, etc.

## Description

### TECHNICAL FIELD

The present application relates to the fields of biology and medical technology, and specifically to C17-site nitrogen-substituted and methylene-substituted oleanane triterpene derivatives, as well as preparation methods therefor and uses thereof.

### BACKGROUND ART

Current research indicates that the Keap-Nrf2-ARE pathway has a protective effect on cells and serves as one of the important channels for the body to maintain redox balance and eliminate damage caused by toxic and harmful substances. It plays a crucial role in anti-stress, anti-apoptosis, anti-inflammatory responses, and neuroprotection. Reactive oxygen species (ROS) and oxidative stress are associated with the occurrence of many diseases through this pathway, making it a research hotspot in recent years. Currently, the structures of Nrf2 agonists are mostly derived from the oleanolic acid triterpene core, such as 2-cyano-3,12-dioxooleana-1,9(11)-dien-28-oic acid (CDDO), methyl bardoxolone (CDDO-Me; RTA 402), and omaveloxolone (RTA 408). However, these molecules primarily focused on the treatment of inflammation through their Nrf2-activating action and are characterized by poor inhibitory activity and low specificity. Therefore, the development of novel Nrf2-Leap1 uncouplers possessing both anti-inflammatory and antioxidant effects is particularly important.

### SUMMARY OF THE INVENTION

To solve the above technical problems, the present application provides C17-site nitrogen-substituted and methylene-substituted oleanane triterpene derivatives, as well as preparation methods therefor and uses thereof.

The present application provides a compound of Formula (I), or a pharmaceutically acceptable salt thereof: 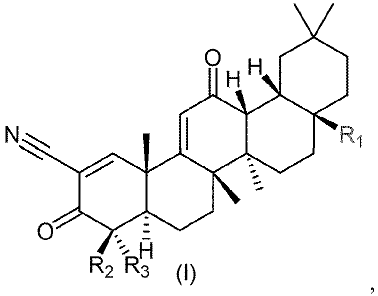 wherein R₁ is independently selected from the group consisting of: -NH-heteroaryl, -NH-heteroaryldiyl-R₁', -NH-C(=O)-alkyl, -NH-C(=O)-substituted alkyl, -NH-C(=O)-alkenyl, -NH-C(=O)-substituted alkenyl, -NH-C(=O)-alkynyl, -NH-C(=O)-substituted alkynyl, -NH-C(=O)-aryl, -NH-C(=O)-aryldiyl-R₁', -NH-C(=O)-heteroaryl, -NH-C(=O)-heteroaryldiyl-R₁', -N(OH)-C(=O)-alkyl, -N(OH)-C(=O)-substituted alkyl, -N(OH)-C(=O)-alkenyl, -N(OH)-C(=O)-substituted alkenyl, -N(OH)-C(=O)-alkynyl, -N(OH)-C(=O)-substituted alkynyl, -N(OH)-C(=O)-aryl, -N(OH)-C(=O)-aryldiyl-R₁', -N(OH)-C(=O)-heteroaryl, -N(OH)-C(=O)-heteroaryldiyl-R₁', -NH-C(=O)-L-amino acid-NH-heteroaryl, -NH-C(=O)-L-amino acid-NH-heteroaryldiyl-R₁', - CH₂-N(OH)-C(=O)-alkyl, -CH₂-N(OH)-C(=O)-substituted alkyl, -CH₂-N(OH)-C(=O)-alkenyl, - CH₂-N(OH)-C(=O)-substituted alkenyl, -CH₂-N(OH)-C(=O)-alkynyl, -CH₂-N(OH)-C(=O)-substituted alkynyl, -CH₂-N(OH)-C(=O)-heteroaryl, and -CH₂-N(OH)-C(=O)-heteroaryldiyl-R₁'; R₂ is hydrogen or methyl; and R₃ is hydrogen or methyl.

Preferably, the compound is further defined as follows: 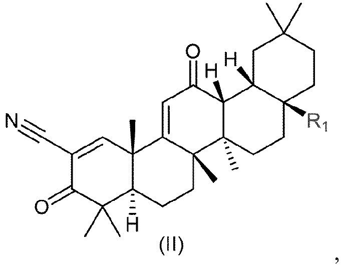 wherein R₁ is independently selected from the group consisting of -NH-heteroaryl, -NH-heteroaryldiyl-R₁', -NH-C(=O)-alkyl, -NH-C(=O)-substituted alkyl, -NH-C(=O)-alkenyl, -NH-C(=O)-substituted alkenyl, -NH-C(=O)-alkynyl, -NH-C(=O)-substituted alkynyl, -NH-C(=O)-aryl, -NH-C(=O)-aryldiyl-R₁', -NH-C(=O)-heteroaryl, -NH-C(=O)-heteroaryldiyl-R₁', -N(OH)-C(=O)-alkyl, -N(OH)-C(=O)-substituted alkyl, -N(OH)-C(=O)-alkenyl, -N(OH)-C(=O)-substituted alkenyl, -N(OH)-C(=O)-alkynyl, -N(OH)-C(=O)-substituted alkynyl, -N(OH)-C(=O)-aryl, -N(OH)-C(=O)-aryldiyl-R₁', -N(OH)-C(=O)-heteroaryl, -N(OH)-C(=O)-heteroaryldiyl-R₁', -NH-C(=O)-L-amino acid-NH-heteroaryl, -NH-C(=O)-L-amino acid-NH-heteroaryldiyl-R₁', - CH₂-N(OH)-C(=O)-alkyl, -CH₂-N(OH)-C(=O)-substituted alkyl, -CH₂-N(OH)-C(=O)-alkenyl, - CH₂-N(OH)-C(=O)-substituted alkenyl, -CH₂-N(OH)-C(=O)-alkynyl, -CH₂-N(OH)-C(=O)-substituted alkynyl, -CH₂-N(OH)-C(=O)-heteroaryl, and -CH₂-N(OH)-C(=O)-heteroaryldiyl-R₁'; R₂ is methyl; and R₃ is methyl.

Preferably, the R₁' in aromatic compound is -Cl, -F, -Br, -OH, isopropyl, linear or branched alkyl (C≤6), linear or branched alkyl (C≤6) substituted with 1 to 5 halogens, -OH, linear or branched alkyl (C≤6) substituted with 1 to 5 -OH groups, linear or branched alkenyl (C≤6), linear or branched alkenyl (C≤6) substituted with 1 to 5 halogens, linear or branched alkenyl (C≤6) substituted with 1 to 5 -OH groups, linear or branched alkynyl (C≤6), linear or branched alkynyl (C≤6) substituted with 1 to 5 halogens, linear or branched alkynyl (C≤6) substituted with 1 to 5 - OH groups, 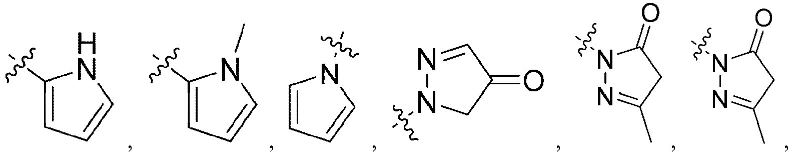 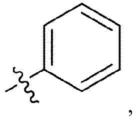 etc.

Preferably, the aryl group is selected from the group consisting of

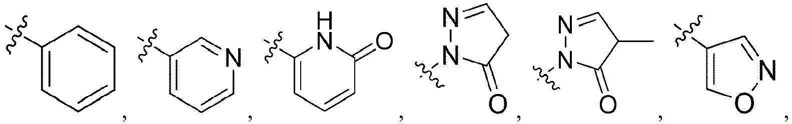

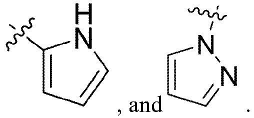

Preferably, the carbon chain length of each of the substituted alkyl, substituted alkenyl, substituted alkynyl, alkyl, alkenyl and alkynyl is less than or equal to 6, and each is independently linear, branched or cyclic. Substituents are selected from 1 to 5 -SO₃H, -OH, -F, -Br, -Cl, methyl, ethyl, and/or propyl groups.

Preferably, the compound, a pharmaceutically acceptable salt thereof, or a stereoisomer thereof is selected from the group consisting of:

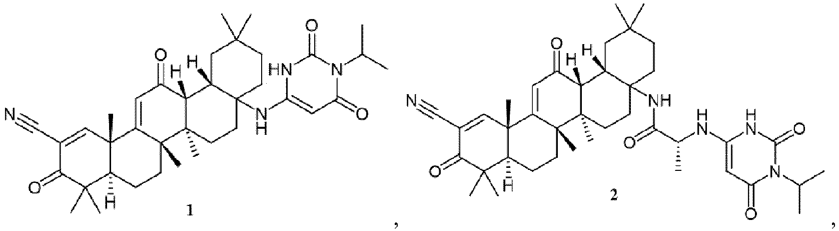

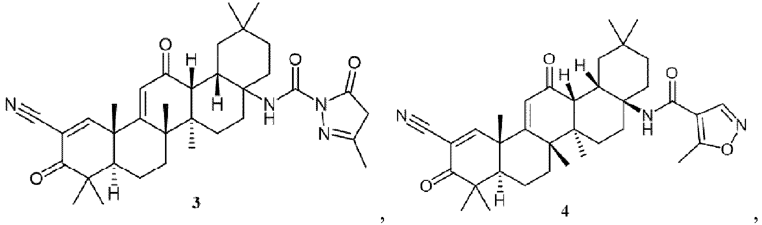

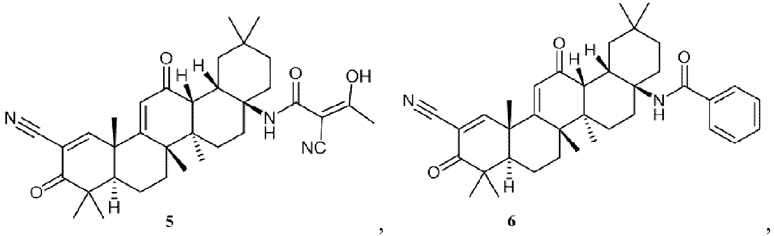

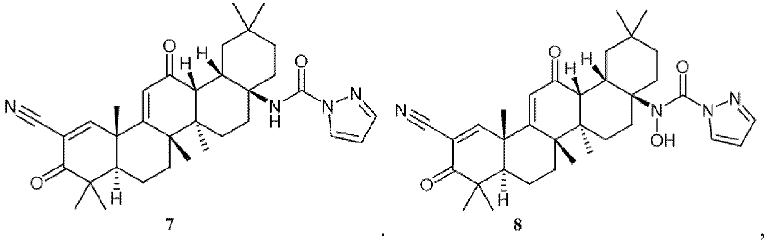

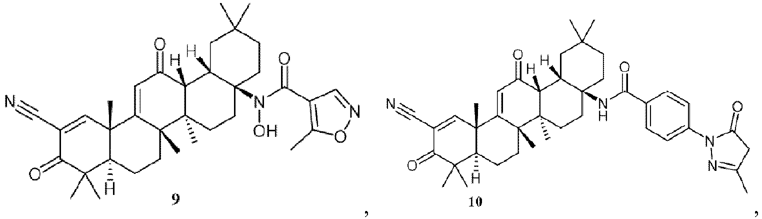

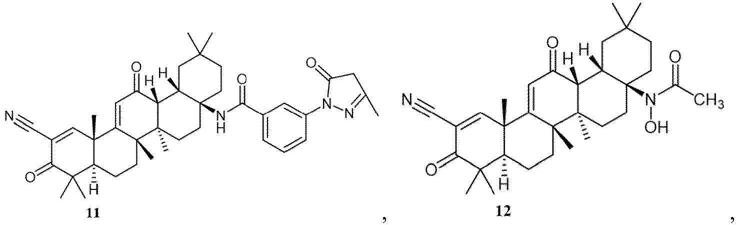

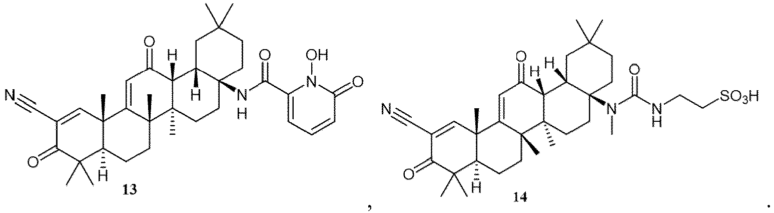

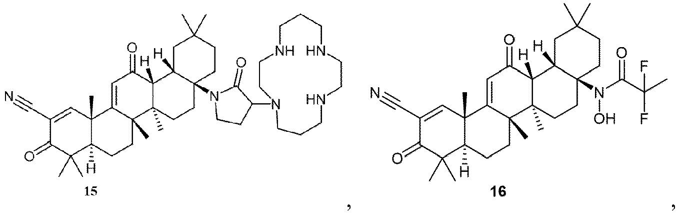

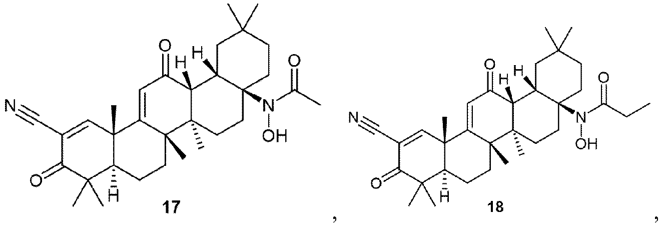

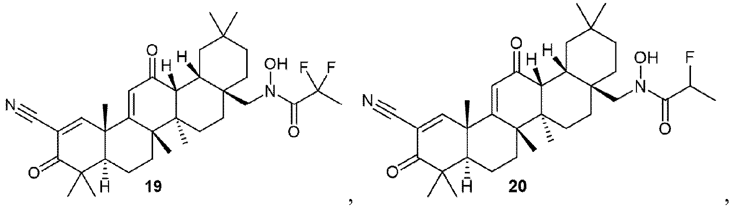

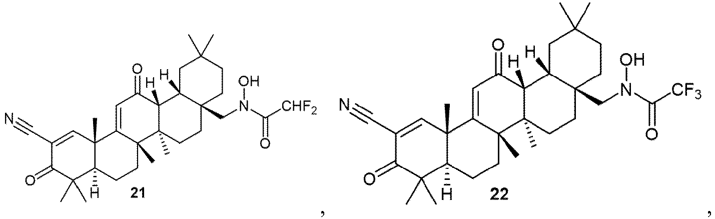

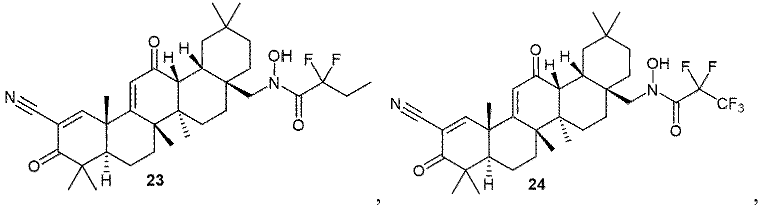

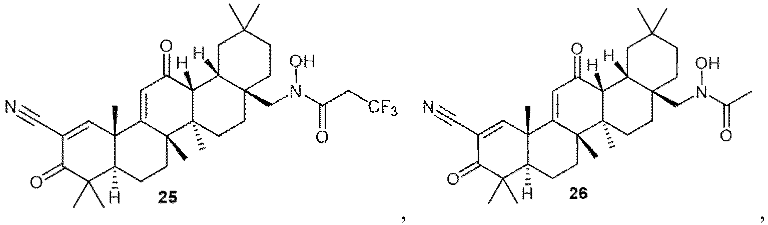

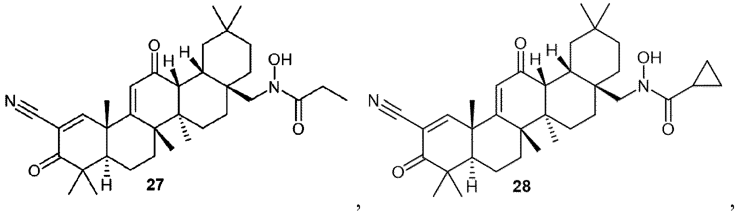

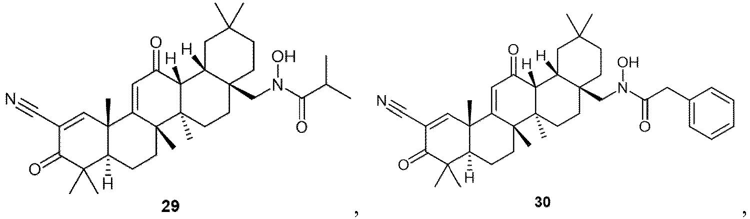

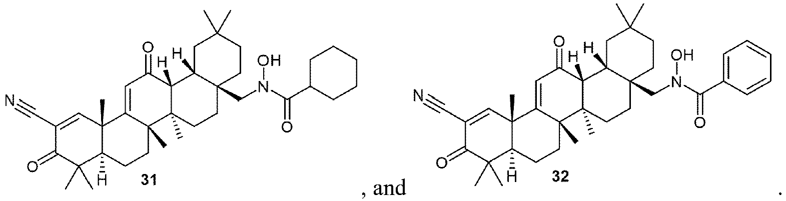

The present application further provides the use of the above-mentioned compounds, pharmaceutically acceptable salts thereof, or stereoisomers thereof in the preparation of an NRF2-Leap1 uncoupler. Biological experiments of the present application show that many of the above-mentioned compounds exhibit single-digit nanomolar human Nrf2 receptor agonist activity, which is significantly superior to the marketed Nrf2 agonist Omaveloxolone. Simultaneously, some preferred compounds also possess activities such as scavenging DPPH free radicals, inhibiting the production of lipid peroxide MDA, and intervening in ferroptosis, thereby exerting antioxidant effects.

The present application further provides the use of the above-mentioned compounds, pharmaceutically acceptable salts thereof, or stereoisomers thereof in the preparation of a medicament for preventing and/or treating diseases in a patient. The medicament is used to prevent or treat diseases including cerebral small vessel disease, mitochondrial encephalomyopathy, autism spectrum disorder, Rett syndrome, Friedreich ataxia, stroke, hemorrhagic stroke, ischemic stroke, multiple sclerosis, amyotrophic lateral sclerosis, schizophrenia, cognitive impairment in schizophrenia, Parkinson's disease, cognitive impairment in Parkinson's disease, Alzheimer's disease, vascular dementia, epilepsy, Huntington's chorea, heart failure, myocardial infarction, renal failure, renal ischemia, etc. Effective experimental data demonstrate that the medicament prepared from the aforementioned compounds, their pharmaceutically acceptable salts, and stereoisomers of the present application exhibits significant therapeutic effects, particularly for stroke, multiple sclerosis, and amyotrophic lateral sclerosis.

Preferably, the use of the compounds, pharmaceutically acceptable salts thereof, and stereoisomers thereof in the preparation of a medicament for preventing or treating stroke, multiple sclerosis, and amyotrophic lateral sclerosis.

### BENEFICIAL EFFECTS

The present application synthesizes for the first time the compound of Formula (I) or pharmaceutically acceptable salts thereof, namely C17-site nitrogen-substituted and methylene-substituted oleanane triterpene derivatives. Meanwhile, the present application provides methods for preparing the compounds of Formula (I) via specific examples. Furthermore, the present application provides the use of the compounds of Formula (I) or pharmaceutically acceptable salts thereof for preparing Nrf2-Keap1 uncouplers. The C17-site nitrogen-substituted and methylene-substituted oleanane triterpene derivatives of the present application, as a novel class of Nrf2-Leap1 uncouplers, not only maintain strong agonist activity at the Nrf2 target but also possess activities such as scavenging DPPH free radicals, inhibiting the production of lipid peroxide MDA, or intervening in ferroptosis to exert antioxidant effects. Finally, the present application also provides a pharmaceutical composition comprising any one of the above-mentioned compounds or pharmaceutically acceptable salts thereof, together with a pharmaceutically acceptable carrier. The pharmaceutical composition can be prepared into various pharmaceutically acceptable dosage forms, such as tablets, capsules, oral liquids, granules, injections, or various sustained and controlled release formulations. The pharmaceutical composition can be administered orally or parenterally (e.g., intravenously, subcutaneously, or topically). The dosage can be appropriately adjusted according to the patient's age, gender, and disease type. The present application can be used in the future for preventing or treating a variety of diseases, including cerebral small vessel disease, mitochondrial encephalomyopathy, autism spectrum disorder, Rett syndrome, Friedreich ataxia, stroke, hemorrhagic stroke, ischemic stroke, multiple sclerosis, amyotrophic lateral sclerosis, schizophrenia, cognitive impairment in schizophrenia, Parkinson's disease, cognitive impairment in Parkinson's disease, Alzheimer's disease, vascular dementia, epilepsy, Huntington's chorea, heart failure, myocardial infarction, renal failure, renal ischemia, etc.

### BRIEF DESCRIPTION OF THE DRAWINGS

Figure 1 shows the effect of Compound 28 on the mNSS score in rats with ischemic stroke. Compared with the sham group: ^{###}*p* < 0.001; compared with the model group: ****p* < 0.001.
Figure 2 shows the effect of Compound 28 on the area of cerebral infarction in rats with ischemic stroke. Compared with the sham group: ^{###}*p* < 0.001; compared with the model group: ****p* < 0.001.
Figure 3 shows the effect of Compound 28 on the neurological function scores in EAE model mice. Compared with the sham group: ****p* < 0.001; compared with the model group: ^{#}*p* < 0.05, ^{##}*p* < 0.01, ^{###}*p* < 0.001; compared with the Omaveloxolone 15 mg/kg group: *^{&&&}p* < 0.001.
Figure 4 shows the effect of Compound 28 on body weight in EAE model mice. Compared with the sham group: ****p* < 0.001; compared with the model group: ^{#}*p* < 0.05, ^{###}*p* < 0.001; compared with the Omaveloxolone 15 mg/kg group: ^{&&&}*p* < 0.001.
Figure 5 shows the effect of Compound 28 on the onset time in SOD1 G93A mice. Compared with the control group: ****p* < 0.001; compared with the model group: ^{#}*p* < 0.05, ^{##}*p* < 0.01; compared with the Omaveloxolone 3 mg/kg group: ^{&}*p* < 0.05.
Figure 6 shows the effect of Compound 28 on motor coordination in SOD1 G93A mice. Compared with the control group: **p* < 0.05, ****p* < 0.001; compared with the model group: ^{###}*p* < 0.001; compared with the Omaveloxolone 3 mg/kg group: ^{&}*p* < 0.05.
Figure 7 shows the effect of Compound 28 on muscle endurance in SOD1 G93A mice; compared with the control group: **p* < 0.05, ****p* < 0.001; compared with the model group: ^{###}*p* < 0.001; compared with the Omaveloxolone 3 mg/kg group: ^{&}*p* < 0.05.

### DETAILED DESCRIPTION OF PREFERRED EMBODIMENTS

The present application will be further described below with reference to specific examples and experimental examples, but it is not intended to limit the scope of the present application in any way.

The structures of the compounds of the present application were determined by Nuclear Magnetic Resonance (NMR) or/and Liquid Chromatography-Mass Spectrometry (LC-MS).

NMR chemical shifts (δ) are reported in parts per million (ppm). NMR measurements were performed using an AVANCE III 600 NMR spectrometer, with deuterated dimethyl sulfoxide (DMSO-d₆), deuterated methanol (CD₃OD), and deuterated chloroform (CDCl₃) as solvents, and tetramethylsilane (TMS) as an internal standard.

Liquid chromatography-mass spectrometry (LC-MS) measurements were performed using a Shimadzu LCMS2020 mass spectrometer. HPLC measurements were performed using a Shimadzu LC20A liquid chromatograph.

The silica gel plates used for thin-layer chromatography were manufactured by Yantai Jiangyou. The specification used for TLC is 0.2 mm ± 0.03 mm, while the specification used for thin-layer chromatographic separation and purification of products is 0.4 mm - 0.5 mm.

Unless otherwise specified in the examples, the SFC separation conditions: column model: DAICL CHIRALPAK IC (250mm*30mm, 10µm); mobile phase: [CO₂-*i*-PrOH/ACN]; B%: 40%, isocratic elution mode.

In the present application, if specific experimental conditions are not indicated, conventional experimental conditions or conditions recommended by the manufacturer are followed. If the reagents or instruments used do not have a specified manufacturer, they are commercially available conventional products.

In the present application, the experimental results are expressed as mean values.

The detection indicators in the present application include the human Nrf2 receptor functional assay (agonist detection), the DPPH free radical scavenging capacity test, and the MDA anti-lipid peroxidation capacity test.

### Example 1

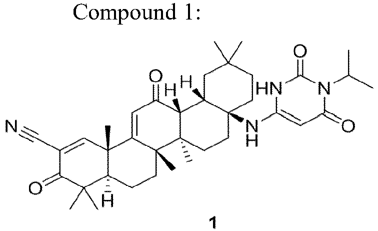

### Synthetic Route:

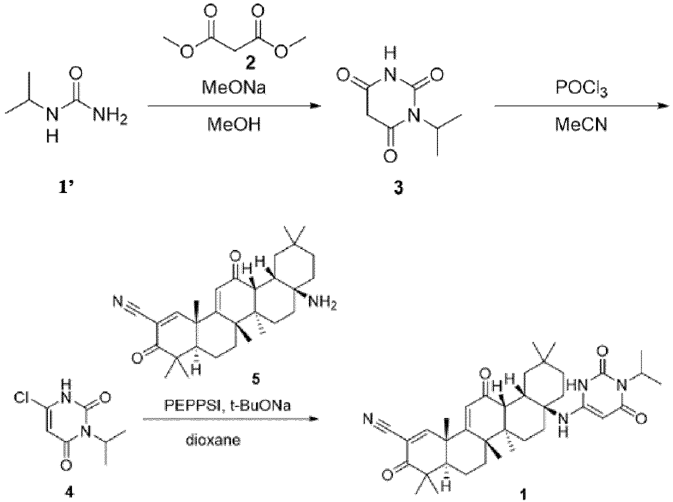

### Step 1: Synthesis of Intermediate 3

Starting material **1'** (25g, 244.78mmol) was dissolved in anhydrous methanol (400mL), and then Intermediate **2** (35.57g, 269.25mmol, 30.85mL) and sodium methoxide methanol solution (5.4M, 113.32mL) were added. The mixture was stirred at 70°C for 12h. After the reaction was complete as determined by LCMS, concentrated hydrochloric acid was added to the reaction mixture at 0°C to adjust the pH to below 7, followed by concentration under reduced pressure to obtain a residue. The residue was purified by column chromatography (silica gel column, petroleum ether/ethyl acetate = 1/0 to 7/3) to obtain Intermediate **3** (31g, yield 70.70%, yellow oil). LCMS: rt = 0.100 min, 169.2 [M-H]⁺; ¹H NMR (400 MHz, DMSO-*d6*) δ = 11.18 (br s, 1H), 4.89 - 4.76 (m, 1H), 3.56 (s, 2H), 1.31 (d, *J* = 7.0 Hz, 6H).

### Step 2: Synthesis of Intermediate 4

Intermediate **3** (31 g, 182.18 mmol) was dissolved in anhydrous acetonitrile (310 mL). Under nitrogen protection, phosphorus oxychloride (30.73 g, 200.39 mmol, 18.68 mL) was added, and the mixture was stirred at 65 °C for 12 h. After the reaction was complete as determined by LCMS, the reaction mixture was quenched by adding to room-temperature water (300 mL) at room temperature, stirred for 1 h, concentrated and filtered to obtain Intermediate **4** (21.4 g, yield 62.28%, yellow solid). ¹H NMR (400 MHz, DMSO-*d6*) δ = 12.20 (br s, 1H), 5.82 (s, 1H), 5.05 - 4.83 (m, 1H), 1.34 (d, *J* = 7.0 Hz, 6H).

### Step 3: Synthesis of Compound 1

Intermediate **5** (50 mg, 108.07 µmol) and Intermediate **4** (30.57 mg, 162.10 µmol) were dissolved in dioxane (1 mL). After purging with nitrogen three times, PEPPSI Pd (6.36 mg, 7.56 µmol) and sodium tert-butoxide (31.16 mg, 324.21 µmol) were added. After purging with nitrogen three times again, the mixture was stirred at 100 °C for 12 h. Then, PEPPSI Pd (6.36 mg, 7.56 µmol) and sodium tert-butoxide (31.16 mg, 324.21 µmol) were added again. After purging with nitrogen three times, the mixture was stirred at 100 °C for 2 h. After the reaction was complete as determined by LCMS, the reaction mixture was filtered under reduced pressure and concentrated to obtain a crude product. The crude product was separated on a thin-layer chromatography plate (dichloromethane/anhydrous methanol = 10/1), then further purified by preparative chromatography (C₁₈ column, 0.1% formic acid solution), and lyophilized to obtain Compound **1** (4.2 mg, white solid, yield <1%). LCMS: rt = 1.502 min, 615.4 [M+H]⁺; purity: 97.48%; ¹H NMR (400 MHz, DMSO-d6) δ = 9.50 (br s, 1H), 8.66 (s, 1H), 6.26 (s, 1H), 5.80 (br s, 1H), 5.01 - 4.83 (m, 1H), 4.65 (s, 1H), 3.07 (br d, *J* = 3.8 Hz, 1H), 2.08 - 1.91 (m, 2H), 1.90 - 1.82 (m, 2H), 1.81 - 1.64 (m, 4H), 1.61 - 1.53 (m, 1H), 1.49 - 1.42 (m, 5H), 1.39 (s, 3H), 1.30 (d, J = 7.0 Hz, 7H), 1.23 (br s, 3H), 1.18 (s, 3H), 1.11 - 1.04 (m, 4H), 0.96 (br d, J = 2.4 Hz, 6H), 0.89 (s, 3H).

### Example 2

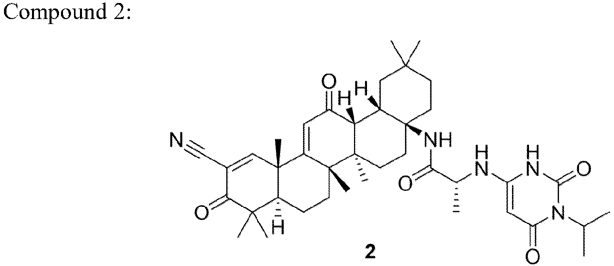

### Synthetic Route:

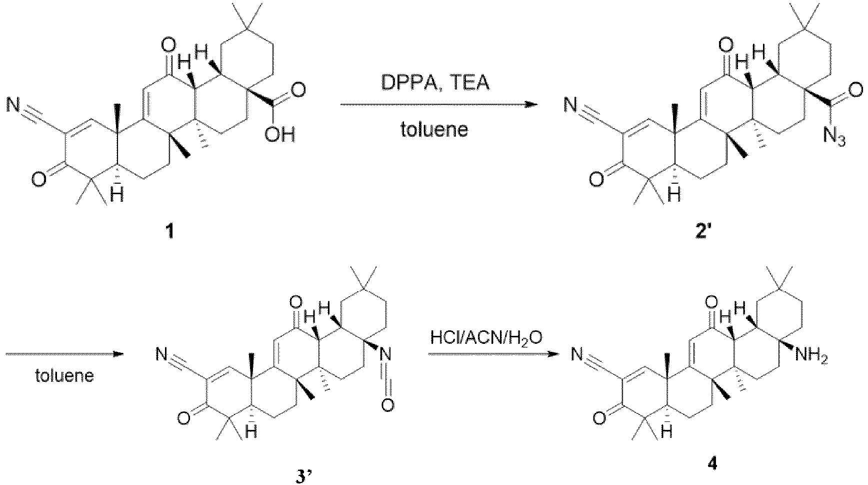

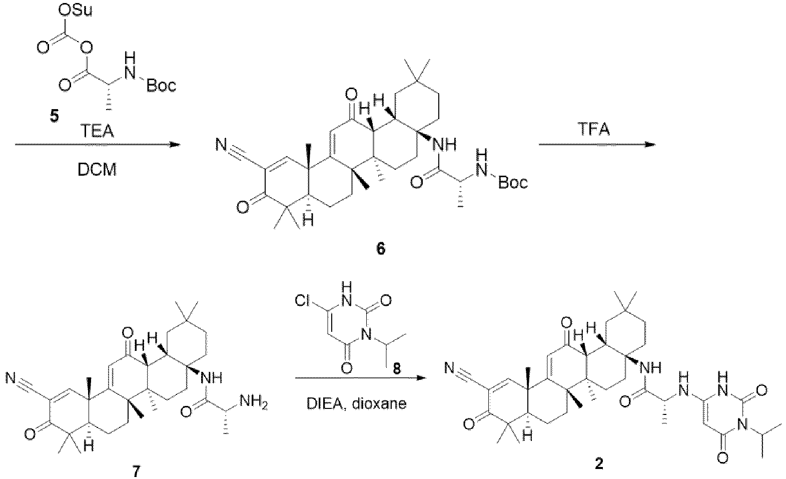

### Step 1: Synthesis of Intermediate 2'

Starting material **1** (3 g, 6.10 mmol) was dissolved in anhydrous toluene (60 mL). Triethylamine (12.35 g, 122.04 mmol, 16.99 mL) and DPPA (5.04 g, 18.31 mmol, 3.95 mL) were added at 0 °C, and the mixture was stirred at 25 °C for 2 h. After the reaction was complete as determined by LCMS, the reaction mixture was concentrated to afford a crude product, which was purified by column chromatography (silica gel, petroleum ether/ethyl acetate = 1:0 to 3:1) to obtain Intermediate **2'** (2.8 g, yield 79.93%, colorless oil). LCMS: rt = 0.677 min, 517.4 [M+H]⁺; purity 90.74%.

### Step 2: Synthesis of Intermediate 3'

Intermediate **2'** (2.8 g, 5.42 mmol) was dissolved in anhydrous toluene (20 mL) and stirred at 80 °C for 2 h. After the reaction was complete as determined by LCMS, the reaction mixture was concentrated to obtain Intermediate **3'** (2.6 g, white solid). LCMS: rt = 0.693 min, 489.3 [M+H]⁺.

### Step 3: Synthesis of Intermediate 4

Intermediate **3'** (2.9 g, 5.93 mmol) was dissolved in anhydrous acetonitrile (30 mL), followed by addition of concentrated hydrochloric acid (12 M, 5 mL). The reaction mixture was stirred at 25 °C for 0.5 h. After the reaction was complete as determined by LCMS, the reaction mixture was adjusted to a pH greater than 8 by adding 20% sodium hydroxide solution (50 mL) at 0°C, and then extracted with ethyl acetate (20 mL × 3). The combined organic layers were washed with saturated brine (20 mL × 2), dried over anhydrous sodium sulfate, filtered under reduced pressure and concentrated to obtain a residue. The residue was purified by column chromatography (silica gel, petroleum ether/ethyl acetate = 1/1, then dichloromethane/methanol = 10/1) to obtain Intermediate **4** (2.2 g, yield 79.32%, white solid). LCMS: rt = 0.481, 0.749 min, 485.3 [M+Na]⁺; purity 99.18%; ¹H NMR (400 MHz, CHLOROFORM-*d*) δ = 8.06 (s, 1H), 5.98 (s, 1H), 3.60 (d, *J* = 4.6 Hz, 1H), 2.22 (br d, *J* = 13.2 Hz, 1H), 2.15 - 2.05 (m, 1H), 2.04 - 1.93 (m, 1H), 1.87 - 1.78 (m, 3H), 1.78 - 1.52 (m, 4H), 1.51 - 1.42 (m, 3H), 1.37 - 1.23 (m, 10H), 1.22 - 1.12 (m, 4H), 1.03 - 0.96 (m, 7H), 0.90 (s, 3H).

### Step 4: Synthesis of Intermediate 6

Intermediate **4** (300 mg, 5.93 mmol) and Intermediate **5** were dissolved in anhydrous dichloromethane (6 mL), triethylamine (196.84 mg, 1.95 mmol, 270.75 µL) was added, and the mixture was stirred at 25 °C for 12 h. After the reaction was complete as determined by LCMS, the reaction mixture was filtered under reduced pressure and concentrated to obtain a crude product, which was purified by reversed phase column chromatography (C₁₈ column, 0.1% aqueous formic acid solution). After rotary evaporation, Intermediate 6 (400 mg, yield 97.32%, yellow solid) was obtained. LCMS: rt = 0.631 min, 656.4 [M+Na]⁺; purity 100%.

### Step 5: Synthesis of Intermediate 7

Intermediate **6** (360 mg, 567.95 µmol) was dissolved in anhydrous dichloromethane (4 mL). Trifluoroacetic acid (0.4 mL) was added, and the mixture was stirred at 25 °C for 3 hours. The reaction mixture was concentrated under reduced pressure to obtain Intermediate **7** (360 mg, yellow oil). LCMS: rt = 0.477 min, 534.3 [M+H]⁺.

### Step 6: Synthesis of Compound 2

Intermediate **7** (180 mg, 337.24 µmol) and Intermediate **8** (95.41 mg, 505.86 µmol) were dissolved in dioxane (2 mL). Diisopropylethylamine (217.93 mg, 1.69 mmol, 293.71 µL) was added, and the mixture was subjected to microwave reaction at 120 °C for 4 hours. After the reaction was complete as determined by LCMS, the reaction mixture was filtered under reduced pressure and concentrated to obtain a crude product, which was purified by preparative chromatography (C₁₈ column, 0.1% formic acid solution) and lyophilized to obtain Compound **2** (18.6 mg, off-white solid, yield 3.84%). LCMS: rt =1.463 min, 686.4 [M+H]⁺; purity 95.47%; ¹H NMR (400 MHz, DMSO-*d6*) δ = 9.95 (s, 1H), 8.65 (s, 1H), 7.59 (s, 1H), 6.27 (d, *J* = 7.8 Hz, 1H), 6.23 (s, 1H), 4.99 - 4.89 (m, 1H), 4.42 (d, *J* = 1.6 Hz, 1H), 3.92 (br t, *J* = 7.4 Hz, 1H), 3.10 - 3.01 (m, 1H), 2.98 (br d, *J* = 4.4 Hz, 1H), 2.19 - 2.10 (m, 1H), 2.07 (s, 1H), 2.01 - 1.92 (m, 1H), 1.89 - 1.78 (m, 3H), 1.74 - 1.62 (m, 3H), 1.55 (br d, *J* = 14.0 Hz, 1H), 1.43 - 1.35 (m, 7H), 1.32 - 1.27 (m, 8H), 1.23 (br d, *J* = 6.6 Hz, 3H), 1.18 (s, 4H), 1.07 - 0.98 (m, 7H), 0.94 (s, 3H), 0.86 (s, 3H).

### Example 3

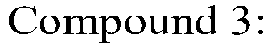

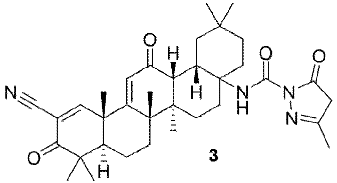

### Synthetic Route:

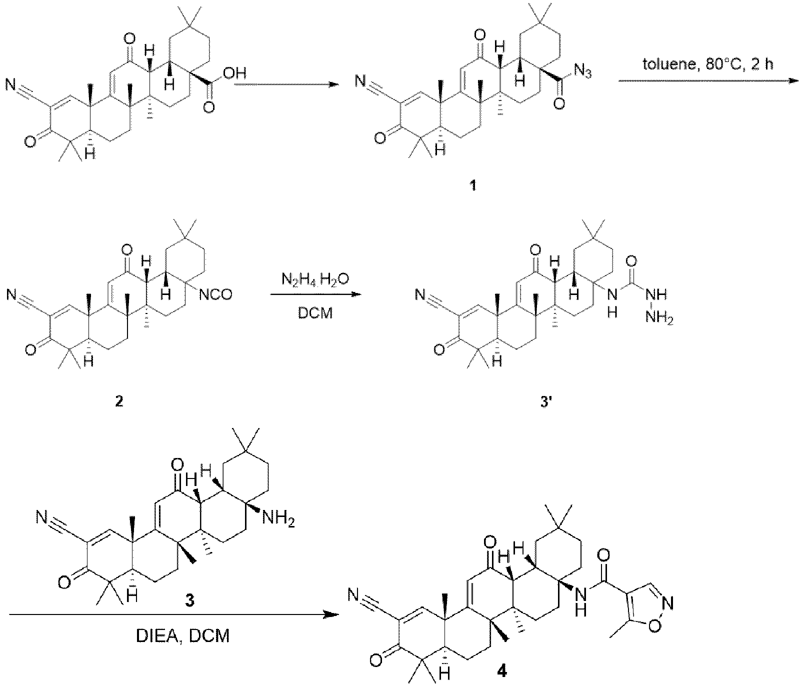

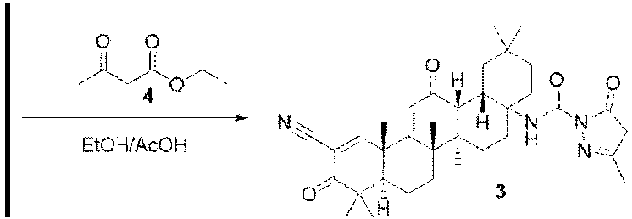

### Example 4

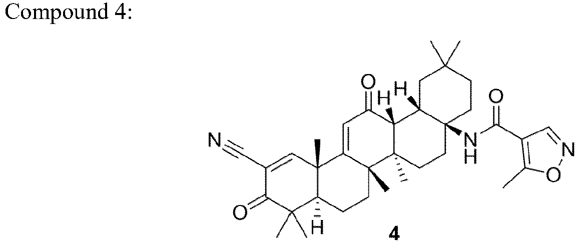

### Synthetic Route:

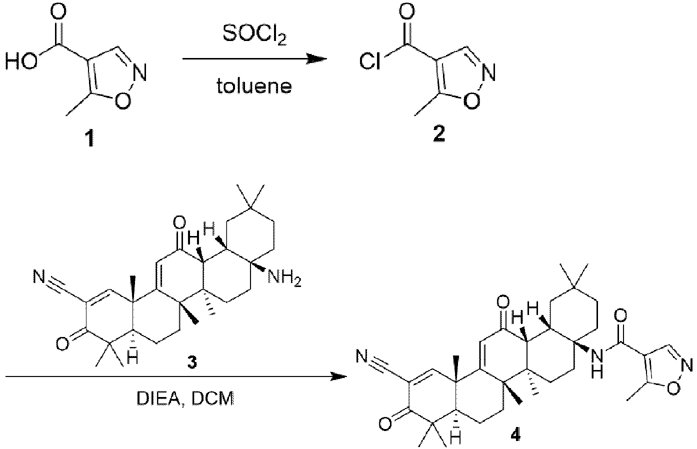

### Step 1: Synthesis of Intermediate 2

Intermediate 1 (150 mg, 1.18 mmol, 1 eq) was added to anhydrous toluene (1.5 mL), and then thionyl chloride (702.04 mg, 5.90 mmol, 428.59 µL, 5 eq) was added. The mixture was stirred at 70 °C for 1 h. After the reaction was complete as determined by LCMS, the reaction mixture was concentrated under reduced pressure to obtain a crude product (150 mg), which was used directly in the next step.

### Step 2: Synthesis of Compound 4

Intermediate 3 (198.68 mg, 429.42 µmol, 0.5 eq) was added to anhydrous dichloromethane (2 mL), and then diisopropylethylamine (333.00 mg, 2.58 mmol, 448.79 µL, 3 eq) was added. The reaction mixture was cooled to 0 °C, and Intermediate 2 (125 mg, 858.85 µmol, 1 eq) was added slowly. The reaction mixture was stirred at 25 °C for 1 h. After the reaction was complete as determined by LCMS, the reaction mixture was concentrated to obtain a crude product, which was purified by preparative chromatography (C₁₈ column, 0.1% formic acid solution) and lyophilized to obtain Compound **4** (73.4 mg, off-white solid, yield 14.85%). LCMS: rt = 1.649 min, 594.3 [M+Na]⁺, purity 99.367%.¹H NMR (400 MHz, DMSO-d6) δ = 8.96 (s, 1H), 8.66 (s, 1H), 7.34 (s, 1H), 6.23 (s, 1H), 3.23 - 3.17 (m, 1H), 2.97 - 2.87 (m, 1H), 2.60 (s, 3H), 2.14 - 2.05 (m, 1H), 2.05 - 1.90 (m, 3H), 1.90 - 1.71 (m, 4H), 1.71 - 1.58 (m, 2H), 1.55 - 1.44 (m, 2H), 1.44 - 1.41 (m, 3H), 1.36 - 1.33 (m, 3H), 1.33 - 1.26 (m, 2H), 1.23 - 1.18 (m, 1H), 1.18 - 1.15 (m, 3H), 1.06 (s, 3H), 1.00 (s, 3H), 0.96 (s, 3H), 0.88 (s, 3H).

### Example 5

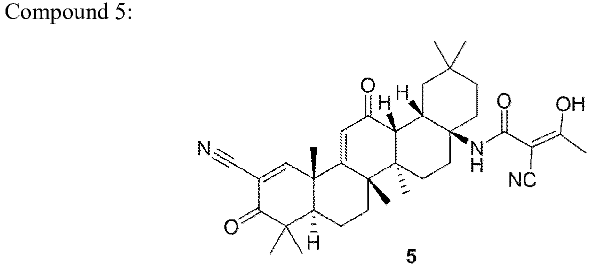

### Synthetic Route:

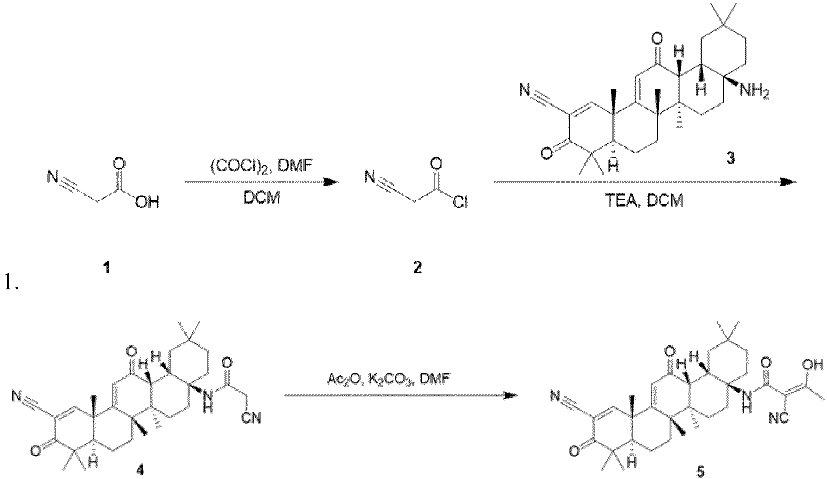

### Example 6

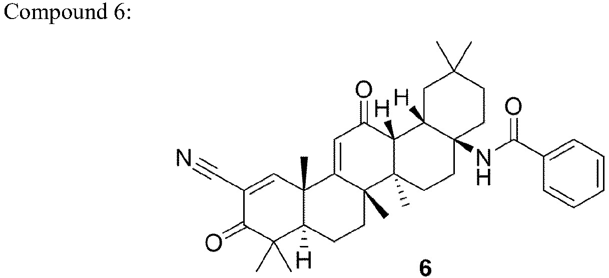

### Synthetic Route:

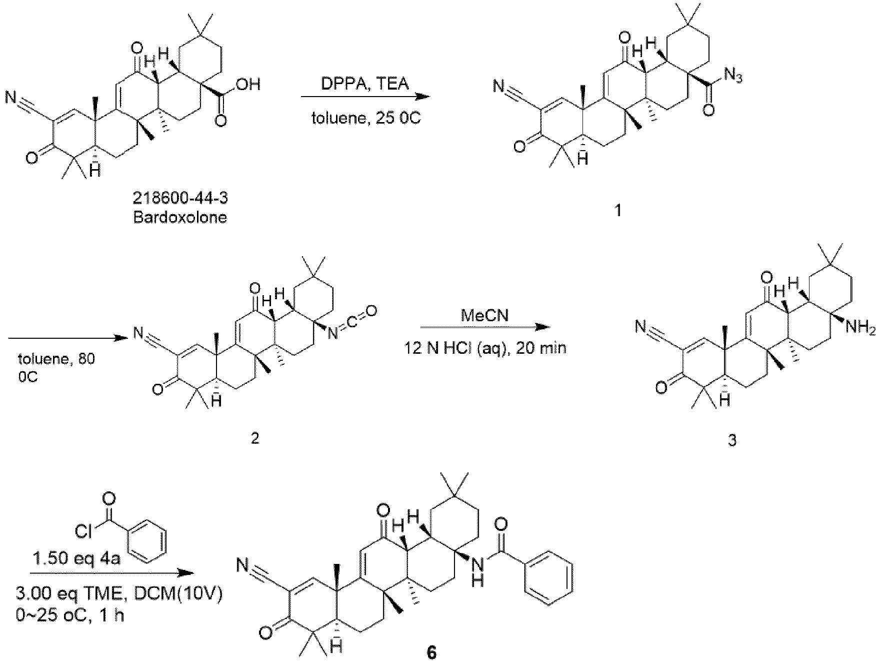

### Step 1: Synthesis of Intermediate 1

DPPA (3.36 g, 12.2 mmol, 2.63 mL, 3.00 eq) and Et₃N (8.24 g, 81.4 mmol, 11.3 mL, 20.0 eq) were added to a toluene solution (40.0 mL) of starting material 218600-44-3 (2.00 g, 4.07 mmol, 1.00 eq). The mixture was stirred dropwise at 0 °C for 6 h. LC-MS (EB11687-32-P1A2) showed that approximately 84.0% of the product was detected. The mixture was concentrated to obtain a crude produc, which was purified by column chromatography (SiO₂, 0-10% EtOAc in CH₂Cl₂ solution) and TLC (CH₂Cl₂: EtOH = 10:1, Rf = 0.5) to obtain Intermediate 1 (1.40 g, 2.71 mmol, yield 67.0%), as a white solid. **LCMS:** (EB11687-32-P1A1_LCMS_SH), RT = 1.236 min, MS (ESI) m/z = 517 [M+1]⁺.

### Step 2: Synthesis of Intermediate 2

A solution of Intermediate 1 (1.40 g, 2.71 mmol, 1.00 eq) in toluene (104 mL) was stirred at 110 °C for 2 h. LC-MS (EB11687-34-P1 C1) showed that approximately 97.0% of the desired compound was detected. The mixture was concentrated to obtain a crude product. Crude Intermediate 2 (1.20 g, 2.46 mmol, yield 90.6%) was obtained as a white solid and used in the next step without further purification. **LCMS**: (EB11687-34-P1C1_LCMS_SH), RT = 1.231 min, MS (ESI) m/z = 489 [M+1]⁺.

### Step 3: Synthesis of Intermediate 3

HCl (12 M, 13.00 mL, 58.6 eq) was added dropwise to a solution of Intermediate 2 (1.20 g, 2.46 mmol, 1.00 eq) in MeCN (7.70 mL). After stirring at 25 °C for 20 min, EtOAc (11.0 mL) was added, and the mixture was cooled to 0 °C. NaOH (0.67 mL, 10.0% aq) and saturated NaHCO₃ (4.60 mL) were added, and the mixture was stirred for 5 min. LC-MS (EB11687-45-P1A1) showed that about 96.6% of the desired compound was detected. The organic phase was separated, washed with brine, dried over NaSO₄, and concentrated. Crude Intermediate 3 (1.10 g, 2.38 mmol, yield 93.3%) was obtained as a yellow solid and used in the next step without further purification. **LCMS**: (EB11687-45-P1A1_LCMS_SH) RT = 1.730 min, MS (ESI) m/z = 463 [M+1]⁺.

### Step 4: Synthesis of Compound 6

Starting material 3a (91.2 mg, 648 µmol, 75.3 µL, 1.50 eq) was slowly added to a solution of Intermediate 3 (200 mg, 432 µmol, 1.00 eq) and TME (131 mg, 1.30 mmol, 181 µL, 3.00 eq) in DCM (2 mL). The reaction mixture was stirred at 0-25°C for 1 h. LCMS (EB11739-8-P1A1) showed that Intermediate 3 had been consumed and the desired MS peak was detected. The mixture was concentrated at 25°C to obtain a residue. The residue was purified by prep-HPLC: Xtimate C18 150*40mm*10µm; mobile phase: [water (NH₄HCO₃)-ACN]; gradient: 52%-92% B/25 to obtain Compound **6** (68.0 mg, 119µmol, yield 27.5%, purity 99.2%) as a white solid. (LCMS: EB11739-8-P1B2y, HNMR: EB11739-8-P1D1, HPLC: EB11739-8-P1 C2). **LCMS:** EB11739-8-P1A1, RT = 1.681 min, MS (ESI) *m*/*z* = 567.4 [M+1]⁺. EB11739-8-P1B2y, RT = 1.361 min, MS (ESI) *m*/*z* = 567.2 [M+1]⁺. **HPLC:** EB11739-8-P1C2,RT = 4.788 min, purity 99.2 %. **¹H NMR:** EB11739-8-P1D1, 400MHz, CDCl3-*d1* δ 8.03 (s, 1H) 7.70 - 7.76 (m, 2H) 7.47 - 7.53 (m, 1H) 7.39 - 7.45 (m, 2H) 5.98 (s, 1H) 5.85 (s, 1H) 3.22 (d, *J* = 4.64 Hz, 1H) 2.78 (d, *J* = 13.00 Hz, 1H) 2.44 (d, *J* = 14.00 Hz, 1H) 2.19 (dt, *J* = 13.74, 3.28 Hz, 1H) 1.96 - 2.05 (m, 2H) 1.71 - 1.94 (m, 7H) 1.51 - 1.64 (m, 3H) 1.42 (s, 3H) 1.29 - 1.39 (m, 3H) 1.25 (s, 3H) 1.20 (d, *J* = 13.14 Hz, 1H) 1.16 (s, 3H) 1.05 (d, *J* = 6.74 Hz, 6H) 0.91 (s, 3H).

### Example 7

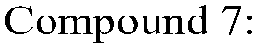

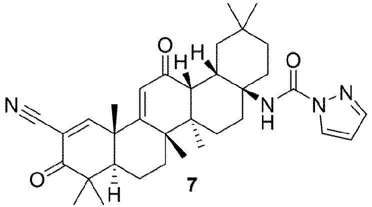

### Synthetic Route:

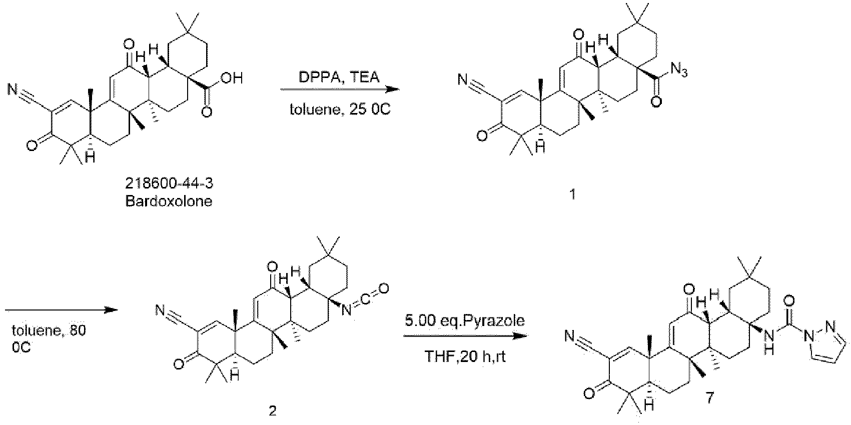

### Step 1: Synthesis of Intermediate 1

DPPA (3.36 g, 12.2 mmol, 2.63 mL, 3.00 eq) and Et₃N (8.24 g, 81.4 mmol, 11.3 mL, 20.0 eq) were added to a solution of starting material 218600-44-3 (2.00 g, 4.07 mmol, 1.00 eq) in toluene (40.0 mL). The mixture was stirred dropwise at 0 °C for 6 h. LC-MS (EB11687-32-P1A2) showed that about 84.0% of the product was detected. The mixture was concentrated to obtain a crude product, which was purified by column chromatography (SiO₂, 0-10% EtOAc in CH₂Cl₂ solution) and TLC (CH₂Cl₂: EtOH = 10:1, Rf = 0.5) to obtain Intermediate 1 (1.40 g, 2.71 mmol, yield 67.0%), as a white solid. **LCMS:** (EB11687-32-1A1_LCMS_SH), RT = 1.236 min, MS (ESI) m/z = 517 [M+1]⁺.

### Step 2: Synthesis of Intermediate 2

A solution of Intermediate 1 (1.40 g, 2.71 mmol, 1.00 eq) in toluene (104 mL) was stirred at 110 °C for 2 h. LC-MS (EB11687-34-P1C1) showed that about 97.0% of the desired compound was detected. The mixture was concentrated to obtain a crude product. Crude Intermediate 2 (1.20 g, 2.46 mmol, yield 90.6%) was obtained as a white solid and used in the next step without further purification. **LCMS:** (EB11687-34-P1C1_LCMS_SH), RT = 1.231 min, MS (ESI) m/z = 489 [M+1]⁺.

Step 3: Synthesis of Compound 7. Pyrazole (453 mg, 6.65 mmol, 5.00 eq) was added to a solution of Intermediate 2 (650 mg, 1.33 mmol, 1.00 eq) in THF (12.0 mL). The mixture was stirred at 25 °C for 20 h. LC-MS (EB11687-36-P1A1) showed a yield of approximately 88.0%. The crude product was purified by reversed phase HPLC (column: Welch Xtimate C₁₈ 40*200mm 7µm; mobile phase: [water (NH₃H₂O+NH₄HCO₃)-ACN]; gradient: 58%-98% B over 25 min). Compound 7 (450 mg, 771 µmol, yield 58.0%, purity 95.4%) was obtained as a white solid. **LCMS:** (EB11687-38-P1A1_LCMS_SH): RT = 2.859 min, MS (ESI) *m*/*z* = 557 [M+1]⁺; RT = 3.027 min, MS (ESI) *m*/*z* = 489 [M-67]⁺. **HPLC:** (EB11687-38-P2C1): RT = 3.273 min; RT = 3.681 min. **1HNMR:** (EB11415-16-P1A) (CDCl3, 400 MHz). δ ppm 0.94 (s, 3H) 1.06 (s, 3H) 1.11 (s, 3H) 1.17 (s, 3H) 1.25 - 1.28 (m, 3H) 1.31 - 1.40 (m, 6H) 1.51 - 1.69 (m, 6H) 1.72 - 1.83 (m, 4H) 1.87 - 2.15 (m, 4H) 2.27 (d, J =13.70, 9.20 Hz, 1H) 2.94 - 3.02 (m, 1H) 3.16 (d, J =4.80 Hz, 1H) 5.99 (s, 1H) 6.39 (dd, J =2.60, 1.63 Hz, 1H) 7.13 (s, 1 H) 7.55 (d, J =0.88 Hz, 1H) 8.04 (s, 1H) 8.16 - 8.21 (m, 1H).

### Example 8

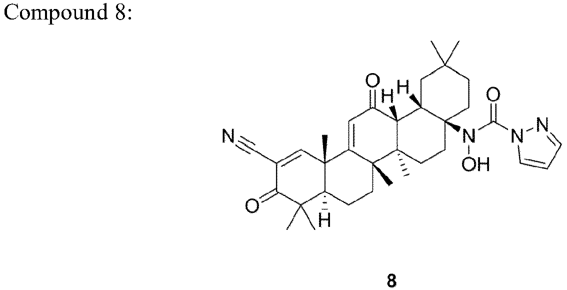

### Synthetic Route:

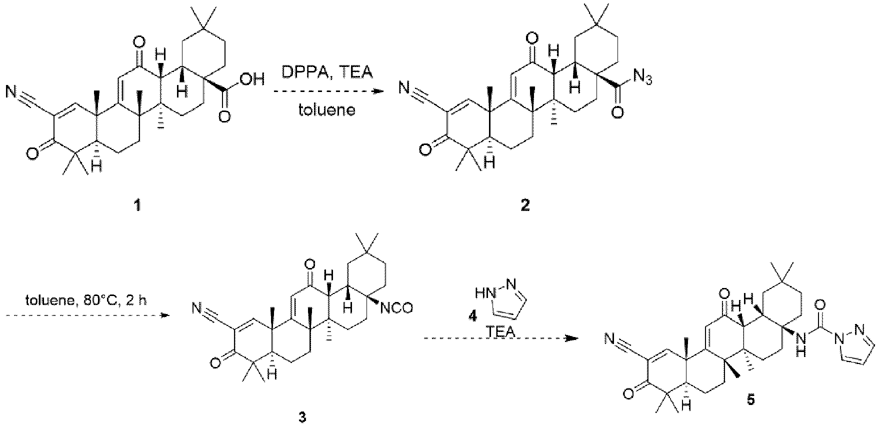

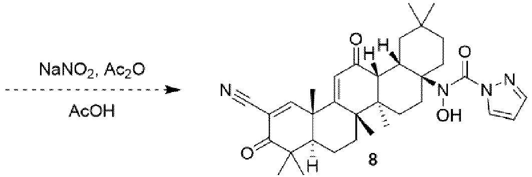

### Example 9

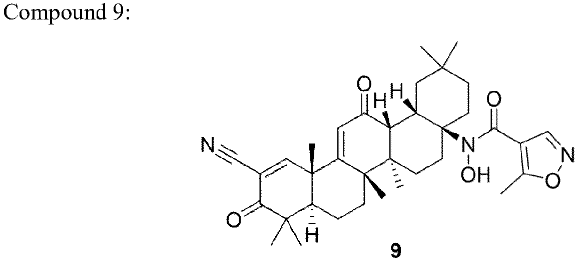

### Synthetic Route:

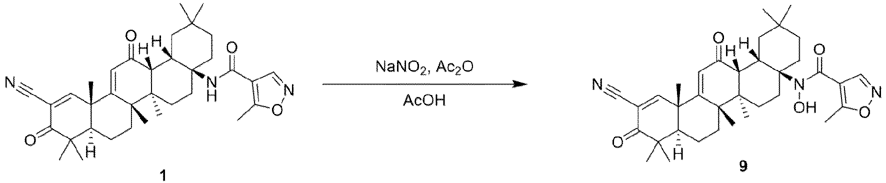

### Example 10

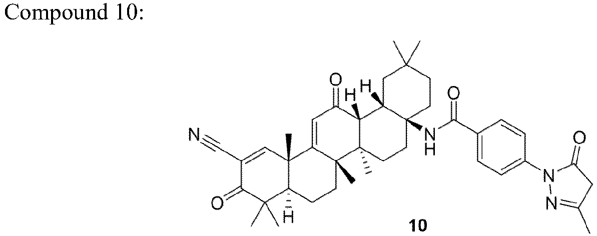

### Synthetic Route:

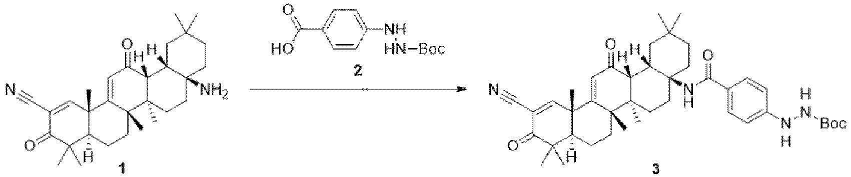

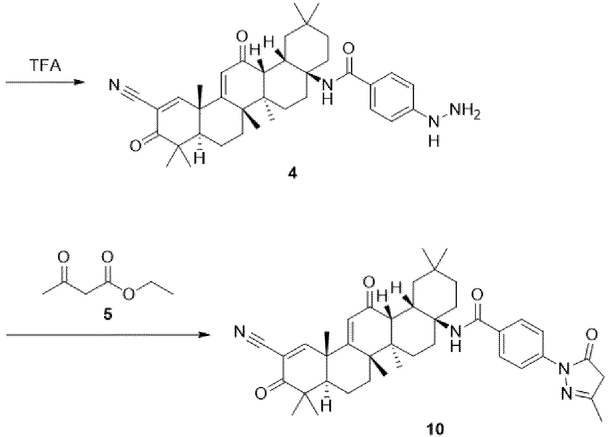

### Step 1: Synthesis of Intermediate 3

Starting material 1 (250 mg, 540.35 µmol, 1 eq) and starting material 2 (136.31 mg, 540.35 µmol, 1 eq) were dissolved in anhydrous N,N-dimethylformamide (3 mL), and then HATU (246.55 mg, 648.42 µmol, 1.2 eq) and diisopropylethylamine (209.50 mg, 1.62 mmol, 282.35 µL, 3 eq) were added. The mixture was then protected with nitrogen and reacted at 25 °C for 12 hours. After the reaction of the starting materials was completed as detected by LCMS, the reaction mixture was poured into water (10 mL) and extracted with ethyl acetate (10 mL*3). The organic phase was washed with saturated brine (10 mL*2), dried over anhydrous sodium sulfate, and concentrated under reduced pressure. The crude product was purified by normal phase column chromatography (petroleum ether: ethyl acetate = 1:0-1:1) to obtain Intermediate 3 (369.4 mg, 710.27 µmol, yield 98.09%) as a yellow solid.

### Step 2: Synthesis of Intermediate 4

Intermediate 3 (445 mg, 638.53 µmol, 1 eq) was dissolved in anhydrous dichloromethane (5 mL), and trifluoroacetic acid (1.54 g, 13.46 mmol, 1 mL, 21.08 eq) was added and reacted at room temperature for 1 hour. After the reaction of the starting materials was completed as detected by LCMS, the reaction mixture was directly concentrated to obtain Intermediate 4 (380 mg, 636.73 µmol, yield 99.72%) as a yellow solid, which was directly used in the next step. LCMS: rt = 0.530 min, 597.4 [M+H]⁺.

### Step 3: Synthesis of Compound 10

Intermediate 4 (380 mg, 636.73 µmol, 1 eq) was dissolved in anhydrous acetonitrile (5 mL), and ethyl acetoacetate (82.86 mg, 636.73 µmol, 80.61 µL, 1 eq) was added. The mixture was then heated to 60 °C and reacted for 1 hour. After the reaction of the starting materials was completed as detected by LCMS, the reaction mixture was concentrated to remove part of the solvent, and the crude product was purified by reverse phase preparative chromatography and lyophilized to obtain Compound **10** (147.8 mg, 215.17 µmol, yield 33.79%) as a white solid. Prep-HPLC: column: YMC-Actus Triart C18 150*30 mm*7 µm; mobile phase: [water(FA)-ACN]; gradient: 62% - 92% B over 10 min. LCMS: rt = 1.593 min, 663.4 [M+H]⁺; HPLC: 2.274 min. ¹H NMR: (400 MHz, CHLOROFORM-d) δ = 8.05 (s, 1H), 7.99 (d, J = 8.8 Hz, 2H), 7.76 (d, J = 8.8 Hz, 2H), 6.02 (s, 1H), 5.67 (s, 1H), 3.47 (s, 2H), 3.22 (d, J = 4.6 Hz, 1H), 2.84 - 2.76 (m, 1H), 2.43 (br d, J = 14.0 Hz, 1H), 2.23 (s, 3H), 2.18 (br t, J = 3.2 Hz, 1H), 2.08 - 1.98 (m, 2H), 1.93 - 1.84 (m, 1H), 1.82 - 1.78 (m, 3H), 1.63 (br d, J = 13.0 Hz, 2H), 1.49 (s, 3H), 1.42 (s, 3H), 1.39 - 1.31 (m, 3H), 1.28 - 1.22 (m, 4H), 1.18 (s, 4H), 1.08 (d, J = 3.4 Hz, 6H), 0.94 (s, 3H).

### Example 11

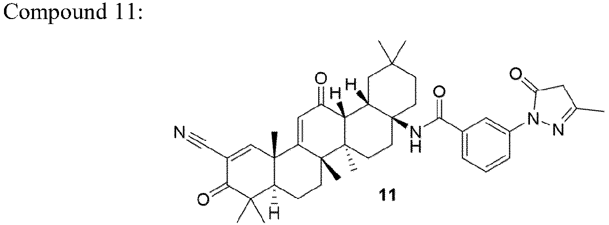

### Synthetic Route:

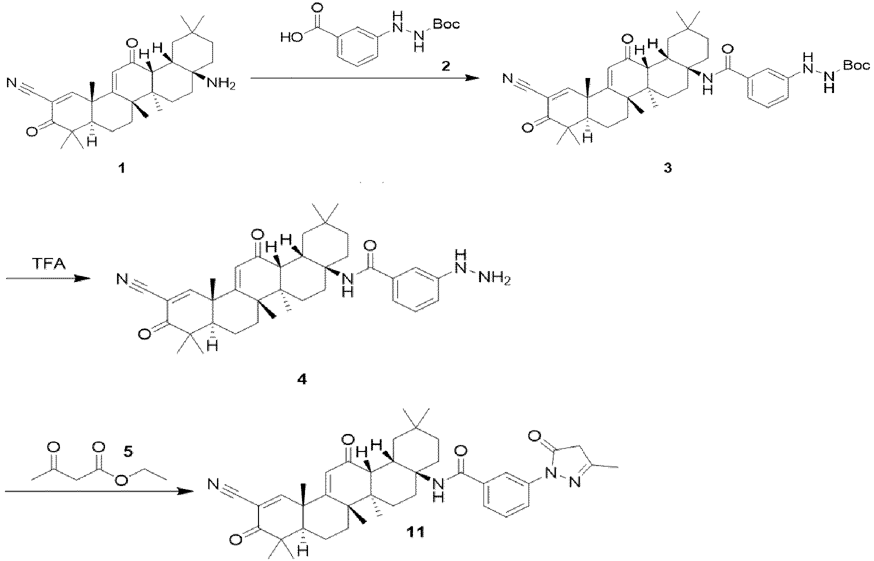

### Step 1: Synthesis of Intermediate 3

Starting material 2 (163.57 mg, 648.42 µmol, 1 eq), HATU (369.82 mg, 972.63 µmol, 1.5 eq), and N,N-diisopropylethylamine (251.41 mg, 1.95 mmol, 338.83 µL, 3 eq) were added to a solution of starting material 1 (300 mg, 648.42 µmol, 1 eq) in N,N-dimethylformamide (5 mL). The reaction mixture was reacted at 20 °C for 12 hours. The reaction was monitored by LC-MS, which showed that the starting materials were completely consumed and the main peak corresponded to the desired product. The reaction mixture was poured into 30 mL of water and extracted three times with ethyl acetate (10 mL*3). The combined organic phases were washed with saturated sodium chloride solution (10 mL), dried over anhydrous sodium sulfate, filtered, concentrated, and mixed with silica gel for sample loading. The crude product was purified by normal phase column chromatography (petroleum ether/ethyl acetate = 0-50%) to obtain Intermediate 3 (376 mg, 501.75 µmol, 87.42% yield) as a light yellow solid. LCMS: Rt = 0.624 min, 641.5 [M-56+H] + ESI pos.

### Step 2: Synthesis of Intermediate 4

Trifluoroacetic acid (2.5 mL) was added to a solution of Intermediate 3 (370 mg, 530.91 µmol, 1 eq) in dichloromethane (5 mL). The reaction mixture was reacted at 30 °C for 1 hour. The reaction was monitored by LC-MS, which showed that the starting materials were completely consumed and the main peak corresponded to the desired product. The reaction mixture was concentrated under reduced pressure to obtain Compound 4 (315 mg, 527.81 µmol, 99.42% yield), as a light yellow solid. LCMS: Rt = 0.515 min, 597.4 [M+H]⁺ ESI pos.

### Step 3: Synthesis of Compound 11

Intermediate 5 (68.69 mg, 527.81 µmol, 66.82 µL, 1 eq) was added to a solution of Intermediate 4 (315 mg, 527.81 µmol, 1 eq) in acetonitrile (4 mL). The reaction mixture was heated to 60 °C and reacted for 1 hour. The reaction was monitored by LC-MS, which showed that the starting materials were completely consumed and the main peak corresponded to the desired product. The reaction mixture was filtered and concentrated to obtain a crude product. The crude product was purified by reverse phase preparative chromatography [water (formic acid)-acetonitrile system] and then lyophilized to obtain Compound 11 (147.2 mg, 210.08 µmol, yield 39.80%, purity 94.6%). Prep-HPLC: column: Phenomenex luna C₁₈ 150*40mm*15um; mobile phase: [water (FA)-ACN]; gradient: 35%-65% B over 22 min. LCMS: Rt = 1.606 min, 663.4 [M+H]⁺ ESI pos. HPLC: Rt = 1.937 min. ¹H NMR (400 MHz, CHLOROFORM-d) δ = 8.23 (s, 1H), 8.04 (d, *J* = 6.8 Hz, 2H), 7.55 (d, *J* = 7.8 Hz, 1H), 7.45 (t, *J* = 7.8 Hz, 1H), 6.01 (s, 1H), 5.80 (s, 1H), 3.46 (s, 2H), 3.25 (d, *J* = 4.6 Hz, 1H), 2.84 (br d, *J* = 13.2 Hz, 1H), 2.45 - 2.30 (m, 1H), 2.20 (s, 3H), 2.18 - 2.15 (m, 2H), 2.12 - 1.86 (m, 1H), 1.83 - 1.75 (m, 4H), 1.66 - 1.54 (m, 2H), 1.48 (d, *J* = 8.4 Hz, 6H), 1.41 - 1.29 (m, 4H), 1.26 (s, 3H), 1.21 (br d, *J* = 13.2 Hz, 1H), 1.17 (s, 3H), 1.07 (d, *J* = 2.8 Hz, 6H), 0.93 (s, 3H).

### Example 12

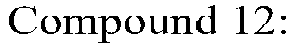

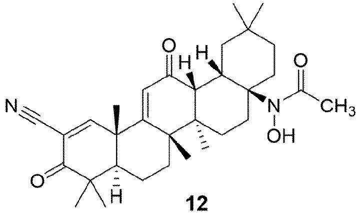

### Synthetic Route:

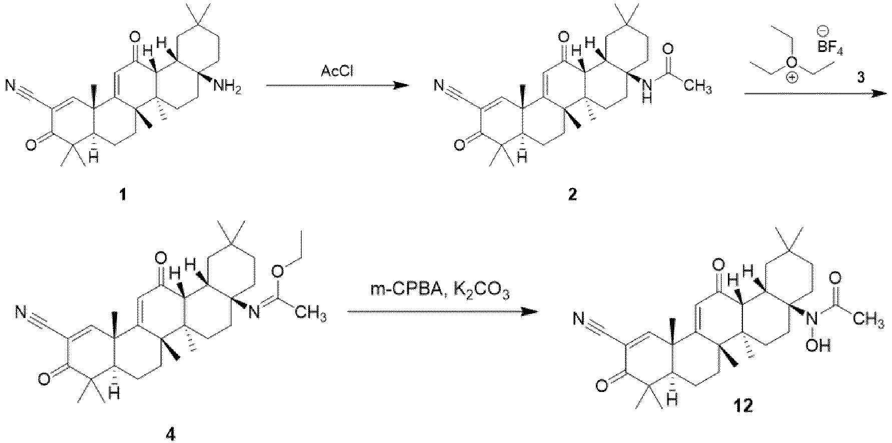

### Example 13

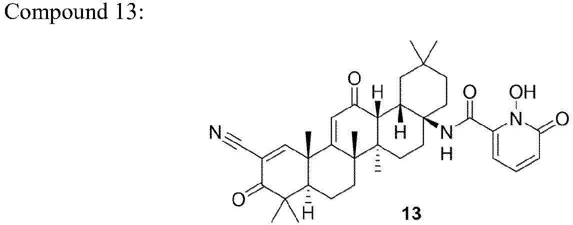

### Synthetic Route:

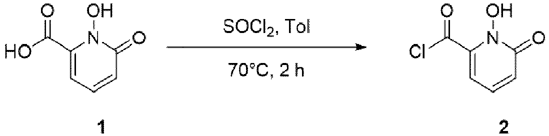

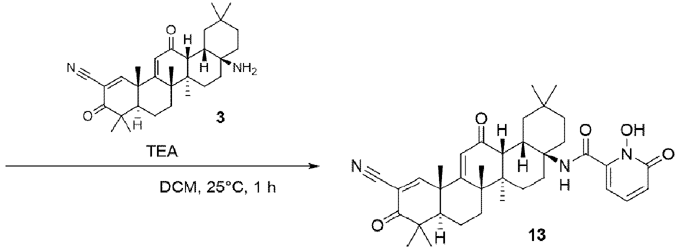

### Step 1: Synthesis of Intermediate 2

Thionyl chloride (6.55 g, 55.07 mmol, 4 mL, 42.71 eq) was added to a solution of starting material 1 (200mg, 1.29mmol, 1eq) in toluene (4 mL). The mixture was purged with nitrogen several times and stirred at 70°C for 2 hours. After the reaction of the starting materials was completed as detected by LCMS, the reaction mixture was concentrated under reduced pressure to obtain Compound 2 (200 mg, crude), as a green solid.

### Step 2: Synthesis of Compound 13

Triethylamine (349.93 mg, 3.46 mmol, 481.34 µL, 8 eq) was added to a solution of starting material 3 (200 mg, 432.28 µmol, 1 eq) in dichloromethane (2 mL). After purging with nitrogen three times, a solution of Intermediate 2 (150.05 mg, 864.56 µmol, 2 eq) in dichloromethane was slowly added, and the mixture was stirred at 25 °C for 1 hour. After the reaction of the starting materials was completed as detected by LCMS, the reaction mixture was concentrated and then purified by reverse phase preparative chromatography and lyophilized to obtain Compound **13** (67.2 mg, 110.48 µmol, 25.56% yield, 98.6% purity), as a grayish-white solid. Prep-HPLC (column: YMC-Actus Triart C18 150*30mm*7µm; mobile phase: [water(FA)-ACN]; gradient: 45%-75% B over 15min). LCMS: Retention time=0.530min, 600.4[M+H]⁺. HPLC: retention time = 2.438 min, purity 98.77%. ¹H NMR (400 MHz, DMSO-d6) δ = 8.65 (s, 1H), 8.47 (br s, 1H), 7.36 (dd, J = 7.2, 8.8 Hz, 1H), 6.52 (dd, J = 1.6, 9.2 Hz, 1H), 6.24 - 6.16 (m, 2H), 3.18 - 3.16 (m, 1H), 3.00 - 2.94 (m, 1H), 2.19 - 2.09 (m, 1H), 1.96 - 1.90 (m, 2H), 1.90 - 1.78 (m, 4H), 1.75 - 1.63 (m, 2H), 1.43 (br d, J = 12.4 Hz, 8H), 1.39 - 1.25 (m, 3H), 1.25 - 1.21 (m, 1H), 1.18 (s, 3H), 1.07 (s, 3H), 1.05 - 1.02 (m, 1H), 1.00 (s, 3H), 0.95 (s, 3H), 0.87 (s, 3H).

### Example 14

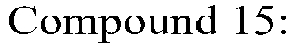

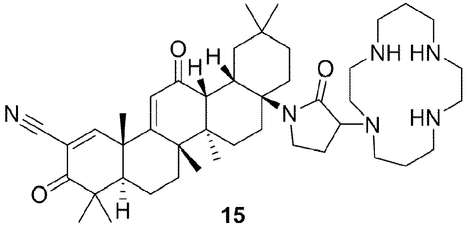

### Synthetic Route:

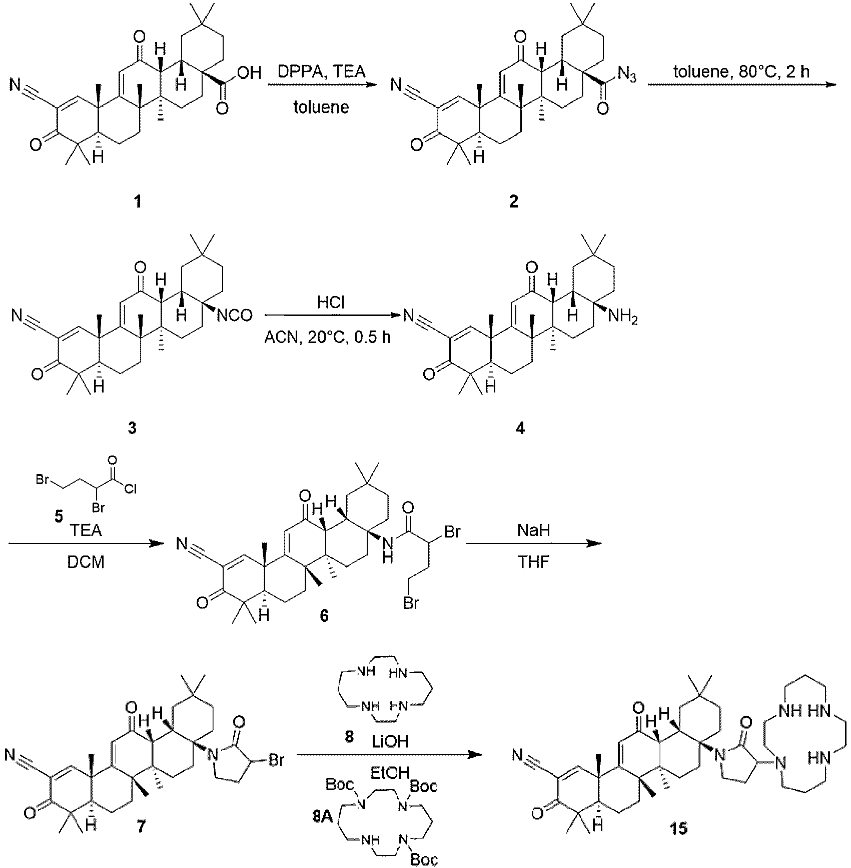

### Example 15

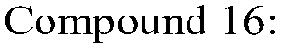

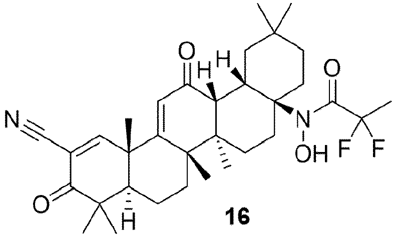

### Synthetic Route:

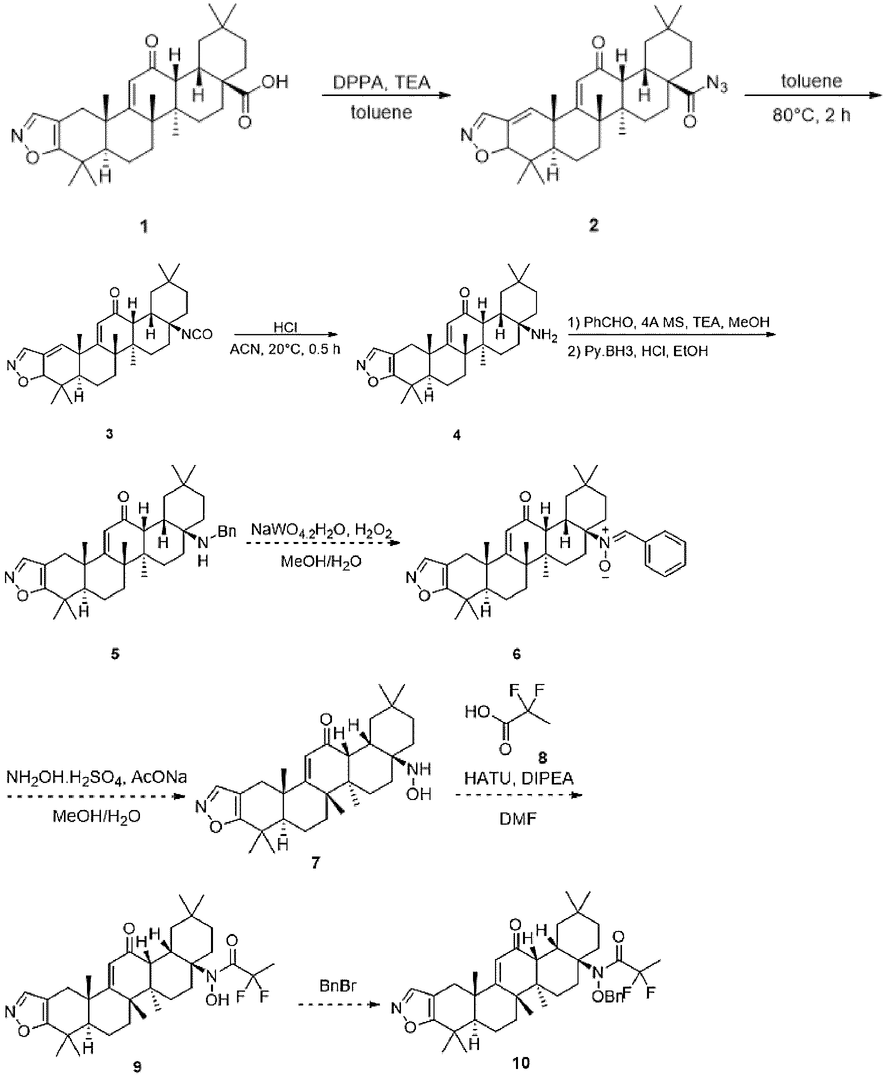

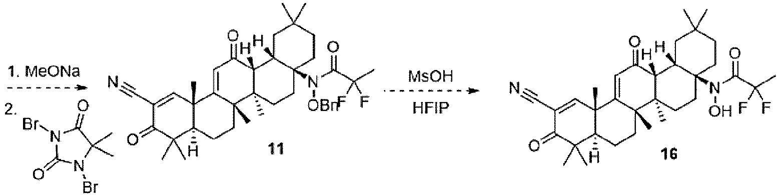

### Example 16

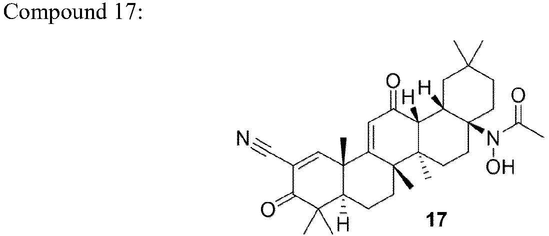

### Synthetic Route:

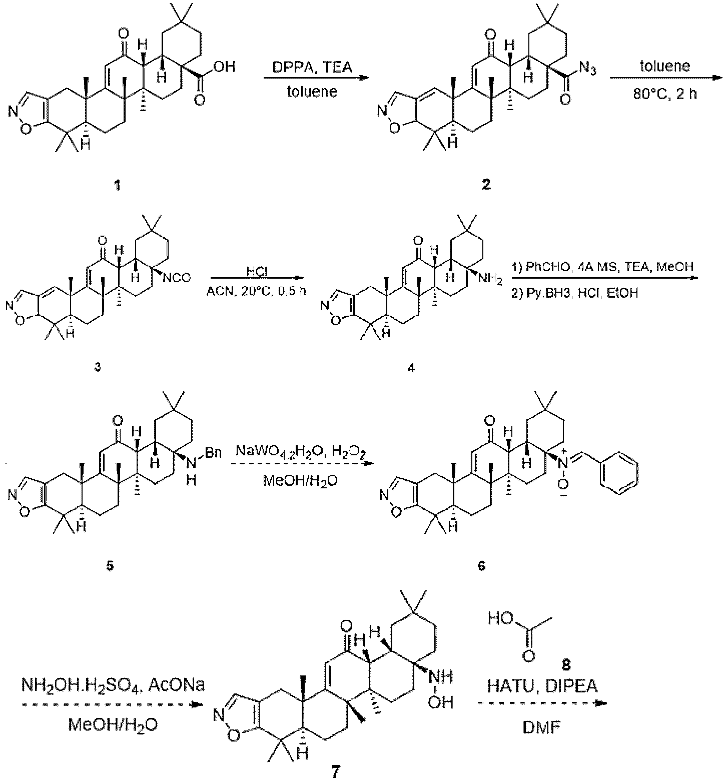

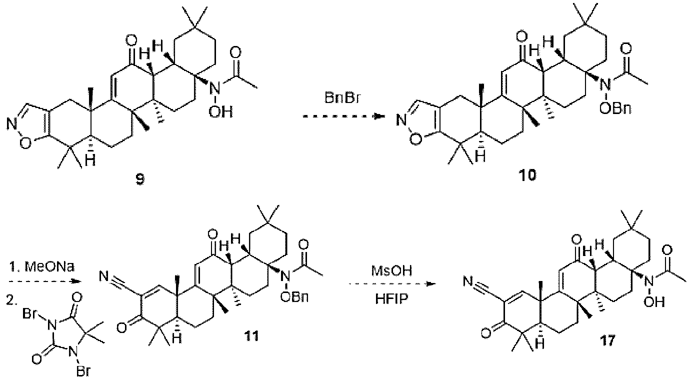

### Example 17

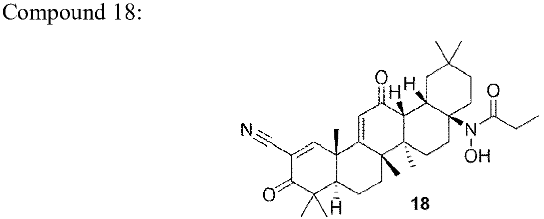

### Synthetic Route:

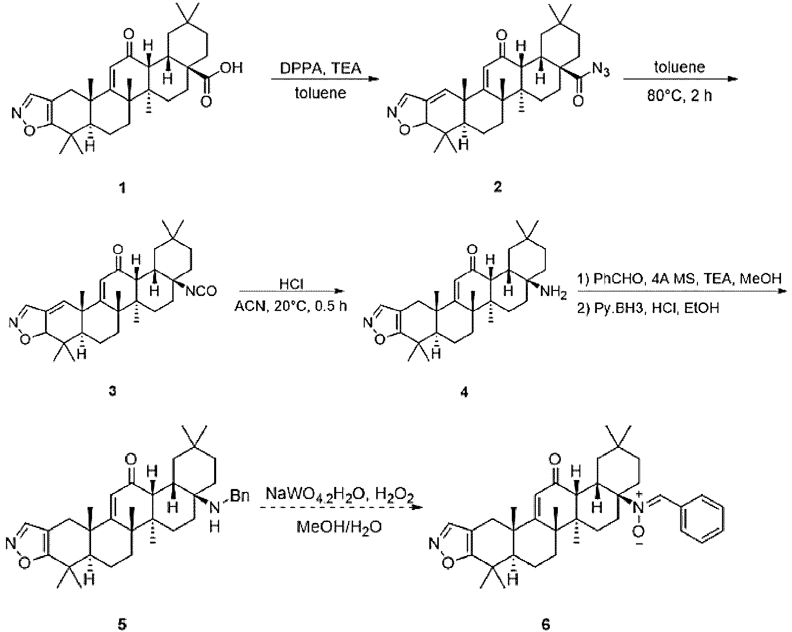

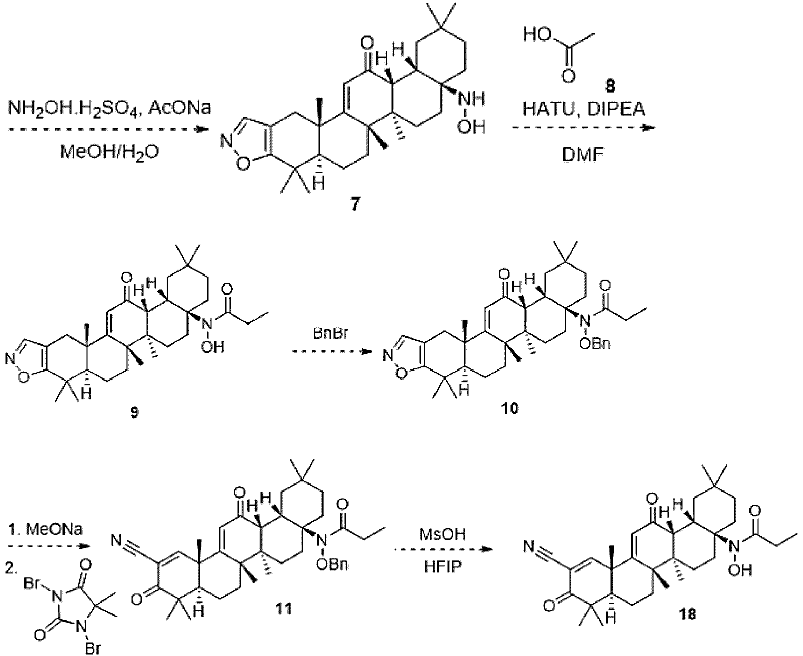

### Example 18

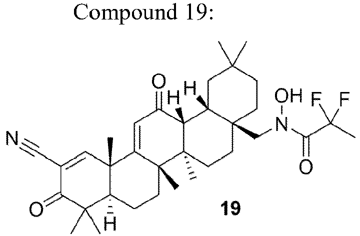

### Synthetic Route:

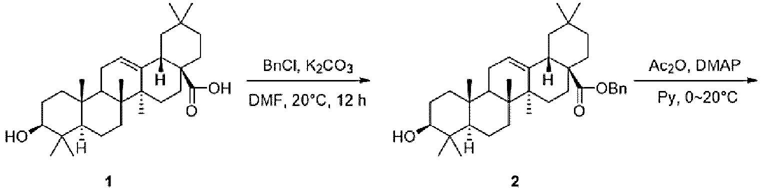

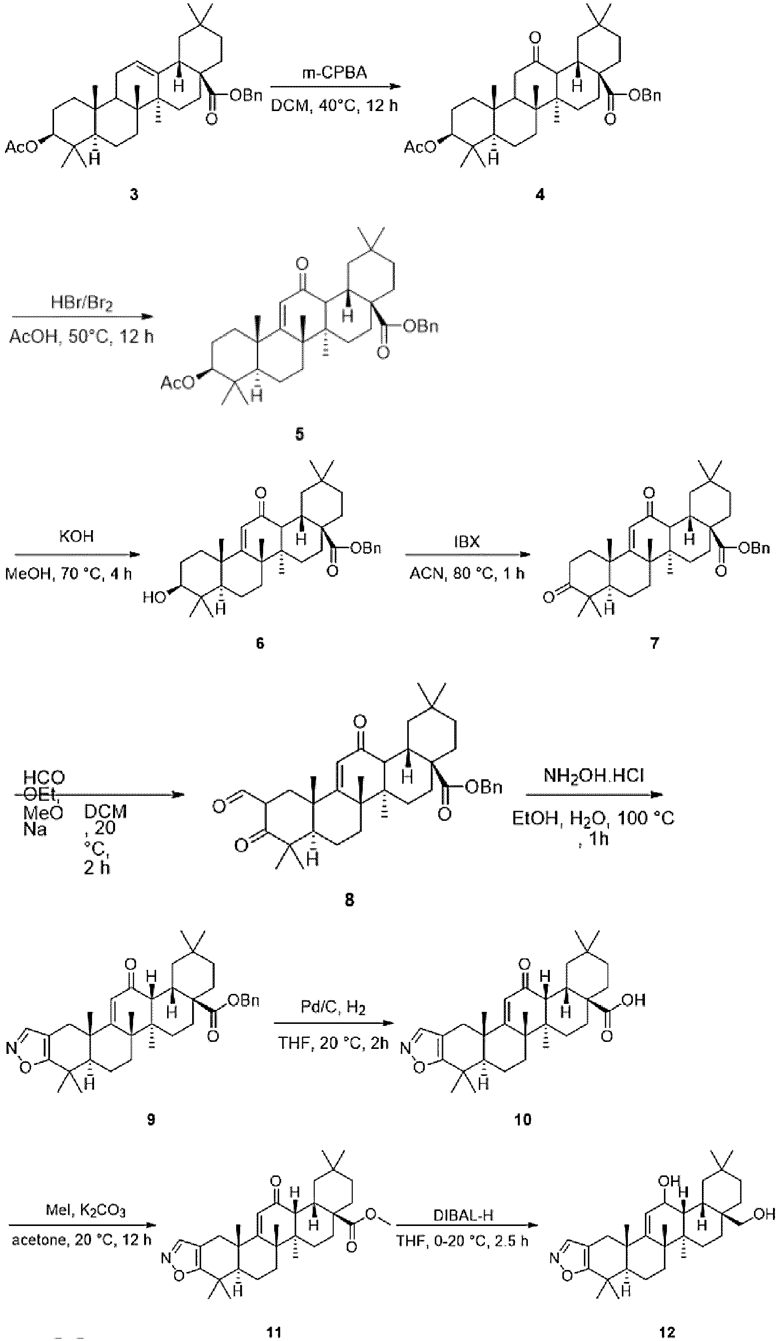

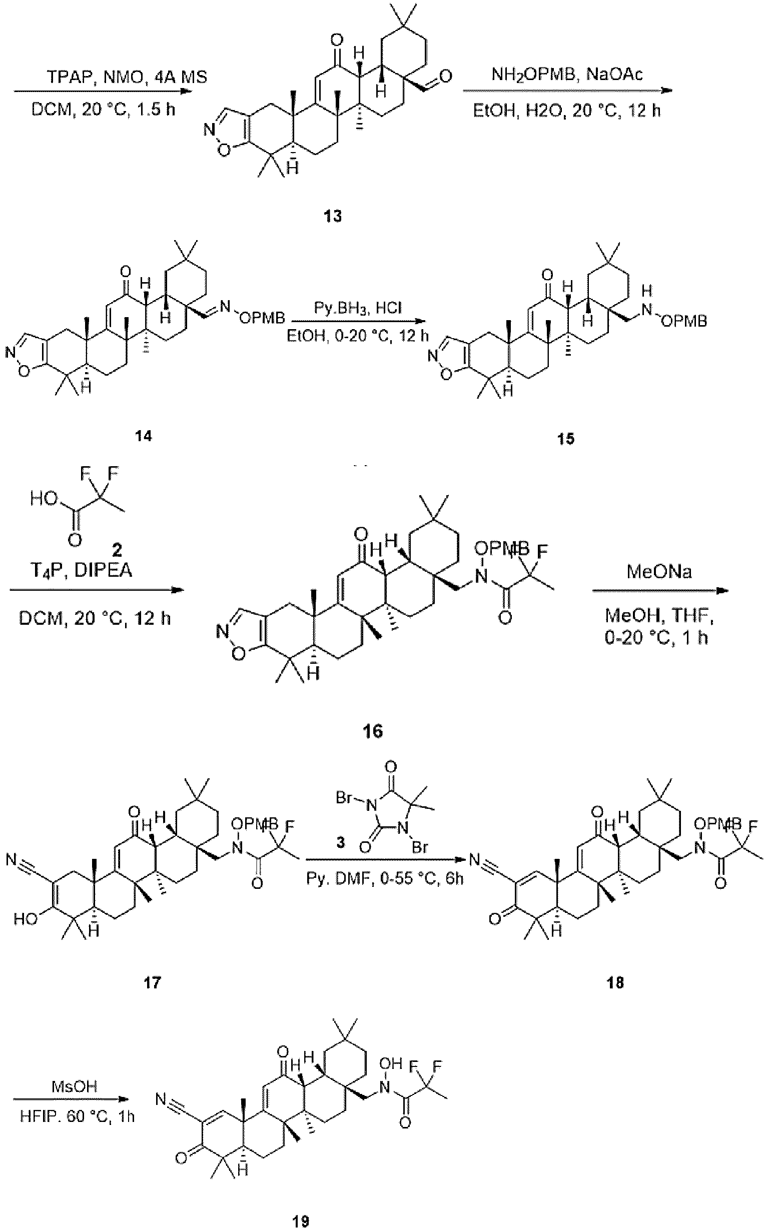

### Step 1: Synthesis of Intermediate 2

Benzyl bromide (23.88 g, 136.85 mmol, 16.59 mL, 98% purity, 1.25 eq) and potassium carbonate (22.70 g, 164.22 mmol, 1.5 eq) were added to a solution of starting material 1 (50 g, 109.48 mmol, 1 eq) in N,N-dimethylformamide (1 L). The mixture was stirred at 20 °C for 12 hours. The reaction was monitored by TLC (petroleum ether/ethyl acetate = 3:1) until the starting material was completely consumed and the product spot was observed. Water (4 L) was added to the reaction mixture and the mixture was extracted three times with ethyl acetate (2 L). The organic phase was washed twice with saturated brine (2 L), dried, and concentrated under reduced pressure to obtain a crude product. The crude product was purified by column chromatography (ethyl acetate/petroleum ether = 10-30%) to obtain Intermediate **2** (50 g, 91.44 mmol, yield 83.52%, purity 90%), as a pale yellow solid. **¹H NMR** (400 MHz, CHLOROFORM-d) δ = 7.35 (s, 5H), 5.32 - 5.28 (m, 1H), 5.14 - 5.02 (m, 2H), 3.26 - 3.18 (m, 1H), 2.91 (br dd, *J* = 4.0, 13.6 Hz, 1H), 2.04 - 1.93 (m, 1H), 1.86 (dd, *J* = 3.6, 8.8 Hz, 2H), 1.77 - 1.65 (m, 3H), 1.62 - 1.57 (m, 4H), 1.55 - 1.49 (m, 3H), 1.49 - 1.40 (m, 2H), 1.39 - 1.30 (m, 3H), 1.25 (br s, 3H), 1.13 (s, 3H), 1.08 - 1.02 (m, 1H), 0.99 (s, 3H), 0.93 (s, 3H), 0.90 (s, 3H), 0.89 (s, 3H), 0.78 (s, 3H), 0.72 (br d, *J* = 11.6 Hz, 1H), 0.62 (s, 3H).

### Step 2: Synthesis of Intermediate 3

4-Dimethylaminopyridine (1.12 g, 9.14 mmol, 0.1 eq) was added to a solution of Intermediate **2** (50 g, 91.44 mmol, 1 eq) in pyridine (500 mL). The mixture was purged with nitrogen three times and the temperature was lowered to 0°C. Acetic anhydride was slowly added at 0°C and the mixture was stirred at 20°C for 12 hours. TLC (petroleum ether/ethyl acetate = 3/1) showed the formation of a new spot, but the starting material was not completely reacted. 4-Dimethylaminopyridine (1.12 g, 9.14 mmol, 0.1 eq) was added to the reaction mixture, and the mixture was purged with nitrogen three times. The temperature was lowered to 0°C, and acetic anhydride was slowly added at 0°C. The mixture was then stirred at 20°C for 24 hours. TLC (petroleum ether/ethyl acetate = 3/1) showed the starting material was completely reacted and the new spot became more concentrated. The reaction mixture was concentrated under reduced pressure to obtain a solid. Water (1 L) was added to the solid, and the solution was extracted three times with ethyl acetate (1 L). The organic phase was dried and concentrated under reduced pressure to obtain Intermediate **3** (48 g, crude product) as a white solid, which was used directly in the next step. **¹H NMR** (400 MHz, CHLOROFORM-d) δ = 7.35 (s, 5H), 5.29 (t, *J* = 3.6 Hz, 1H), 5.13 - 5.02 (m, 2H), 4.52 - 4.47 (m, 1H), 2.91 (br dd, *J* = 4.0, 13.6 Hz, 1H), 2.05 (s, 3H), 1.86 (dd, *J* = 3.6, 8.8 Hz, 2H), 1.75 - 1.60 (m, 7H), 1.59 - 1.45 (m, 4H), 1.44 - 1.29 (m, 3H), 1.28 - 1.16 (m, 3H), 1.13 (s, 3H), 1.08 - 1.01 (m, 2H), 0.94 - 0.89 (m, 9H), 0.86 (d, *J* = 4.0 Hz, 7H), 0.62 (s, 3H).

### Step 3: Synthesis of Intermediate 4

m-Chloroperoxybenzoic acid (49.65 g, 244.55 mmol, 85% purity, 3 eq) was added to a solution of Intermediate **3** (48 g, 81.50 mmol, 1 eq) in dichloromethane (2 L) in portions. The reaction mixture was stirred at 40 °C for 12 hours. The reaction was monitored by TLC (petroleum ether/ethyl acetate = 5:1) until the starting material was completely consumed and the product spot was observed. Aqueous sodium sulfite solution was slowly added to the reaction mixture at 0 °C until starch-iodide paper no longer turned blue. Water (2 L) was added, and the mixture was extracted with dichloromethane (1 L) three times. The organic phase was dried and concentrated under reduced pressure to obtain a crude product, which was purified by column chromatography (ethyl acetate/petroleum ether = 1 - 5%) to obtain Intermediate **4** (50 g, 70.26 mmol, yield 86.20%, purity 85%) as a white solid. **¹H NMR** (400 MHz, CHLOROFORM-d) δ = 7.40 - 7.33 (m, 5H), 5.26 - 5.20 (m, 1H), 5.14 - 5.07 (m, 1H), 4.49 (dd, *J* = 5.2, 11.6 Hz, 1H), 2.92 - 2.81 (m, 1H), 2.48 (d, *J* = 4.4 Hz, 1H), 2.26 - 2.18 (m, 1H), 2.08 (s, 3H), 1.97 - 1.83 (m, 3H), 1.75 - 1.43 (m, 10H), 1.43 - 1.32 (m, 3H), 1.28 - 1.20 (m, 3H), 1.06 (br d, *J* = 4.0 Hz, 1H), 1.02 (s, 3H), 1.01 - 0.95 (m, 2H), 0.93 (s, 6H), 0.88 (d, *J* = 1.2 Hz, 6H), 0.85 (s, 3H), 0.68 - 0.61 (m, 3H).

### Step 4: Synthesis of Intermediate 5

Intermediate **4** (20 g, 33.07 mmol, 1 eq) was dissolved in acetic acid (100 mL). A solution of hydrobromic acid in acetic acid (3.24 g, 13.23 mmol, 2.18 mL, 33% purity, 0.4 eq) was added dropwise at room temperature, and the mixture was heated to 50 °C. Liquid bromine (6.34 g, 39.68 mmol, 2.04 mL, 1.2 eq) was added slowly under heating. After addition was complete, the mixture was stirred at 50 °C for 12 hours. The reaction was monitored by LC-MS, which showed that the starting materials were completely consumed and that products were present. The reaction mixture was diluted with water (500 mL), quenched with 20% aqueous sodium thiosulfate solution (50 mL), and extracted with dichloromethane (200 mL*3). The organic layers were combined, washed with saturated brine, dried over anhydrous sodium sulfate, filtered, and concentrated to obtain a crude product. The crude product was purified by column chromatography (ethyl acetate/petroleum ether = 0 - 26%) to obtain Intermediate **5** (7.89 g, 11.52 mmol, yield 34.83%) as a yellow solid. **LCMS**: rt = 0.869 min, 603.3 [M+H]⁺.

### Step 5: Synthesis of Intermediate 6

Intermediate **5** (35.6 g, 59.05 mmol, 1 eq) was dissolved in anhydrous methanol (300 mL), potassium hydroxide (23.19 g, 413.38 mmol, 7 eq) was added, the reaction mixture was heated to 70°C and stirred for 4 hours. The reaction was monitored by LC-MS, which showed that the starting materials were completely consumed and that products were present. The pH of the reaction mixture was adjusted to 2-3 with 6N HCl in an ice-water bath, then diluted with water (400 mL) and filtered. The filter cake was washed with water (300 mL*3) and then dried under reduced pressure to obtain Intermediate **6** (30.9 g, crude product) as a yellow solid. **LCMS**: rt = 0.789 min, 561.4 [M+H]⁺.

### Step 6: Synthesis of Intermediate 7

Intermediate **6** (30.9 g, 55.10 mmol, 1 eq) was dissolved in acetonitrile (600mL) and 2-iodobenzoic acid (30.86 g, 110.20 mmol, 2 eq) was added. The reaction mixture was heated to 80°C and stirred for 1 hour. The reaction was monitored by LC-MS, which showed that the starting materials were completely consumed and that products were present. The reaction mixture was filtered, and the filter cake was washed with ethyl acetate (400 mL*3). The filtrate was concentrated to obtain Intermediate **7** (30.79 g, crude product) as a yellow solid. **LCMS**: rt = 0.816 min, 559.4 [M+H]⁺.

### Step 7: Synthesis of Intermediate 8

Intermediate **7** (30.79 g, 55.10 mmol, 1 eq) was dissolved in anhydrous dichloromethane (300 mL), and ethyl formate (16.33 g, 220.41 mmol, 17.73 mL, 4 eq) and sodium methoxide (17.86 g, 330.61 mmol, 6 eq) were added sequentially. The reaction mixture was stirred at room temperature for 2 hours. The reaction was monitored by LC-MS, which showed that the starting materials were completely consumed and that products were present. The reaction mixture was filtered, and the filter cake was washed with dichloromethane (500 mL*3). The filtrate was concentrated to obtain a crude product, which was purified by column chromatography (ethyl acetate/petroleum ether = 0-15%) to obtain Intermediate **8** (30.47 g, crude product) as a yellow solid. **LCMS**: rt = 0.873 min, 587.3 [M+H]⁺.

### Step 8: Synthesis of Intermediate 9

Intermediate **8** (1 g, 1.70 mmol, 1 eq) was dissolved in ethanol (10 mL) and water (1 mL), and hydroxylamine hydrochloride (1.18 g, 17.04 mmol, 10 eq) was added. The reaction mixture was heated to 100 °C and stirred for 1 hour. The reaction was monitored by LC-MS, which showed that the starting materials were completely consumed and that products were present. The reaction mixture was concentrated, diluted with water (20 mL), and then extracted with dichloromethane (20 mL*3). The organic layers were combined, washed with saturated brine, dried over anhydrous sodium sulfate, filtered and concentrated to obtain a crude product. The crude product was purified by column chromatography (dichloromethane/petroleum ether = 0-88%) to obtain Intermediate **9** (580 mg, 993.49 µmol, yield 58.30%) as a white solid. **LCMS**: rt = 0.822 min, 584.4 [M+H]⁺. ¹H NMR (400 MHz, CHLOROFORM-d) δ = 8.07 (s, 1H), 7.40 - 7.28 (m, 5H), 5.86 (s, 1H), 5.16 (d, J = 4.8 Hz, 2H), 3.15 - 3.05 (m, 1H), 2.83 (d, J = 4.6 Hz, 1H), 2.77 (d, J = 15.0 Hz, 1H), 2.38 (d, J = 15.0 Hz, 1H), 1.96 - 1.84 (m, 2H), 1.78 - 1.52 (m, 9H), 1.50 - 1.32 (m, 6H), 1.26 (s, 3H), 1.13 (s, 4H), 1.02 (s, 3H), 1.00 (s, 3H), 0.96 (s, 3H), 0.90 (s, 3H).

### Step 9: Synthesis of Intermediate 10

Intermediate **9** (20.08 g, 34.40 mmol, 1 eq) was dissolved in anhydrous tetrahydrofuran (300 mL) and wet palladium on carbon (4.09 g, 3.84 mmol, purity 10%, 1.12 e-1 eq) was added. The reaction mixture was stirred at room temperature for 2 hours under hydrogen balloon protection. The reaction was monitored by LC-MS, which showed that the starting materials were completely consumed and that products were present. The reaction mixture was filtered, and the filter cake was washed with dichloromethane (500 mL*3). The filtrate was concentrated to obtain a crude product, which was purified by column chromatography (petroleum ether/ethyl acetate = 0-31%) to obtain Intermediate **10** (9.8 g, 17.07 mmol, yield 49.63%) as a white solid. LCMS: rt = 0.684 min, 494.3 [M+H]⁺. ¹H NMR (400 MHz, CHLOROFORM-d) δ = 8.07 (s, 1H), 5.90 (s, 1H), 3.09 - 2.98 (m, 2H), 2.79 (d, J = 15.0 Hz, 1H), 2.42 (d, J = 14.8 Hz, 1H), 2.00 - 1.90 (m, 2H), 1.83 - 1.69 (m, 5H), 1.67 - 1.60 (m, 2H), 1.59 - 1.48 (m, 3H), 1.46 - 1.39 (m, 1H), 1.36 (s, 3H), 1.32 (s, 3H), 1.29 - 1.28 (m, 1H), 1.27 (s, 3H), 1.23 (br t, J = 4.4 Hz, 1H), 1.17 (s, 3H), 1.05 (s, 3H), 1.03 (s, 3H), 0.92 (s, 3H).

### Step 10: Synthesis of Intermediate 11

Intermediate **10** (7 g, 14.18 mmol, 1 eq) was dissolved in acetone (70 mL), and potassium carbonate (5.88 g, 42.54 mmol, 3 eq) and iodomethane (6.04 g, 42.54 mmol, 2.65 mL, 3 eq) were added. The reaction mixture was stirred at room temperature for 12 hours. The reaction was monitored by LC-MS, which showed that the starting materials were completely consumed and that products were present. The reaction mixture was filtered, and the filter cake was washed with dichloromethane (100 mL*3). The filtrate was concentrated to obtain a crude product, which was purified by column chromatography (petroleum ether/ethyl acetate = 0-30%) to obtain Intermediate **11** (6.3 g, 11.42 mmol, yield 80.51%) as a white solid. **LCMS**: rt = 0.774 min, 508.2 [M+H]⁺. ¹H NMR (400 MHz, CHLOROFORM-d) δ = 8.07 (s, 1H), 5.90 (s, 1H), 3.71 (s, 3H), 3.06 (td, J = 3.6, 13.4 Hz, 1H), 2.95 (d, J = 4.8 Hz, 1H), 2.79 (d, J = 15.0 Hz, 1H), 2.41 (d, J = 15.0 Hz, 1H), 1.97 - 1.82 (m, 2H), 1.81 - 1.66 (m, 5H), 1.66 - 1.60 (m, 1H), 1.57 (s, 3H), 1.55 - 1.47 (m, 3H), 1.39 - 1.34 (m, 3H), 1.30 (s, 3H), 1.28 (s, 3H), 1.23 (br d, J = 2.6 Hz, 1H), 1.17 (s, 3H), 1.04 (s, 3H), 1.02 (s, 3H), 0.91 (s, 3H).

### Step 11: Synthesis of Intermediate 12

Intermediate **11** (957 mg, 1.88 mmol, 1 eq) was dissolved in anhydrous tetrahydrofuran (10 mL). Diisobutylaluminum hydride (1 M, 9.42 mL, 5 eq) was slowly added dropwise under an ice-water bath. After the addition was complete, the mixture was stirred under an ice-water bath for 0.5 hours. Then the reaction mixture was slowly raised to room temperature and stirred for 2 hours. The reaction was monitored by LC-MS, which showed that the starting materials were completely consumed and that products were present. The reaction mixture was quenched by slowly adding water (10 mL) dropwise under an ice-water bath. Then, 1N HCl (50 mL) was added for acidification and the solution was extracted with ethyl acetate (50 mL*4). The organic layers were combined and washed successively with water and saturated brine, dried over anhydrous sodium sulfate, filtered and concentrated to obtain Intermediate **12** (970 mg, crude product) as a white solid. **LCMS**: rt = 0.713 min, 464.2 [M-OH]⁺.

### Step 12: Synthesis of Intermediate 13

Intermediate **12** (970 mg, 2.01 mmol, 1 eq) was dissolved in anhydrous dichloromethane (20 mL), and 4A molecular sieve (2 g) and N-methylmorpholine oxide (518.96 mg, 4.43 mmol, 467.54 µL, 2.2 eq) were added. The reaction mixture was reacted at room temperature for 10 min under nitrogen protection. Then, tetrapropylammonium perruthenate (70.77 mg, 201.37 µmol, 0.1 eq) was added to the reaction mixture, and the reaction mixture was stirred at room temperature for 1.5 hours. The reaction was monitored by LC-MS, which showed that the starting materials were completely consumed and that products were present. The reaction mixture was quenched with saturated aqueous sodium carbonate solution (50 mL), followed by extraction with dichloromethane (50 mL*3). The organic layers were combined and washed successively with water and saturated brine, dried over anhydrous sodium sulfate, filtered and concentrated to obtain a crude product. The crude product was purified by column chromatography (ethyl acetate/petroleum ether = 0-22%) to obtain Intermediate **13** (647 mg, 1.19 mmol, 59.19% yield) as a white solid. **LCMS**; rt = 0.743 min, 478.3 [M+H]⁺.

### Step 13: Synthesis of Intermediate 14

Intermediate **13** (300 mg, 628.04 µmol, 1 eq) was dissolved in anhydrous ethanol (3 mL) and water (0.2 mL), and p-methoxybenzyloxyamine hydrochloride (154.83 mg, 816.45 µmol, 1.3 eq) and sodium acetate (92.74 mg, 1.13 mmol, 1.8 eq) were added. The reaction mixture was stirred at room temperature for 12 hours. The reaction was monitored by LC-MS, which showed that the starting materials were completely consumed and that products were present. The reaction mixture was diluted with water (20 mL), and then extracted with dichloromethane (20 mL*3). The organic layers were combined, washed with saturated brine, dried over anhydrous sodium sulfate, filtered and concentrated to obtain Intermediate **14** (384 mg, crude) as a white solid. **LCMS**: rt = 0.846 min, 613.3 [M+H]⁺.

### Step 14: Synthesis of Intermediate 15

Intermediate **14** (384 mg, 626.59 µmol, 1 eq) was dissolved in anhydrous ethanol (10 mL). Pyridine borane (174.69 mg, 1.88 mmol, 188.04 µL, 3 eq) and 10% aqueous hydrochloric acid solution (2.19 g, 6.02 mmol, 2.15 mL, 10% purity, 9.6 eq) were slowly added dropwise under an ice-water bath. After the addition was complete, the reaction mixture was brought to room temperature and stirred for 12 hours. The reaction was monitored by LC-MS, which showed the remaining starting material and the presence of product. The reaction mixture was adjusted to alkaline pH with saturated aqueous sodium bicarbonate solution, and then extracted with dichloromethane (20 mL*3). The organic layers were combined, washed with saturated brine, dried over anhydrous sodium sulfate, filtered and concentrated to obtain a crude product. The crude product was purified by column chromatography (ethyl acetate/petroleum ether = 0-15%) to obtain Intermediate **15** (280 mg, 318.77 µmol, 50.87% yield) as a colorless gel. **LCMS**: rt = 0.754 min, 615.3 [M+H]⁺.

### Step 15: Synthesis of Intermediate 16

Intermediate **15** (280 mg, 455.39 µmol, 1 eq) was dissolved in anhydrous dichloromethane (4 mL), and then starting material 2 (150.36 mg, 1.37 mmol, 3 eq), N,N-diisopropylethylamine (588.55 mg, 4.55 mmol, 793.19 µL, 10 eq) and n-butylphosphine anhydride (50% ethyl acetate solution) (984.36 mg, 1.37 mmol, 50% purity, 3 eq) were added sequentially. The reaction mixture was stirred at room temperature for 12 hours. The reaction was monitored by LC-MS, which showed a small amount of starting material remaining and the presence of product. The reaction mixture was concentrated to obtain a crude product, which was purified by column chromatography (petroleum ether/ethyl acetate = 0-20%) to obtain Intermediate **16** (179 mg, 230.43 µmol, 50.60% yield) as a colorless gel. **LCMS**: rt = 0.786 min, 707.3 [M+H]⁺.

### Step 16: Synthesis of Intermediate 17

Intermediate **16** (179 mg, 253.22 µmol, 1 eq) was dissolved in anhydrous methanol (1 mL) and tetrahydrofuran (1 mL). A 30% solution of sodium methoxide in methanol (227.98 mg, 1.27 mmol, 30% purity, 5 eq) was added under ice-water bath. The reaction mixture was brought to room temperature and stirred for 1 hour. The reaction was monitored by LC-MS, which showed a small amount of starting material remaining and the presence of product. The reaction mixture was diluted with 1N aqueous hydrochloric acid solution (10 mL), and then extracted with dichloromethane (10 mL*3). The organic layers were combined, washed with saturated brine, dried over anhydrous sodium sulfate, filtered and concentrated to obtain Intermediate **17** (179 mg, crude) as a colorless gel. **LCMS**: rt = 0.733 min, 729.4 [M+Na]⁺.

### Step 17: Synthesis of Intermediate 18

Intermediate **17** (179 mg, 253.22 µmol, 1 eq) was dissolved in anhydrous N,N-dimethylformamide (2 mL). Starting material 3 (36.20 mg, 126.61 µmol, 0.5 eq) was added under ice-water bath, and the reaction mixture was kept under ice-water bath for 2 hours. Then pyridine (80.12 mg, 1.01 mmol, 81.75 µL, 4 eq) was added, and the reaction mixture was heated to 55 °C and stirred for 4 hours. The reaction was monitored by LC-MS, which showed a small amount of starting material remaining and the presence of product. The reaction mixture was diluted with water (10 mL), then extracted with ethyl acetate (10 mL*3), the organic layers were combined, washed with saturated brine, dried over anhydrous sodium sulfate, filtered and concentrated to obtain a crude product, which was purified by column chromatography (ethyl acetate/petroleum ether = 0-20%) to obtain Intermediate **18** (130 mg, 184.43 µmol, 72.83% yield) as a white solid. **LCMS**: rt = 0.743 min, 727.2 [M+Na]⁺.

### Step 18: Synthesis of Compound 19

Intermediate **18** (125 mg, 177.33 µmol, 1 eq) was dissolved in hexafluoroisopropanol (1.5 mL) and methanesulfonic acid (170.44 mg, 1.77 mmol, 126.72 µL, 10 eq) was added, and the mixture was heated to 60 °C and reacted for 1 hour. The reaction was monitored by LC-MS, which showed that the starting materials were completely consumed and that products were present. The reaction mixture was quenched with saturated sodium bicarbonate (10 mL), followed by extraction with dichloromethane (10 mL*3). The combined organic layers were combined, washed with saturated brine, dried over anhydrous sodium sulfate, filtered, and concentrated to obtain a crude product. The crude product was then purified by reverse phase preparative chromatography and lyophilized to obtain Compound **19** (58.01 mg, 95.22 µmol, 53.69% yield) as a white solid. **Prep-HPLC:** column: Phenomenex Luna C₁₈ 150*25mm*10µm; mobile phase: [H₂O(0.225% FA)-ACN]; gradient: 48%-78% B over 15.0min. **LCMS**: rt = 0.654 min, 585.3 [M+H]⁺. **HPLC**: rt = 3.863 min. **¹H NMR** (400 MHz, DMSO-d6) δ = 10.17 (s, 1H), 8.66 (s, 1H), 6.22 (s, 1H), 3.77 (br d, *J* = 14.4 Hz, 1H), 3.51 (br d, *J* = 14.8 Hz, 1H), 3.19 (br d, *J* = 4.6 Hz, 1H), 2.31 - 2.22 (m, 1H), 1.99 - 1.77 (m, 8H), 1.76 - 1.60 (m, 2H), 1.53 - 1.39 (m, 7H), 1.38 - 1.20 (m, 4H), 1.18 (s, 3H), 1.16 - 1.10 (m, 2H), 1.08 (s, 3H), 1.06 - 0.99 (m, 1H), 0.93 (s, 3H), 0.84 (d, *J* = 8.4 Hz, 6H).

### Example 19

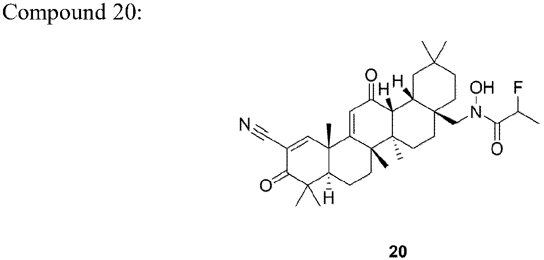

### Synthetic Route:

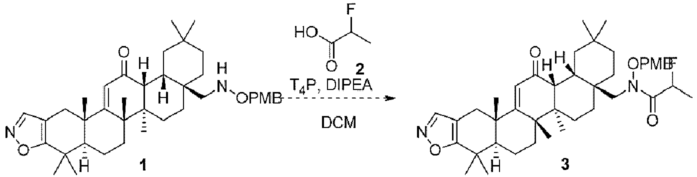

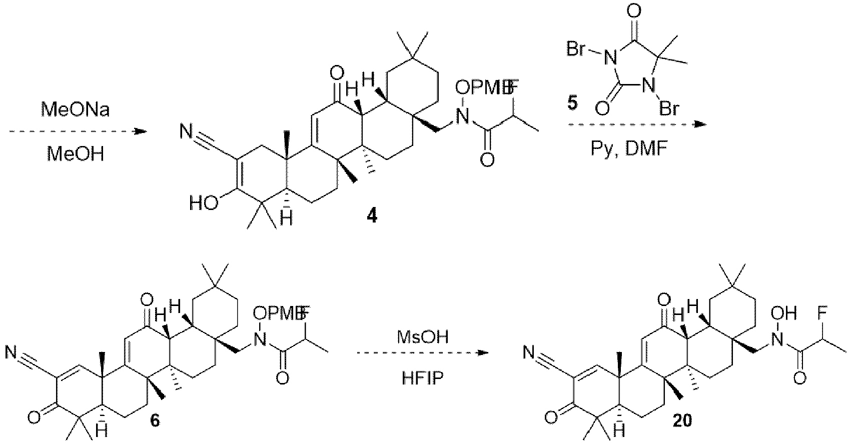

### Example 20

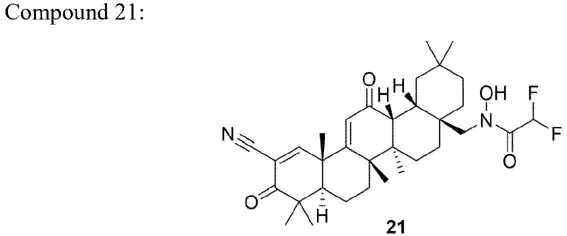

### Synthetic Route:

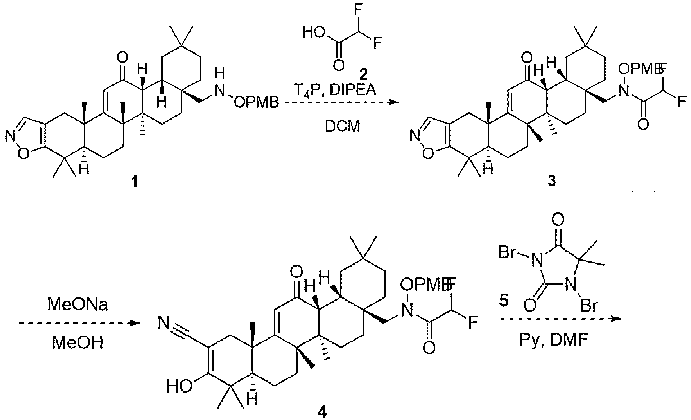

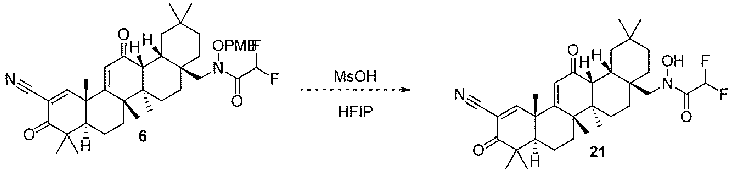

### Example 21

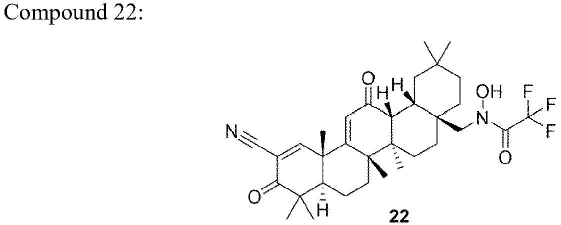

### Synthetic Route:

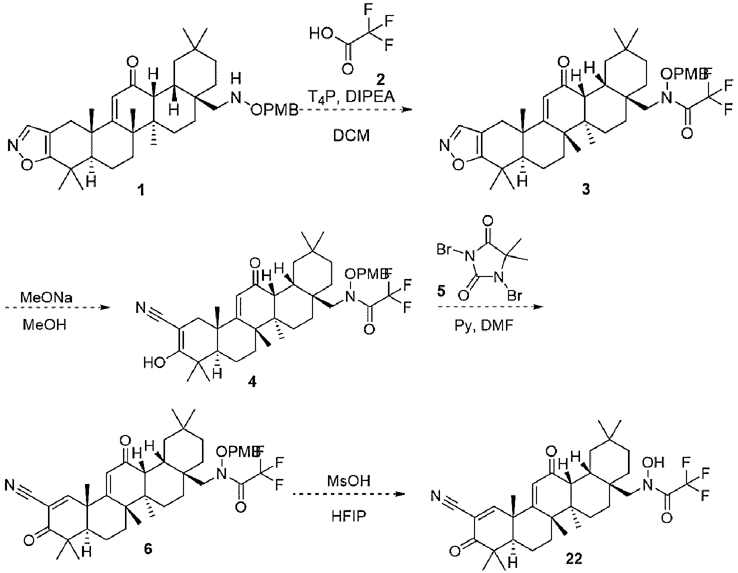

### Example 22

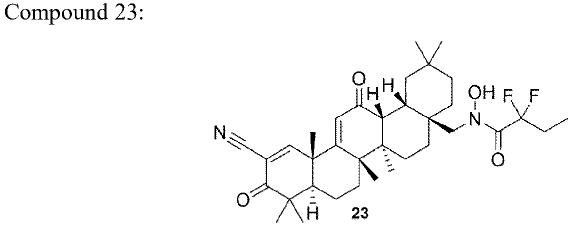

### Synthetic Route:

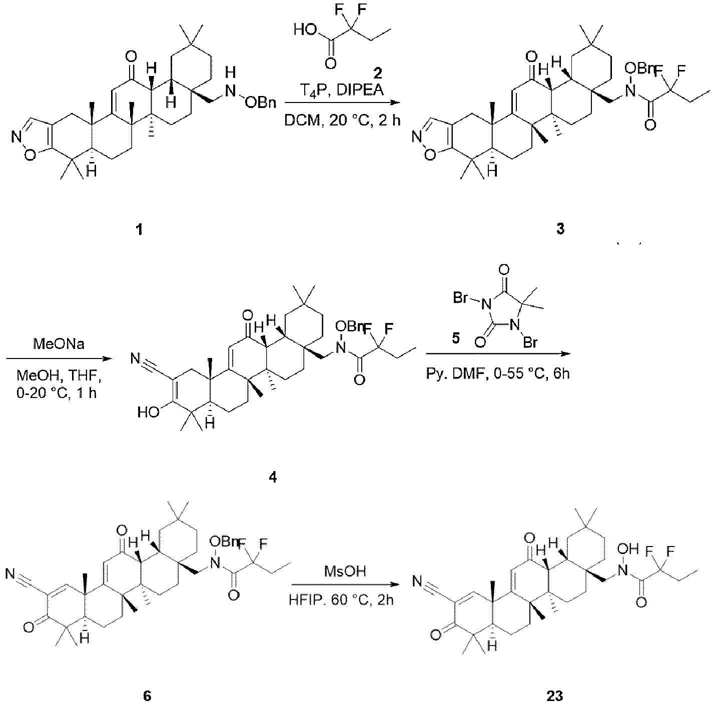

### Step 1: Synthesis of Intermediate 3

Starting material 1 (916 mg, 1.57 mmol, 1 eq) was dissolved in anhydrous dichloromethane (10 mL), and then starting material 2 (583.06 mg, 4.70 mmol, 3 eq), N,N-diisopropylethylamine (2.02 g, 15.66 mmol, 2.73 mL, 10 eq), and n-butylphosphine (50% ethyl acetate solution) (3.39 g, 4.70 mmol, 50% purity, 3 eq) were added sequentially. The reaction mixture was stirred at room temperature for 2 hours. The reaction was monitored by LC-MS, which showed that the starting materials were completely consumed and that products were present. The reaction mixture was concentrated to obtain a crude product, which was purified by column chromatography (ethyl acetate/petroleum ether = 0-21%) to obtain Compound 3 (980 mg, 1.38 mmol, 87.84% yield) as a white solid. **LCMS:** rt = 0.834 min, 691.3 [M+H]⁺. **¹H NMR** (400 MHz, CHLOROFORM-d) δ = 8.09 (s, 1H), 7.47 - 7.33 (m, 5H), 5.91 (s, 1H), 4.90 (s, 2H), 3.22 (br s, 1H), 2.81 (d, *J =* 15.0 Hz, 1H), 2.47 - 2.32 (m, 2H), 2.31 - 2.15 (m, 2H), 2.15 - 2.07 (m, 1H), 2.05 - 1.90 (m, 2H), 1.85 - 1.70 (m, 3H), 1.65 - 1.59 (m, 2H), 1.57 - 1.47 (m, 5H), 1.41 - 1.34 (m, 4H), 1.31 (s, 4H), 1.25 (br d, *J* = 7.0 Hz, 1H), 1.21 (s, 3H), 1.19 - 1.07 (m, 5H), 1.07 - 1.00 (m, 5H), 0.93 (s, 3H), 0.88 (s, 3H).

### Step 2: Synthesis of Intermediate 4

Intermediate **3** (880 mg, 1.27 mmol, 1 eq) was dissolved in anhydrous methanol (5 mL) and tetrahydrofuran (5 mL). A 30% solution of sodium methoxide in methanol (1.15 g, 6.37 mmol, 30% purity, 5 eq) was added under an ice-water bath. The reaction mixture was brought to room temperature and stirred for 1 hour. The reaction was monitored by LC-MS, which showed a small amount of starting material remaining and the presence of product. The reaction mixture was diluted with 1N aqueous hydrochloric acid solution (30 mL), followed by extraction with dichloromethane (30 mL*3). The organic layers were combined, washed with saturated brine, dried over anhydrous sodium sulfate, filtered, and concentrated to obtain Intermediate **4** (880 mg, crude) as a white solid. **LCMS**: rt = 0.759 min, 691.4 [M+H]⁺.

### Step 3: Synthesis of Intermediate 6

Intermediate **4** (880 mg, 1.27 mmol, 1 eq) was dissolved in anhydrous N,N-dimethylformamide (10 mL). Starting material **5** (182.09 mg, 636.85 µmol, 0.5 eq) was added under an ice-water bath, and the reaction mixture was then kept under an ice-water bath for 2 hours. Pyridine (403.00 mg, 5.09 mmol, 411.22 µL, 4 eq) was then added, and the reaction mixture was heated to 55 °C and stirred for 4 hours. The reaction was monitored by LC-MS, which showed the remaining starting material and the presence of product. The reaction mixture was diluted with water (20 mL), then extracted with ethyl acetate (20 mL*3). The organic layers were combined, washed with saturated brine, dried over anhydrous sodium sulfate, filtered and concentrated to obtain a crude product. The crude product was purified by column chromatography (ethyl acetate/petroleum ether = 0-19%) to obtain Intermediate **6** (796 mg, 1.11 mmol, 87.09% yield) as a white solid. **LCMS**: rt = 0.769 min, 689.3 [M+H]⁺.

### Step 4: Synthesis of Compound 23

Intermediate **6** (640 mg, 929.04 µmol, 1 eq) was dissolved in hexafluoroisopropanol (10 mL), and methanesulfonic acid (1.34 g, 13.94 mmol, 995.80 µL, 15 eq) was added. The mixture was heated to 60 °C and reacted for 2 hours. The reaction was monitored by LC-MS, which showed the remaining starting material and the presence of product. The reaction mixture was slowly added to a stirred saturated aqueous sodium bicarbonate solution (20 mL), followed by extraction with dichloromethane (20 mL*3). The organic layers were combined, washed with saturated brine, dried over anhydrous sodium sulfate, filtered and concentrated to obtain a crude product. The crude product was purified by column chromatography (ethyl acetate/petroleum ether = 0-28%) and then purified by reverse phase preparative chromatography and lyophilized to obtain Compound **23** (65.2 mg, 106.62 µmol, yield 11.48%) as a white solid. **Prep-HPLC:** column: Phenomenex Luna C₁₈ 150*25mm*10µm; mobile phase: [H₂O(0.225% FA)-ACN]; gradient:58%-88% B over 15.0 min. **LCMS**: rt = 0.678 min, 599.3 [M+H]⁺. **HPLC**: rt = 4.015 min. **¹H NMR** (400 MHz, DMSO-d6) δ = 10.14 (s, 1H), 8.66 (s, 1H), 6.21 (s, 1H), 3.82 (br d, *J* = 14.4 Hz, 1H), 3.47 (br d, *J* = 14.4 Hz, 1H), 3.23 (br d, *J* = 4.4 Hz, 1H), 2.29 - 2.12 (m, 3H), 1.97 - 1.79 (m, 5H), 1.76 - 1.62 (m, 2H), 1.47 (d, *J =* 8.6 Hz, 7H), 1.38 - 1.20 (m, 4H), 1.18 (s, 3H), 1.14 (br s, 1H), 1.08 (s, 3H), 1.07 - 0.98 (m, 2H), 0.97 - 0.91 (m, 6H), 0.84 (d, *J =* 7.8 Hz, 6H).

### Example 23

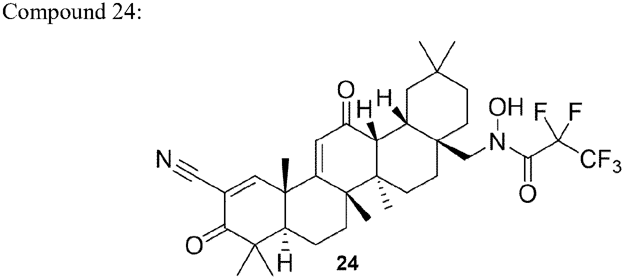

### Synthetic Route:

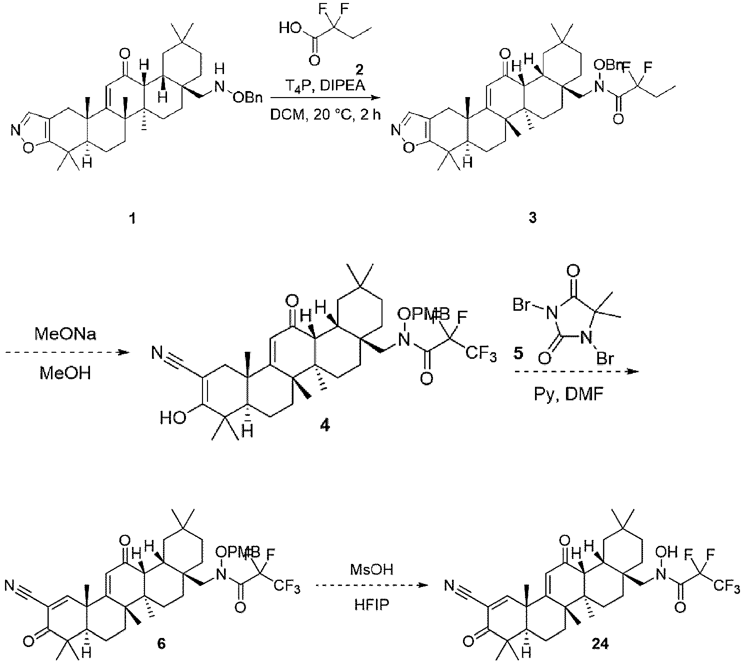

### Example 24

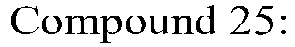

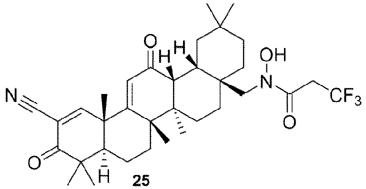

### Synthetic Route:

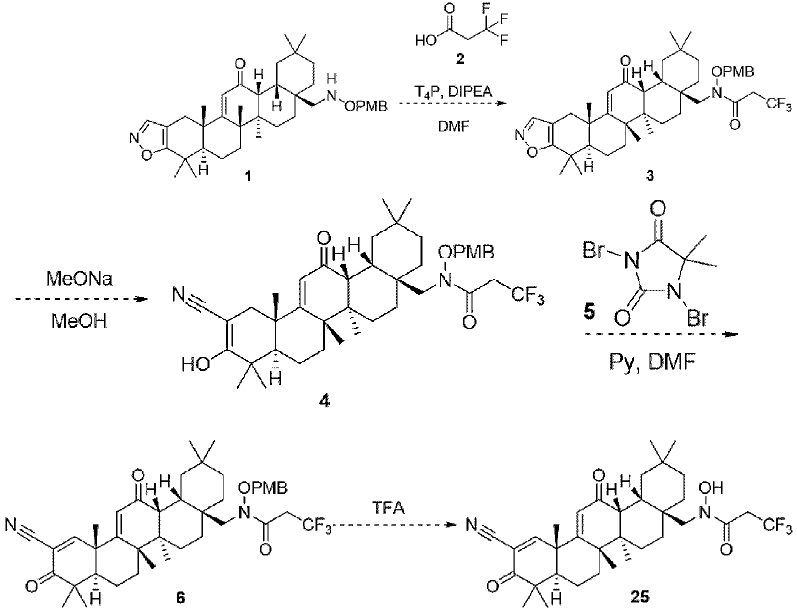

### Example 25

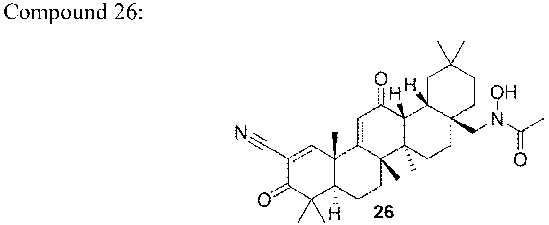

### Synthetic Route:

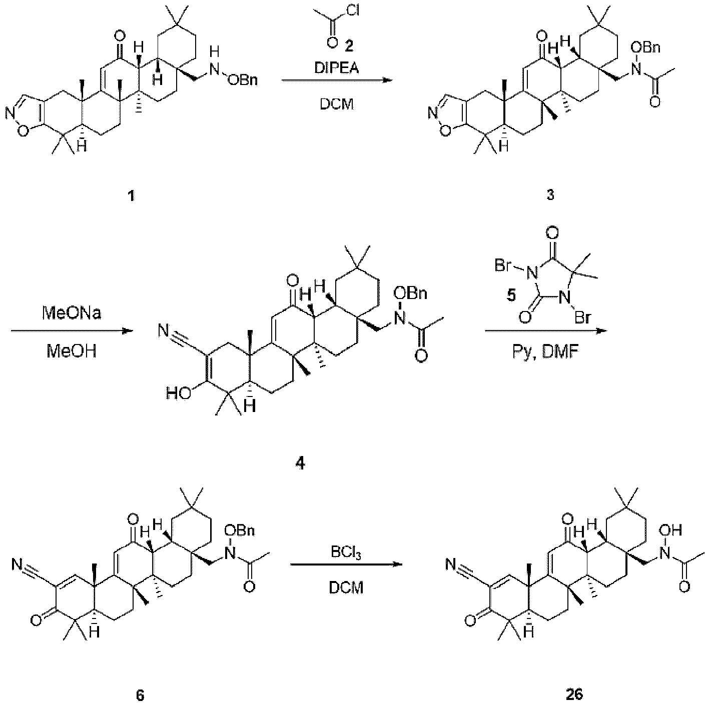

### Step 1: Synthesis of Intermediate 3

N,N-diisopropylethylamine (1.33 g, 10.26 mmol, 1.79 mL, 3 eq) was added to a solution of starting material 1 (2 g, 3.42 mmol, 1 eq) in dichloromethane (20 mL), and then a solution of starting material 2 (402.67 mg, 5.13 mmol, 364.74 µL, 1.5 eq) in dichloromethane (20mL) was added at 0°C. The reaction mixture was reacted at 20°C for 2 hours. The reaction was monitored by LC-MS, which showed that the starting materials were completely consumed and the main peak corresponded to the desired product. The reaction mixture was concentrated under reduced pressure to obtain a crude product, and the crude product was purified by normal phase silica gel column chromatography (petroleum ether: ethyl acetate = 1:0 to 3:1) and then concentrated under reduced pressure to obtain Intermediate **3** (1.3 g, 1.85 mmol, 53.97% yield, 89% purity), as a white solid. **LCMS**: Rt = 0.766 min, 627.5 [M+H]⁺ ESI.

### Step 2: Synthesis of Intermediate 4

Sodium methoxide solution (1.87 g, 10.37 mmol, 30% purity, 5 eq) was slowly added to a solution of Intermediate 3 (1.3 g, 2.07 mmol, 1 eq) in anhydrous methanol (20 mL) at 0 °C, and the reaction mixture was reacted at 20 °C for 1 hour. The reaction was monitored by LC-MS, which showed that the main peak was the desired product. The reaction mixture was slowly poured into 1N HCl solution to adjust the pH to 3, and then extracted with ethyl acetate (20 mL*2). The combined organic phases were washed with saturated brine (20 mL), dried over anhydrous sodium sulfate, and concentrated under reduced pressure to obtain Intermediate 4 (1.3 g, crude product), as a white solid. **LCMS**: Rt = 0.706 min, 627.5 [M+H]⁺ ESI.

### Step 3: Synthesis of Intermediate 6

Starting material **5** (296.47 mg, 1.04 mmol, 0.5 eq) was slowly added to a solution of Intermediate **4** (1.3 g, 2.07 mmol, 1 eq) in N,N-dimethylformamide (15 mL) at 0 °C. The reaction mixture was reacted at 0 °C for 2 hours, and then pyridine (656.15 mg, 8.30 mmol, 669.54 µL, 4 eq) was slowly added. The reaction mixture was heated to 55 °C and reacted for 4 hours. The reaction was monitored by LC-MS, which showed that the main peak was the desired product. The reaction mixture was poured into water (20 mL) and extracted with ethyl acetate (20 mL*2). The combined organic phases were washed with saturated brine (20 mL), dried over anhydrous sodium sulfate, concentrated under reduced pressure to obtain a crude product, and the crude product was purified by normal phase silica gel column chromatography (petroleum ether: ethyl acetate = 1:0 to 3:1) and concentrated under reduced pressure to obtain Intermediate **6** (1 g, 1.50 mmol, 72.54% yield, 94% purity), as a white solid. **LCMS**: Rt = 0.718 min, 625.5 [M+H]⁺ ESI.

### Step 4: Synthesis of Compound 26

Boron trichloride solution (1 M, 7.20 mL, 5 eq) was slowly added to a solution of Intermediate 6 (900 mg, 1.44 mmol, 1 eq) in dichloromethane (20 mL) at -70°C, and the reaction mixture was reacted at 35°C for 4 hours. The reaction was monitored by LC-MS, which showed that the starting materials were completely consumed and the main peak corresponded to the desired product. The reaction mixture was slowly poured into a saturated aqueous sodium bicarbonate solution (20 mL) to adjust the pH to 8, and extracted with dichloromethane (20 mL*2). The combined organic phases were washed with saturated brine (20 mL), dried over anhydrous sodium sulfate, concentrated under reduced pressure to obtain a crude product, and the crude product was separated and purified by reverse phase preparative chromatography [water (formic acid)-acetonitrile system] to obtain Compound 26 (96.37 mg, 164.00 µmol, 11.39% yield, 91% purity), as a yellow solid. **Prep-HPLC** column: Phenomenex luna C₁₈ 150*40mm* 15µm; mobile phase: [H₂O(0.225% FA)-ACN]; gradient: 50%-80% B over 15.0 min. **LCMS:** rt = 0.634 min, 535.5 [M+H]⁺. **HPLC**: rt = 2.803 min; **¹H NMR** (400 MHz, DMSO-d6) δ = 9.69 (s, 1H), 8.66 (s, 1H), 6.21 (s, 1H), 3.66 - 3.55 (br m, 1H), 3.46 - 3.35 (m, 1H), 3.30 -3.20 (m, 1H), 2.24 - 2.16 (m, 1H), 2.06 - 1.91 (m, 5H), 1.89 - 1.79 (m, 4H), 1.76 - 1.61 (m, 2H), 1.50 - 1.42 (m, 6H), 1.38 - 1.26 (m, 3H), 1.20 - 1.16 (m, 4H), 1.12 (br s, 1H), 1.08 (s, 3H), 1.04 - 0.96 (m, 2H), 0.93 (s, 3H), 0.85 (d, *J* = 12.0 Hz, 6H).

### Example 26

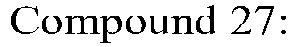

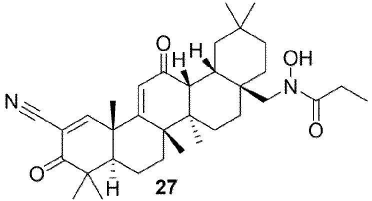

### Synthetic Route:

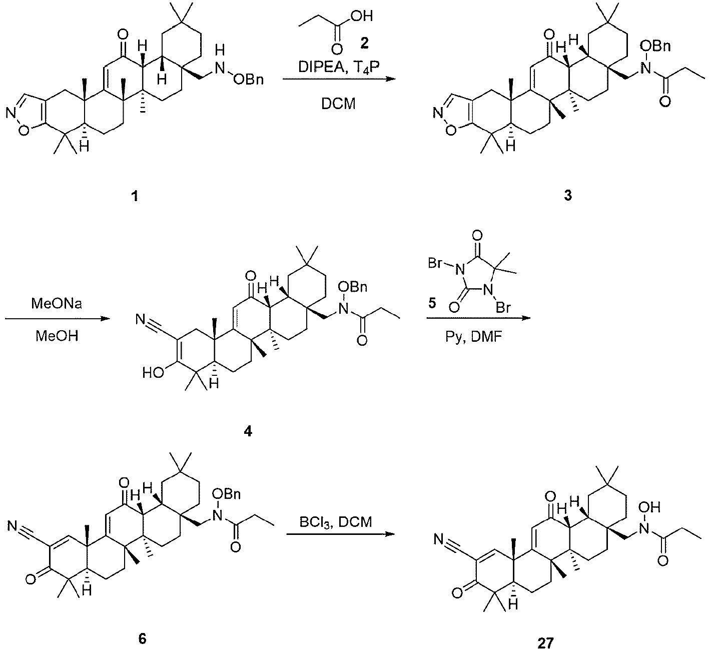

### Step 1: Synthesis of Intermediate 3

N,N-diisopropylethylamine (4.20 g, 32.49 mmol, 5.66 mL, 10 eq) and T₄P (7.02 g, 9.75 mmol, 50% purity, 3 eq) were added to a solution of starting material **1** (1.9 g, 3.25 mmol, 1 eq) and starting material **2** (722.00 mg, 9.75 mmol, 727.09 µL, 3 eq) in dichloromethane (50 mL). The reaction mixture was reacted at 25 °C for 12 hours. The reaction was monitored by LC-MS, which showed that the starting materials were completely consumed and the main peak corresponded to the desired product. The reaction mixture was concentrated under reduced pressure to obtain a crude product, and the crude product was purified by normal phase silica gel column chromatography (petroleum ether: ethyl acetate = 1:0 to 4:1) and then concentrated under reduced pressure to obtain Intermediate **3** (1.8 g, 2.67 mmol, 82.13% yield, 95% purity), as a white solid. **LCMS**: Rt = 0.786 min, 641.5 [M+H]⁺ ESI.

### Step 2: Synthesis of Intermediate 4

Sodium methoxide solution (2.53 g, 14.04 mmol, 30% purity, 5 eq) was slowly added to a solution of Intermediate **3** (1.8 g, 2.81 mmol, 1 eq) in anhydrous methanol (20 mL) at 0 °C, and the reaction mixture was reacted at 25 °C for 1.5 hours. The reaction was monitored by LC-MS, which showed that the main peak was the desired product. The reaction mixture was slowly poured into 1N HCl solution to adjust the pH to 3, and extracted with ethyl acetate (20 mL*2). The combined organic phases were washed with saturated brine (20 mL), dried over anhydrous sodium sulfate, and concentrated under reduced pressure to obtain Intermediate **4** (1.78 g, 2.53 mmol, 89.99% yield, 91% purity), as a white solid. **LCMS**: Rt = 0.735 min, 641.5 [M+H]⁺ ESI.

### Step 3: Synthesis of Intermediate 6

Starting material **5** (397.06 mg, 1.39 mmol, 0.5 eq) was slowly added to a solution of Intermediate **4** (1.78 g, 2.78 mmol, 1 eq) in N,N-dimethylformamide (20 mL) at 0 °C. The reaction mixture was reacted at 0 °C for 2 hours, and then pyridine (878.76 mg, 11.11 mmol, 896.69 µL, 4 eq) was slowly added. The reaction mixture was heated to 55 °C and reacted for 4 hours. The reaction was monitored by LC-MS, which showed that the main peak was the desired product. The reaction mixture was poured into water (20 mL) and extracted with ethyl acetate (20 mL*2). The combined organic phases were washed with saturated brine (20 mL), dried over anhydrous sodium sulfate, concentrated under reduced pressure to obtain a crude product, and the crude product was purified by normal phase silica gel column chromatography (petroleum ether: ethyl acetate = 1:0 to 3:1) and concentrated under reduced pressure to obtain Intermediate 6 (1.3 g, 1.99 mmol, 71.80% yield, 98% purity), as a white solid. **LCMS**: Rt = 0.746 min, 639.5 [M+H]⁺ ESI.

### Step 4: Synthesis of Compound 27

Boron trichloride solution (1 M, 7.83 mL, 5 eq) was slowly added to a solution of Intermediate **6** (1 g, 1.57 mmol, 1 eq) in dichloromethane (20 mL) at -70 °C, and the reaction mixture was reacted at 30 °C for 12 hours. The reaction was monitored by LC-MS, which showed that the starting material remained and the main peak was the desired product. The reaction mixture was slowly poured into a saturated aqueous sodium bicarbonate solution (20 mL) to adjust the pH to 8, and extracted with dichloromethane (20 mL*2). The combined organic phases were washed with saturated brine (20 mL), dried over anhydrous sodium sulfate, concentrated under reduced pressure to obtain a crude product, and the crude product was purified by normal phase silica gel column chromatography (petroleum ether: ethyl acetate = 1:0 to 1:1) and then separated and purified by reverse phase preparative chromatography [water (formic acid)-acetonitrile system] to obtain Compound 27 (113.80 mg, 199.29 µmol, yield 12.73%, purity 96.10%), as a white solid. **Prep-HPLC**: column: Phenomenex Luna C₁₈ 150*25mm*10µm; mobile phase: [H₂O(0.225% FA)-ACN]; gradient: 50%-80% B over 10.0 min; **LCMS**: rt = 0.646 min, 549.4 [M+H]⁺. **HPLC**: rt = 2.928 min; **¹H NMR** (400 MHz, DMSO-d6) δ =9.54 (s, 1H), 8.66 (s, 1H), 6.20 (s, 1H), 3.71 - 3.42 (m, 2H), 3.27 (br d, *J* = 3.6 Hz, 1H), 2.43 - 2.36 (m, 2H), 2.20 (br d, *J* = 12.8 Hz, 1H), 2.03 - 1.92 (m, 1H), 1.89 - 1.77 (m, 4H), 1.75 - 1.62 (m, 2H), 1.52 - 1.42 (m, 7H), 1.38 - 1.31 (m, 1H), 1.30 - 1.21 (m, 2H), 1.18 (s, 3H), 1.15 - 1.11 (m, 1H), 1.08 (s, 4H), 1.03 - 0.94 (m, 5H), 0.93 (s, 3H), 0.86 (s, 3H), 0.83 (s, 3H).

### Example 27

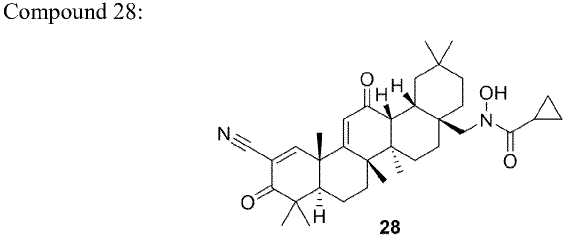

### Synthetic Route:

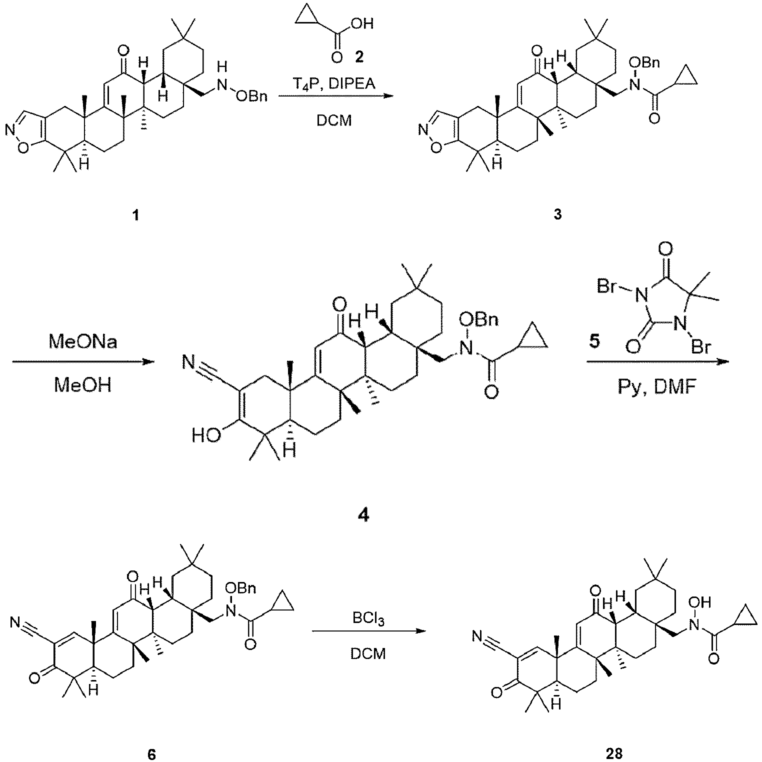

### Step 1: Synthesis of Intermediate 3

Starting material **2** (441.61 mg, 5.13 mmol, 405.89 µL, 3 eq) and N,N-diisopropylethylamine (1.33 g, 10.26 mmol, 1.79 mL, 3 eq) were added to a solution of starting material **1** (1 g, 1.71 mmol, 1 eq) in dichloromethane (20 mL), and then T₄P (3.70 g, 5.13 mmol, 50% purity, 3 eq) was added. The reaction mixture was stirred at 20 °C for 2 hours. The reaction was monitored by LC-MS, which showed that the starting materials were completely consumed and the main peak corresponded to the desired product. The reaction mixture was concentrated under reduced pressure to obtain a crude product, and the crude product was purified by normal phase silica gel column chromatography (petroleum ether:ethyl acetate = 1:0 to 3:1) and concentrated under reduced pressure to obtain Intermediate **3** (900 mg, 1.20 mmol, 70.14% yield, 87% purity) as a white solid. **LCMS**: Rt = 0.808 min, 653.5 [M+H]⁺ ESI.

### Step 2: Synthesis of Intermediate 4

Sodium methoxide solution (1.02 g, 5.67 mmol, 30% purity, 5 eq) was added slowly o a solution of Intermediate **3** (740 mg, 1.13 mmol, 1 eq) in anhydrous methanol (10 mL) at 0 °C. The reaction mixture was stirred at 20 °C for 1 hour. The reaction was monitored by LC-MS, which showed that the main peak was the desired product. The reaction mixture was slowly poured into 1N HCl solution to adjust the pH to 3, and then extracted with ethyl acetate (20 mL*2). The combined organic phases were washed with saturated brine (20 mL), dried over anhydrous sodium sulfate, and concentrated under reduced pressure to obtain Intermediate **4** (740 mg, crude) as a white solid. **LCMS**: Rt = 0.738 min, 653.5 [M+H]⁺ ESI.

### Step 3: Synthesis of Intermediate 6

Starting material **5** (162.03 mg, 566.70 µmol, 0.5 eq) was added slowly to a solution of Intermediate **4** (740 mg, 1.13 mmol, 1 eq) in N,N-dimethylformamide (10 mL) at 0 °C. The reaction mixture was stirred at 0 °C for 2 hours, and then pyridine (358.61 mg, 4.53 mmol, 365.92 µL, 4 eq) was added slowly. The reaction mixture was heated to 55 °C for 4 hours. The reaction was monitored by LC-MS, which showed that the main peak was the desired product. The reaction mixture was poured into water (20 mL) and extracted with ethyl acetate (20 mL*2). The combined organic phases were washed with saturated brine (20 mL), dried over anhydrous sodium sulfate, and concentrated under reduced pressure to obtain a crude product. The crude product was purified by normal phase silica gel column chromatography (petroleum ether:ethyl acetate = 1:0 to 3:1) and concentrated under reduced pressure to obtain Intermediate **6** (500 mg, 736.69 µmol, 65.00% yield, 95.9% purity) as a white solid. **LCMS**: Rt = 0.752 min, 651.5 [M+H]⁺ ESI.

### Step 4: Synthesis of Compound 28

Boron trichloride solution (1M, 3.07 mL, 5 eq) was added slowly to a solution of Intermediate **6** (400 mg, 614.55 µmol, 1 eq) in dichloromethane (8 mL) at -70 °C. The reaction mixture was stirred at 35 °C for 2 hours. The reaction was monitored by LC-MS, which showed that the starting materials were completely consumed and the main peak corresponded to the desired product. The reaction mixture was slowly poured into saturated aqueous sodium bicarbonate solution (20 mL) to adjust the pH to 8, and then extracted with dichloromethane (20 mL*2). The combined organic phases were washed with saturated brine (20 mL), dried over anhydrous sodium sulfate, and concentrated under reduced pressure to obtain a crude product. The crude product was separated and purified by reverse phase preparative chromatography [water (formic acid)-acetonitrile system] to obtain Compound **28** (48.49 mg, 84.41 µmol, 13.74% yield, 97.618% purity) as a yellow solid. **Prep-HPLC** column: Phenomenex luna Cis 150*40mm* 15µm; mobile phase: [H₂O(0.225% FA)-ACN]; gradient: 55%-85% B over 15.0 min. **LCMS:** rt = 0.655 min, 561.5 [M+H]⁺. **HPLC:** rt = 1.939 min; **¹H NMR** (400 MHz, DMSO-d6) δ = 9.80 (s, 1H), 8.66 (s, 1H), 6.20 (s, 1H), 3.69 - 3.62 (m, 1H), 3.59 - 3.52 (m, 1H), 3.29 - 3.22 (m, 1H), 2.33 - 2.28 (m, 1H), 2.23 - 2.16 (m, 1H), 2.02 - 1.94 (m, 1H), 1.88 - 1.82 (m, 3H), 1.74 - 1.63 (m, 2H), 1.46 (d, *J* = 2.0 Hz, 6H), 1.38 - 1.22 (m, 4H), 1.21 - 1.13 (m, H), 1.11 - 1.03 (m, 5H), 1.01 - 0.96 (m, 1H), 0.93 (s, 3H), 0.85 (d, *J* = 12.8 Hz, 6H), 0.77 - 0.73 (m, 2H), 0.72 - 0.68 (m, 2H).

### Example 28

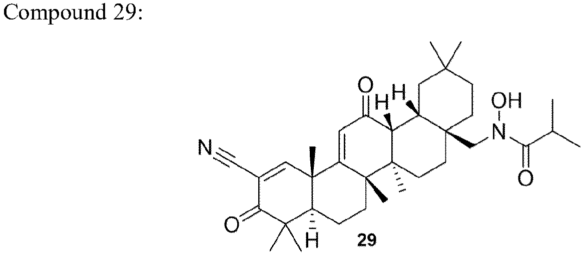

### Synthetic Route:

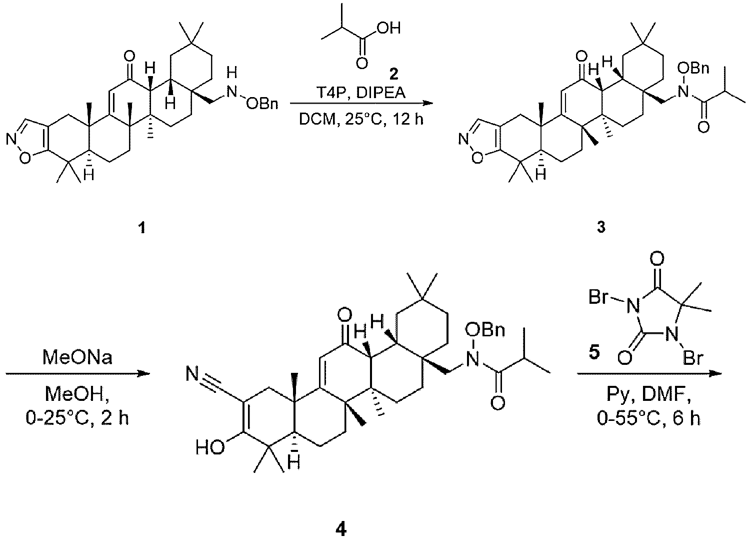

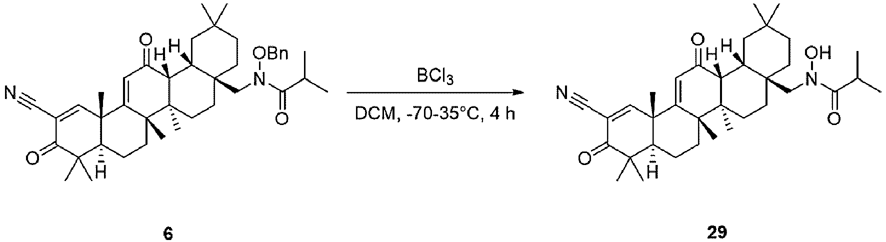

### Step 1: Synthesis of Intermediate 3

Starting material 2 (858.71 mg, 9.75 mmol, 903.90 µL, 3 eq), N,N-diisopropylethylamine (4.20 g, 32.49 mmol, 5.66 mL, 10 eq), and T₄P (7.02 g, 9.75 mmol, 50% purity, 3 eq) were added to a solution of starting material 1 (1.9 g, 3.25 mmol, 1 eq) in dichloromethane (50 mL). The mixture was stirred at 25 °C for 12 hours. After the reaction of the starting materials was completed as detected by LCMS, the reaction mixture was concentrated under reduced pressure to obtain a crude product, which was purified by column chromatography (ethyl acetate/petroleum ether = 20 - 25%) to obtain Intermediate 3 (1.6 g, 2.20 mmol, 67.68% yield, 90% purity) as a white solid. **LCMS:** rt = 0.821 min, 655.3 [M+H]⁺.

### Step 2: Synthesis of Intermediate 4

Sodium methoxide (2.20 g, 12.22 mmol, 30% purity, 5 eq) was added to a solution of Intermediate 3 (1.6 g, 2.44 mmol, 1 eq) in methanol (20 mL) at 0 °C. The mixture was stirred at 25 °C under nitrogen for 2 hours. After the reaction of the starting materials was completed as detected by LCMS, the reaction mixture was adjusted to pH 3-4 with 1N hydrochloric acid, extracted with dichloromethane, washed, dried, and concentrated under reduced pressure to obtain Intermediate 4 (1.5 g, crude) as a white solid. **LCMS:** rt = 0.757 min, 655.3 [M+H]⁺.

### Step 3: Synthesis of Intermediate 6

Starting material 5 (327.43 mg, 1.15 mmol, 0.5 eq) was added to a solution of Intermediate 4 (820 mg, 3.01 mmol, 1 eq) in N,N-dimethylformamide (15 mL) at 0 °C under nitrogen. The mixture was stirred at 0 °C for 2 hours. Pyridine (724.67 mg, 9.16 mmol, 739.46 µL, 4 eq) was added to the reaction mixture, and the mixture was stirred at 55 °C for 4 hours. After the reaction of the starting materials was completed as detected by LCMS, the reaction mixture was concentrated under reduced pressure and poured into water. The resulting precipitate was filtered, and the collected solid was triturated with water to obtain Intermediate 6 (1.4 g, 1.93 mmol, yield 84.26%, purity 90%) as a light yellow solid. **LCMS:** rt = 0.767 min, 653.5 [M+H]⁺.

### Step 4: Synthesis of Compound 29

A solution of Compound 6 (1 g, 1.53 mmol, 1 eq) in dichloromethane (10 mL) was purged with nitrogen three times and cooled to -70 °C. Boron trichloride (1M, 7.66 mL, 5 eq) was added slowly, and the reaction mixture was stirred at 35 °C for 4 hours. After the reaction of the starting materials was completed as detected by LCMS, the reaction mixture was poured into saturated sodium bicarbonate solution, extracted with dichloromethane, washed, dried, and concentrated under reduced pressure to obtain a crude product. The crude product was separated and purified by reverse phase preparative chromatography [water (formic acid)-acetonitrile system] and lyophilized to obtain Compound 29 (150.65 mg, 267.69 µmol, 17.48% yield) as a white solid. **Prep-HPLC:** column: Phenomenex luna C₁₈ 150*25mm*10µm; mobile phase: [H₂O(0.225% FA)-ACN]; gradient: 60%-90% B over 10.0 min. **LCMS**: Retention time = 4.177 min, 563.4 [M+H]⁺. **HPLC:** retention time = 2.396 min, purity 98.87%; **¹H NMR** (400 MHz, DMSO-d6) δ = 9.56 (s, 1H), 8.67 (s, 1H), 6.21 (s, 1H), 3.74 (br d, *J* = 14.4 Hz, 1H), 3.41 (br d, *J* = 14.4 Hz, 1H), 3.29 (br d, *J* = 4.8 Hz, 1H), 3.10 (quin, *J* = 6.8 Hz, 1H), 2.27 - 2.17 (m, 1H), 2.02 - 1.93 (m, 1H), 1.92 - 1.79 (m, 4H), 1.75 - 1.63 (m, 2H), 1.48 (d, *J* = 8.4 Hz, 6H), 1.46 - 1.39 (m, 1H), 1.37 - 1.31 (m, 1H), 1.31 - 1.21 (m, 2H), 1.19 (s, 3H), 1.15 (br d, *J* = 3.2 Hz, 1H), 1.12 (br s, 1H), 1.09 (s, 3H), 1.04 (br s, 1H), 1.00 (dd, *J* = 5.6, 6.8 Hz, 7H), 0.93 (s, 3H), 0.86 (s, 3H), 0.83 (s, 3H).

### Example 29

### Biological Evaluation

Test Example 1: *In vitro* Human Nrf2 Receptor Agonist Activity Assay
Reagents: Opti-MEM serum-free medium; PEI 40000 transfection reagent; DMEM complete medium.
Equipment: Cx7Pro High Content Rapid Imaging Platform, etc.

For each well of cells, 10 µL of Opti-MEM serum-free medium (Cienry) was used to dilute 360 ng of Nrf2 phase transition probe plasmid, mixed thoroughly to form a DNA dilution, and allowed to stand for 5 minutes. 10 µL of Opti-MEM serum-free medium was used to dilute 0.6 µL of PEI 40000 transfection reagent (YEASEN), mixed thoroughly to form a PEI 40000 dilution, and allowed to stand for 5 minutes. The DNA dilution and PEI40000 dilution were mixed and incubated at room temperature for 20 minutes to form DNA-PEI cationic nucleic acid transfection reagent complexes. 20 µL of cell growth medium was removed from each well, and 20 µL of DNA-PEI cationic nucleic acid transfection reagent complex was added to each well. The plate was shaken to mix gently. Cells were cultured in an incubator at 37 °C with 5% CO₂. 1 hour after transfection, 75 µL of cell growth medium was removed from each well, and 75 µL of fresh pre-warmed DMEM complete medium (Meilunbio) was added to each well, maintaining a total volume of 100 µL per well. Eighteen hours after transfection, serially diluted drugs (0.0625 nM, 0.125 nM, 0.25 nM, 0.5 nM, 1 nM, 2 nM, 4 nM, 8 nM, 16 nM, 32 nM, 128 nM) were first mixed into pre-warmed DMEM complete medium. 100 µL of medium containing the serially diluted drugs was added to each sample well, resulting in a final volume of 200 µL per well. Six hours after drug addition, 16 fields of view were randomly selected for photographing for from each well using a Cx7Pro high-content rapid imaging platform (Thermo Fisher) with a 20x high-resolution lens. The nucleus location was determined by the independently expressed NLS-mTagBFP2 in the probe, thus determining the number of transfected cells. The total fluorescence intensity of the Nrf2 phase transition probe was calculated. The total fluorescence intensity of the Nrf2 phase transition probe "droplet" in each cell was statistically analyzed. The total fluorescence intensity of the Nrf2 phase transition probe in each cell of the drug group was compared with that in each cell of the DMSO group. The EC₅₀ of each drug was statistically analyzed using the log(agonist) vs. response - Variable slope (four parameters) analysis method in GraphPad Prism, and the results are shown in Table 1. The structural formula of Omaveloxolone is as follows:

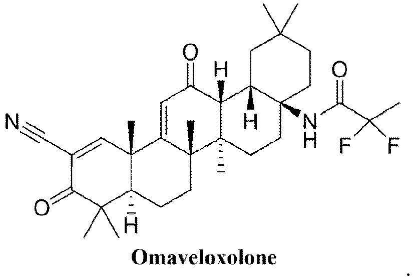

**Table 1: Test results of in vitro human Nrf2 receptor agonist activity of representative compounds of the present application**

| Compound | Human Nrf2 Receptor Agonist Activity EC₅₀ (nM) |
|---|---|
| Edaravone | Inactive |
| Omaveloxolone | 6.60 |
| 2 | 680.80 |
| 4 | 3.57 |
| 6 | 20.50 |
| 7 | 15.20 |
| 10 | 699.2 |
| 11 | 293.5 |
| 13 | 24.01 |
| 19 | 2.68 |
| 23 | 4.37 |
| 26 | 2.43 |
| 27 | 2.40 |
| 28 | 3.27 |
| 29 | 2.30 |

The biological experimental data are shown in Table 1. Moreover, the test data show that Compounds 2, 4, 6, 7, 11, 13, 19, 23, 26, 27, 28, and 29 prepared in the present application all exhibit Nrf2 receptor agonist activity. In particular, Compounds 4, 19, 23, 26, 27, 28, and 29 prepared in the present application exhibit single-digit nanomolar human Nrf2 receptor agonist activity, superior to the control drug Omaveloxolone. The above *in vitro* human Nrf2 receptor agonist activity data indicate that the compounds of the present application, their pharmaceutically acceptable salts, and stereoisomers can be used for the preparation of NRF2-Leap1 uncouplers. Moreover, compared with the control drug Omaveloxolone, the compounds of the present application also show significant differences in biological activities such as DPPH free radical scavenging and MDA anti-lipid peroxidation, as shown in Test Examples 2 to 4.

### Test Example 2: DPPH Free Radical Scavenging Activity Test

### Experimental Objective: To determine the DPPH free radical scavenging activity of the compounds of the present application.

### Experimental Materials:

| **Material** | **Manufacturer** | **Catalog No.** |
|---|---|---|
| Dimethyl sulfoxide | Sigma | D2650 |
| D4Plus pipette tips rack | Tecan | 30097371 |
| T8Plus pipette tips rack | Tecan | 30097370 |
| 96-well plate | Corning | 3599 |
| Absolute ethanol | Sangon | A500737 |
| 2,2-Diphenyl-1-picrylhydrazyl (DPPH) | MCE | HY-112053 |

### Experimental Instruments:

| **Instrument** | **Manufacturer** | **Model** |
|---|---|---|
| Digital Dispenser | Tecan | HP D300 |
| Microplate Reader | FlexStation 3 | Molecular Devices |
| Centrifuge | Eppendorf | 5425R |
| Tissue Grinder | Shanghai Jingxin | JXFSTPRP-CL-48 |
| Dry Thermostat | Hm-Kylin | GL-150B |

### Experimental Method:

First, 20 µL of the test compound was added to a 96-well plate and serially diluted at a 1:2 ratio with DMSO. Then, 200 µL of 200 µM DPPH prepared in absolute ethanol as solvent was added to each well. After gentle shaking, incubation was performed at room temperature in the dark for 30 minutes. Absorbance value at 517 nm was measured using a microplate reader. The DPPH scavenging rate was calculated using the following formula: DPPH scavenging rate% = (1-Ai/A0)*100%, where Ai was the absorbance value of the sample and A0 was the absorbance value of the DMSO control group. Data were processed using XLfit 5.3.1.3 software, and IC₅₀ values were obtained using a non-linear fitting formula. Results are shown in Table 2.

**Table 2: Test results of the DPPH free radical scavenging ability of the representative compound of the present application**

| Compound | Free radical scavenging DPPH EC₅₀ (µM) |
|---|---|
| Edaravone | 73.28 µM |
| Omaveloxolone | Inactive |
| 27 | 482.79 |
| 28 | 408.57 |

The results show that Compounds 27 and 28 prepared in the present application possess DPPH free radical scavenging activity, while Omaveloxolone has no such activity.

### Test Example 3: MDA Anti-lipid Peroxidation Activity Test

### Experimental Objective: To test the MDA anti-lipid peroxidation activity of the compounds of the present application.

### Experimental Materials:

| **Material** | **Manufacturer** | **Catalog No.** |
|---|---|---|
| DPBS ( 1× ) | Corning | 21-031-CVC |
| Dimethyl sulfoxide | Sigma | D2650 |
| D4Plus pipette tips rack | Tecan | 30097371 |
| T8Plus pipette tips rack | Tecan | 30097370 |
| 96-well plate | Corning | 3599 |
| Ascorbic acid | ST1434 | Beyotime |
| Lipid Peroxidation (MDA) Assay Kit | S0131M | Beyotime |

### Experimental Instruments:

| **Instrument** | **Manufacturer** | **Model** |
|---|---|---|
| Digital Dispenser | Tecan | HP D300 |
| Microplate Reader | FlexStation 3 | Molecular Devices |
| Centrifuge | Eppendorf | 5425R |
| Tissue Grinder | Shanghai Jingxin | JXFSTPRP-CL-48 |
| Dry Thermostat | Hm-Kylin | GL-150B |

### Experimental Method:

First, brain tissue homogenate was extracted; an adult male SD rat was anesthetized with isoflurane and sacrificed by cervical dislocation. The whole brain was removed, and washed twice with DPBS, and the meninges were stripped. The brain was transferred to a 50 mL centrifuge tube containing 10 mL of DPBS, cut into pieces with scissors, and dispensed into 10 1.5 mL centrifuge tubes. Three grinding beads were added to each tube, and the tissue was ground at 90 Hz for 60 min, repeated 3 times. The ground tissue homogenate was transferred to a new 50 mL centrifuge tube, and DPBS was added to a total volume of 30 mL and thoroughly mixed. Then, 20 µL of test compound was added to a 96-well plate and serially diluted at a ratio of 1:3 with DMSO. Subsequently, 100 µL of brain tissue homogenate, 50 µL of DPBS, and 50 µL of 200 µg/mL vitamin C were added sequentially, and a series of concentration standards were prepared as a standard curve. After shaking, incubation was performed at 37°C for 1 hour. Then, 400 µL of MDA working solution was added, and the plate was heated at 100°C for 15 minutes. After cooling to room temperature, centrifugation was performed at 1000 g for 10 minutes. 200 µL of supernatant was transferred to a new plate, and absorbance value at 532 nm was measured using a microplate reader. The MDA clearance rate was calculated using the following formula: MDA clearance rate% = [((A1-A0)-(A2-A0))/((A1-A0)-(A3-A0))]*100%, where A1 was the absorbance value of the high control group, A2 was the absorbance value of the sample, A3 was the absorbance value of the low control group, and A0 was the absorbance value of the blank group. Data were processed using XLfit 5.3.1.3 software, and IC₅₀ values were obtained using a non-linear fitting formula. Results are shown in Table 3.

**Table 3: Test results of the MDA anti-lipid peroxidation ability of the compounds of the present application**

| Compound | Antioxidant MDA EC₅₀ (µM) |
|---|---|
| Edaravone | 85.82 |
| Omaveloxolone | Inactive |
| 19 | > 250 |
| 23 | 102.78 |
| 26 | 11.85 |
| 27 | 12.59 |
| 28 | 11.17 |
| 29 | 11.38 |

The results show that Compounds 23, 26, 27, 28, and 29 prepared in the present application all possess anti-lipid peroxidation ability, and their activity is superior to Edaravone, while Omaveloxolone has no such activity.

### Test Example 4: Interventional Effect of Representative Compounds on Ferroptosis (qPCR)

1) Experimental Objective: The interventional effect of a series of compounds on the ferroptosis process was tested by detecting changes in the expression levels of key genes in the ferroptosis signaling pathway at the cellular level. The specific indicator was the mRNA level of PTGS2, a key gene in ferroptosis process (detected by real-time fluorescent quantitative RT-PCR).
2) Experimental Method:

HT-1080 cells (Cat. No. CCL-121, Shanghai Cell Bank, originally from ATCC) were plated in 6-well plates at 4 × 10⁵ cells/well for 22 h. HT-1080 cells were treated in advance with different concentration gradients of test compounds (1.37 nM to 333 nM, 6 concentration gradients in total) for 1 hour. Then, HT-1080 cells were treated with 200 nM of the classical ferroptosis inducer RSL3 (Cat. No. HY-100218A, MCE, USA) for 16 hours. mRNA was extracted, reverse transcribed to cDNA, and PTGS2 mRNA levels were detected by qRT-PCR (model: ABI7500, Thermo Fisher, USA). Ferroptosis inhibitor Fer-1 (Cat. No. HY-100579, MCE, USA) was used as a positive control.

### 3) Experimental Results:

The log(agonist) vs. response - Variable slope (four parameters) analysis method in GraphPad Prism was used to calculate the IC₅₀ of each drug. Results are shown in Table 4.

**Table 4: Intervention effect of the compounds of the present application on the ferroptosis process**

| RSL3 Treatment and qPCR Detection of PTGS2 mRNA | |
|---|---|
| Compound | PTSG2 IC₅₀ (nM) |
| Omaveloxolone | Inactive |
| 26 | 77.52 |
| 27 | 50.82 |
| 28 | 119.1 |
| 29 | 51.37 |

The results show that Compounds 26, 27, 28, and 29 prepared in the present application can all inhibit ferroptosis, while Omaveloxolone has no such activity.

The above pharmacological tests demonstrate that the preferred NRF2-Keap1 compounds prepared in the present application, such as Compounds 26, 27, 28, 29, etc., not only maintain Nrf2 agonist activity similar to the marketed Nrf2 agonist Omaveloxolone but also possess additional activities such as scavenging DPPH free radicals, inhibiting the production of lipid peroxide MDA, or intervening in ferroptosis.

Furthermore, *in vivo* biological tests have verified that the compounds of the present application have significant effects on various diseases, including cerebral small vessel disease, mitochondrial encephalomyopathy, autism spectrum disorder, Rett syndrome, Friedreich ataxia, stroke, hemorrhagic stroke, ischemic stroke, multiple sclerosis, amyotrophic lateral sclerosis, schizophrenia, cognitive impairment in schizophrenia, Parkinson's disease, cognitive impairment in Parkinson's disease, Alzheimer's disease, vascular dementia, epilepsy, Huntington's chorea, heart failure, myocardial infarction, renal failure, and renal ischemia. In particular, they show significant effects on diseases such as stroke, multiple sclerosis, and amyotrophic lateral sclerosis, and can be used as a medicament for preventing or treating stroke, multiple sclerosis, and amyotrophic lateral sclerosis.

Test Example 5: Protective Effect of Representative Compound on Ischemic Stroke Rats
1) Reagents: Omaveloxolone, MedChemexpress Biotechnology Co., USA; Transient Middle Cerebral Artery Occlusion Model (tMCAO) suture, Beijing Ruiwode Life Science Technology Co., Ltd.; DMAO, Beyotime Biotechnology Co., Ltd.; Solutal, Sigma-Aldrich (Shanghai) Trading Co., Ltd.; Physiological saline, Sinopharm Chemical Reagent Co., Ltd.; TTC (2.3.5-Triphenyltetrazolium chloride), Sigma-Aldrich (Shanghai) Trading Co., Ltd.
2) Establishment of cerebral ischemia rat model using tMCAO method:
   Rats were anesthetized with 10% chloral hydrate (350 mg/kg) and fixed in a supine position on the operating table. The neck was disinfected. A midline neck incision was made, and the intermuscular space between the left sternocleidomastoid muscle and the sternohyoid muscle was bluntly dissected to expose the common carotid artery. The common carotid artery was picked out with ophthalmic curved forceps and threaded with silk suture for later use. The external carotid artery was dissected under the right digastric muscle and hyoid bone and threaded with silk suture for later use. The suture was passed through and tied tightlyaround the common carotid artery. A small incision was made at the free end of the common carotid artery and a loose knot was tied. The occluder was inserted through the incision along the common carotid artery. The occluder was slowly advanced and stopped when resistance was felt. The loose knot tied in advance was tied tightly. The body temperature was maintained at 37°C during the operation. The occluder was removed after 1.5 hours. For the rats in the sham group, the occluder was not inserted after the blood vessel was dissected, but the other operation steps were the same.
3) Experimental grouping and administration:

SD male rats, weighing 230-250 g, 40 rats, were randomly divided into the sham group, model group, Omaveloxolone 3 mg/kg group, Omaveloxolone 9 mg/kg group, and Compound 28 3.03 mg/kg group. After 3 days of acclimatization, the rats were administered via tail vein injection during the model establishment and occluder insertion. Rats in the sham group and the model group were given an equal volume of vehicle (10% DMSO + 10% solutal + 80% physiological saline).

### 4) Indicator detection:

mNSS score: Neurological behavior scores were assessed 24 hours after modeling. The evaluation mainly focused on sensory and motor aspects to assess the neurological function of ischemic stroke rats.

Cerebral infarction area measurement: Rats were euthanized with an overdose of chloral hydrate. The brains were removed and placed in a -20 °C freezer for 20 min. The brains were taken out and placed in a brain matrix for sectioning into 2 mm thick slices. The brain slices were placed in 2% TTC solution, protected from light, and incubated in a 37 °C incubator for 15 min. After staining, images were taken for cerebral infarction area analysis.

### 5) Statistical methods:

All data in this study are expressed as mean ± standard deviation (Mean ± SD) and analyzed using GraphPad Prism 7.0. Differences among multiple groups were evaluated by one-way ANOVAs followed by Tukey's test. Behavioral tests were analyzed using the Kruskal-Wallis test. P < 0.05 was considered statistically significant.

### 6) Experimental results:

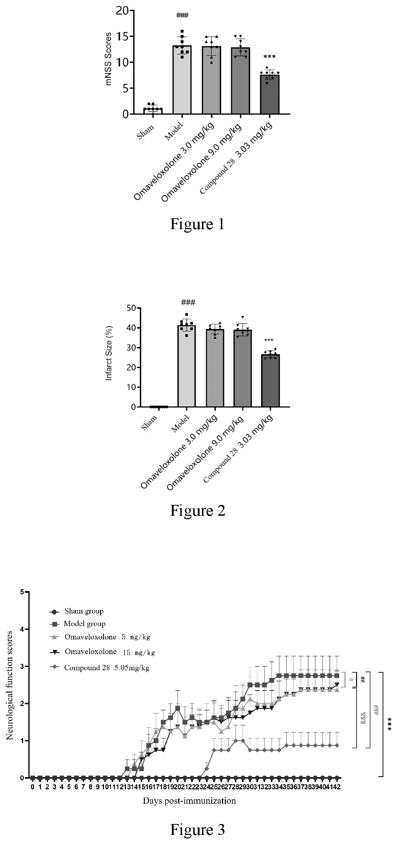

Effect of Compound 28 on neurological function in ischemic stroke rats:
As shown in Figure 1, Omaveloxolone 3 mg/kg and 9 mg/kg had no effect on mNSS scores in cerebral ischemia rats. At the same molar dose as Omaveloxolone 3 mg/kg, Compound 28 (3.03 mg/kg) significantly reduced the mNSS score in cerebral ischemia rats.

Effect of Compound 28 on cerebral infarction area in ischemic stroke rats:
As shown in Figure 2, Omaveloxolone 3 mg/kg and 9 mg/kg had no effect on cerebral infarction area in cerebral ischemia rats. At the same molar dose as Omaveloxolone 3 mg/kg, Compound 28 (3.03 mg/kg) significantly reduced the cerebral infarction area in cerebral ischemia rats.

7) Conclusion: The Nrf2-Keap1 uncoupler of the present application, such as Compound 28, can reduce the cerebral infarction area in ischemic stroke rats and has a protective effect on neurological function impairment.

Test Example 6: Effect of Representative Compound on Neurobehavioral Function in Multiple Sclerosis Model Mice

### 1. Materials and Methods

### 1) Main reagents

| | | |
|---|---|---|
| **Material** | **Manufacturer** | **Country** |
| MOG₃₅₋₅₅ peptide | Sangon | China |
| Pertussis toxin | Aibixin | China |
| Complete Freund's Adjuvant, 5 mg/ml | chondrex | USA |
| Solutol^{®}HS 15 | Sigma-Aldrich | Germany |
| Tween-80 | Beyotime | China |
| DMSO | Beyotime | China |
| PEG300 | Sigma-Aldrich | Germany |

### 2) Experimental animals and grouping and administration

Female C57BL/6J mice were randomly divided into 5 groups: sham group, model group, Omaveloxolone 5 mg/kg group, Omaveloxolone 15 mg/kg group, and Compound 28 5.05 mg/kg group, with 8 mice in each group. Drugs were administered by oral gavage. The mice in the sham group and the model group were given an equal volume of vehicle (10% solutol + 90% physiological saline) twice a day for 42 consecutive days.

### 3) Preparation of multiple sclerosis model - EAE model

The EAE model is a commonly used animal model for multiple sclerosis. A mixed emulsion containing myelin oligodendrocyte glycoprotein MOG35-55 peptide and complete Freund's adjuvant (CFA) containing Mycobacterium tuberculosis was injected subcutaneously into the back of the mice. Pertussis toxin (PTX) was injected intraperitoneally on the day of immunization and 48 hours later to establish the EAE (experimental autoimmune encephalomyelitis) model.

### 4) Body weight and neurological function scoring (5-point scale)

From the day of immunization induction (Day 0), mice were weighed and their neurological function scores were recorded daily. Neurological function scoring criteria (5-point scale): 0, no clinical deficits; 1, partial tail paralysis; 2, complete tail paralysis; 3, partial hind limb paralysis; 4, complete hind limb paralysis; 5, forelimb paralysis; 6, death.

### 5) Statistical analysis

All data in this study are expressed as mean ± standard deviation (Mean ± SD). Differences in neurological function scores and body weight between groups were compared using two-way ANOVA and Tukey's multiple comparison test. All data were analyzed using GraphPad Prism 9.0.0 software. p < 0.05 indicated statistically significant differences.

### 2. Experimental results

As shown in Figure 3, Omaveloxolone 5 mg/kg and 15 mg/kg significantly reduced the neurological function scores in EAE model mice, but there was no significant difference between the two groups, indicating that Omaveloxolone 5 mg/kg reached the maximum effect. At the same molar dose as Omaveloxolone 5 mg/kg, Compound 28 (5.05 mg/kg) significantly reduced the neurological function scores in EAE model mice, and the effect was superior to Omaveloxolone 15 mg/kg, with statistically significant differences.

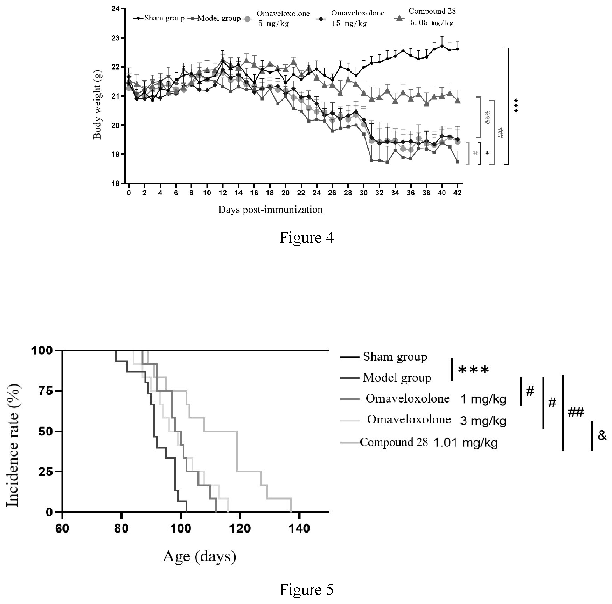

As shown in Figure 4, Omaveloxolone 5 mg/kg and 15 mg/kg significantly increased the body weight of EAE model mice, but there was no significant difference between the two groups, indicating that Omaveloxolone 5 mg/kg reached the maximum effect. At the same molar dose as Omaveloxolone 5 mg/kg, Compound 28 (5.05 mg/kg) significantly improved the body weight loss of EAE model mice, and the effect was superior to Omaveloxolone 15 mg/kg, with statistically significant differences.

### 3. Experimental conclusion

Compound 28 has a protective effect on neurological function in multiple sclerosis model mice, and its effect is superior to that of Omaveloxolone.

### Test Example 6: Protective Effect of Representative Compound on Amyotrophic Lateral Sclerosis Model Mice

### 1. Materials and methods

### 1) Main reagents

Omaveloxolone was purchased from MCE Biotechnology Co., Ltd.; Solutal was purchased from Sigma-Aldrich (Shanghai) Trading Co., Ltd.; and physiological saline was purchased from Sinopharm Chemical Reagent Co., Ltd.

### 2) Animals

Forty-eight B6SJL-Tg (SOD1 G93A)-1Gur/J transgenic mice (model for amyotrophic lateral sclerosis, ALS), half male and half female, were purchased from Shanghai Model Organisms Technology Center, Inc.

### 3) Experimental grouping and administration

SOD1 G93A mice were randomly divided into model group, Omaveloxolone 1 mg/kg group, Omaveloxolone 3 mg/kg group, and Compound 28 1.01 mg/kg group, with 12 mice in each group. Concurrently, 12 C57BL/6J mice were set as the control group. Drugs were administered intraperitoneally. The mice in the control group and the model group were given an equal volume of vehicle (1% DMSO + 4% solutal + 95% physiological saline), once a day for 10 consecutive weeks.

### 4) Indicator detection

### Rotarod test

The rotarod test is a classical behavioral test used to evaluate motor coordination in mice. Rotarod test (Panlab rotarod apparatus, purchased from Harvard Bioscience, USA) was performed twice each week. The rotating speed range of the rotarod was from 4 r/min to 40 r/min. Experimental procedures were as follows: 1. Before the formal experiment, mice underwent habituation adaptation training with the rotation speed set at 12 r/min, for 5 min each time, twice daily for 3 consecutive days; 2. Formal experiment: three consecutive tests (3 min/each test, with a 30 min interval between tests); 3. The time each mouse stayed on the rotarod was recorded for each test, and the longest time on the rotarod among the three tests was taken as the fall latency.

### Onset time

The rotarod test was used to determine the onset time of disease in mice. The date when a mouse first fell off the rotarod within 3 minutes was recorded as the onset date.

### Hanging cage test

The hanging cage test was used to assess the grip strength and endurance of the mouse's limbs. Hanging cage test was performed twice each week. Each mouse was placed on the center of a wire mesh, which was gently shaken to make the mouse grip tightly. The wire mesh was then slowly inverted to a horizontal position, and the time the mouse hung onto the wire mesh was recorded. Each mouse was measured three times with a 30-minute interval between tests, and the longest time thereof was taken as the fall latency.

### 2. Experimental results

### 1) Effect of Compound 28 on the onset time in SOD1 G93A mice

As shown in Figure 5, Omaveloxolone 1 mg/kg and 3 mg/kg both significantly delayed the onset time in SOD1 G93A mice, but there was no significant difference between the two groups, indicating that Omaveloxolone 1 mg/kg reached the maximum effect. At the same molar dose as Omaveloxolone 1 mg/kg, Compound 28 (1.01 mg/kg) significantly delayed the onset time in SOD1 G93A mice, and the effect was superior to Omaveloxolone 3 mg/kg, with statistically significant differences.

### 2) Effect of Compound 28 on motor coordination in SOD1 G93A mice

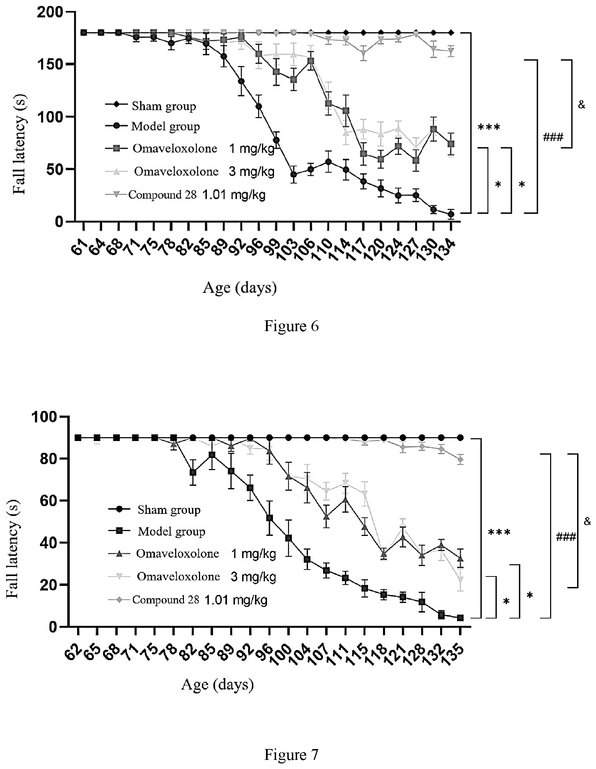

As shown in Figure 6, Omaveloxolone 1 mg/kg and 3 mg/kg both significantly improved motor coordination in SOD1 G93A mice, as evidenced by a significant increase in fall latency. However, there was no significant difference between the two groups, indicating that Omaveloxolone 1 mg/kg reached the maximum effect. At the same molar dose as Omaveloxolone 1 mg/kg, Compound 28 (1.01 mg/kg) significantly increased the fall latency in mice, and the effect was superior to Omaveloxolone 3 mg/kg, with statistically significant differences.

### 3) Effect of Compound 28 on muscle endurance in SOD1 G93A mice

As shown in Figure 7, Omaveloxolone 1 mg/kg and 3 mg/kg both significantly improved muscle endurance in SOD1 G93A mice, as evidenced by a significant increase in fall latency. However, there was no significant difference between the two groups, indicating that Omaveloxolone 1 mg/kg reached the maximum effect. At the same molar dose as Omaveloxolone 1 mg/kg, Compound 28 (1.01 mg/kg) significantly increased the fall latency in mice, and the effect was superior to Omaveloxolone 3 mg/kg, with statistically significant differences.

### 3. Experimental conclusion

### Compound 28 improves the neurobehavioral functions in amyotrophic lateral sclerosis model mice, and its effect is superior to that of Omaveloxolone.

Combining the above experimental results, it is demonstrated that the C17-site nitrogen-substituted and methylene-substituted oleanane triterpene derivatives disclosed in the present application can serve as a novel class of Nrf2-Keap1 uncouplers, possessing excellent Nrf2 agonistic activity, while also exhibiting antioxidant effects through activities such as scavenging DPPH free radicals, inhibiting the production of lipid peroxide MDA, or intervening in ferroptosis. Meanwhile, the present application verifies through *in vivo* biological experiment that the compounds of the present application have significant effects on the prevention or treatment of various diseases, the related diseases including: cerebral small vessel disease, mitochondrial encephalomyopathy, autism spectrum disorder, Rett syndrome, Friedreich ataxia, stroke, hemorrhagic stroke, ischemic stroke, multiple sclerosis, amyotrophic lateral sclerosis, schizophrenia, cognitive impairment in schizophrenia, Parkinson's disease, cognitive impairment in Parkinson's disease, Alzheimer's disease, vascular dementia, epilepsy, Huntington's chorea, heart failure, myocardial infarction, renal failure, renal ischemia, etc. In particular, the present application also verifies through the *in vivo* biological experiment that some compounds have significant effects on the treatment and/or prevention of stroke, multiple sclerosis, and amyotrophic lateral sclerosis.

The preferred embodiments of the present application have been described in detail above. However, the present application is not limited to the specific details of the above-mentioned embodiments. Within the scope of the technical concept of the present application, various equivalent transformations can be made to the technical solutions of the present application, and these equivalent transformations all fall within the protection scope of the present application. Additionally, it should be noted that the specific technical features described in the above-mentioned specific embodiments can be combined in any suitable manner without contradiction. To avoid unnecessary repetition, various possible combinations are not further described. Furthermore, various different embodiments of the present application can also be arbitrarily combined, as long as they do not violate the idea of the present application, and they should also be regarded as the content disclosed by the present application.

## Claims

1. A compound of Formula (I), or a pharmaceutically acceptable salt thereof, as shown below: 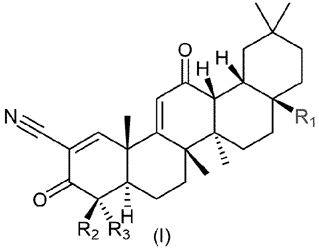 wherein R₁ is independently selected from the group consisting of -NH-heteroaryl, -NH-heteroaryldiyl-R₁', -NH-C(=O)-alkyl, -NH-C(=O)-substituted alkyl, -NH-C(=O)-alkenyl, -NH-C(=O)-substituted alkenyl, -NH-C(=O)-alkynl, -NH-C(=O)-substituted alkynl, -NH-C(=O)-aryl, - NH-C(=O)-aryldiyl-R₁', -NH-C(=O)-heteroaryl, -NH-C(=O)-heteroaryldiyl-R₁', -N(OH)-C(=O)-alkyl, -N(OH)-C(=O)-substituted alkyl, -N(OH)-C(=O)-alkenyl, -N(OH)-C(=O)-substituted alkenyl, -N(OH)-C(=O)-alkynyl, -N(OH)-C(=O)-substituted alkynyl, -N(OH)-C(=O)-aryl, - N(OH)-C(=O)-aryldiyl-R₁', -N(OH)-C(=O)-heteroaryl, -N(OH)-C(=O)-heteroaryldiyl-R₁', -NH-C(=O)-L-amino acid-NH-heteroaryl, -NH-C(=O)-L-amino acid-NH-heteroaryldiyl-R₁', -CH₂-N(OH)-C(=O)-alkyl, -CH₂-N(OH)-C(=O)-substituted alkyl, -CH₂-N(OH)-C(=O)-alkenyl, -CH₂-N(OH)-C(=O)-substituted alkenyl, -CH₂-N(OH)-C(=O)-alkynyl, -CH₂-N(OH)-C(=O)-substituted alkynyl, -CH₂-N(OH)-C(=O)-heteroaryl, and -CH₂-N(OH)-C(=O)-heteroaryldiyl-R₁'; R₂ is hydrogen or methyl; and R₃ is hydrogen or methyl.

2. The compound according to claim 1, wherein the compound is represented by Formula (II): 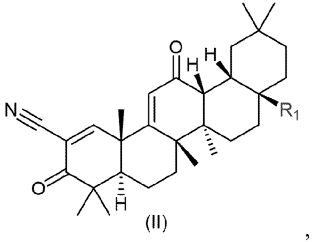
wherein R₁ is independently selected from the group consisting of -NH-heteroaryl, -NH-heteroaryldiyl-R₁', -NH-C(=O)-alkyl, -NH-C(=O)-substituted alkyl, -NH-C(=O)- alkenyl, -NH-C(=O)-substituted alkenyl, -NH-C(=O)-alkynyl, -NH-C(=O)-substituted alkynyl, -NH-C(=O)-aryl, -NH-C(=O)-aryldiyl-R₁', -NH-C(=O)-heteroaryl, -NH-C(=O)-heteroaryldiyl-R₁', -N(OH)-C(=O)-alkyl, -N(OH)-C(=O)-substituted alkyl, -N(OH)-C(=O)-alkenyl, -N(OH)-C(=O)-substituted alkenyl, -N(OH)-C(=O)-alkynyl, -N(OH)-C(=O)-substituted alkynyl, -N(OH)-C(=O)-aryl, -N(OH)-C(=O)-aryldiyl-R₁', -N(OH)-C(=O)-heteroaryl, -N(OH)-C(=O)-heteroaryldiyl-R₁', -NH-C(=O)-L-amino acid-NH-heteroaryl, -NH-C(=O)-L-amino acid-NH-heteroaryldiyl-R₁', - CH₂-N(OH)-C(=O)-alkyl, -CH₂-N(OH)-C(=O)-substituted alkyl, -CH₂-N(OH)-C(=O)-alkenyl, - CH₂-N(OH)-C(=O)-substituted alkenyl, -CH₂-N(OH)-C(=O)-alkynyl, -CH₂-N(OH)-C(=O)-substituted alkynyl, -CH₂-N(OH)-C(=O)-heteroaryl, and -CH₂-N(OH)-C(=O)-heteroaryldiyl-R₁';
R₂ is methyl; and R₃ is methyl.

3. The compound according to claim 1 or 2, wherein the R₁' in the aromatic compound is independently selected from the group consisting of -Cl, -F, -Br, -OH, isopropyl, linear or branched alkyl (C≤6), linear or branched alkyl (C≤6) substituted with 1 to 5 halogens, -OH, linear or branched alkyl (C≤6) substituted with 1 to 5 -OH groups, linear or branched alkenyl (C≤6), linear or branched alkenyl (C≤6) substituted with 1 to 5 halogens, linear or branched alkenyl (C≤6) substituted with 1 to 5 -OH groups, linear or branched alkynyl (C≤6), linear or branched alkynyl (C≤6) substituted with 1 to 5 halogens, linear or branched alkynyl (C≤6) substituted with 1 to 5 - OH groups, 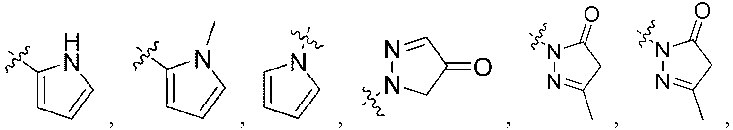 and

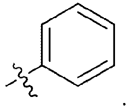

4. The compound according to claim 3, wherein the aryl group is selected from the group consisting of

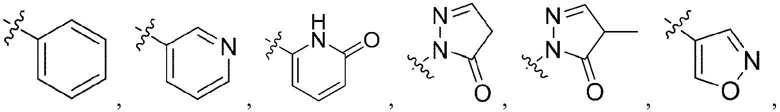

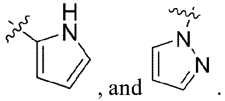

5. The compound according to claim 3, wherein the carbon chain length of each of the substituted alkyl, substituted alkenyl, substituted alkynyl, alkyl, alkenyl and alkynyl is less than or equal to 6, and each is independently linear, branched or cyclic.

6. The compound according to claim 1, wherein the compound is selected from the group consisting of:

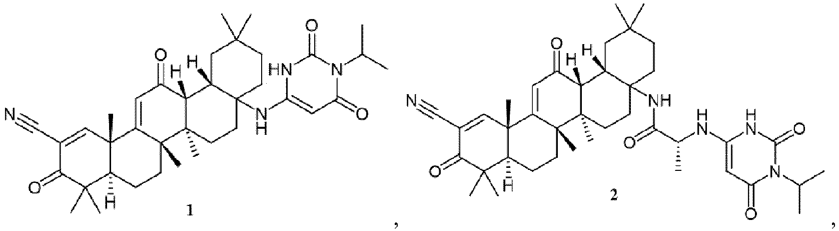

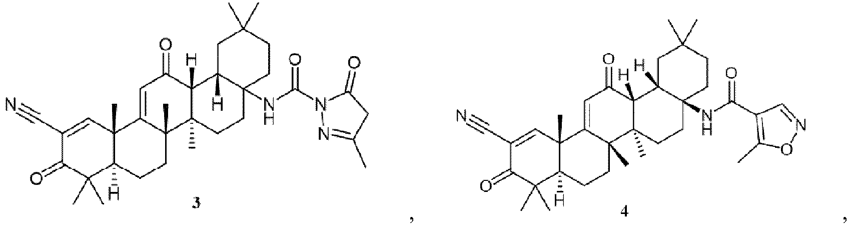

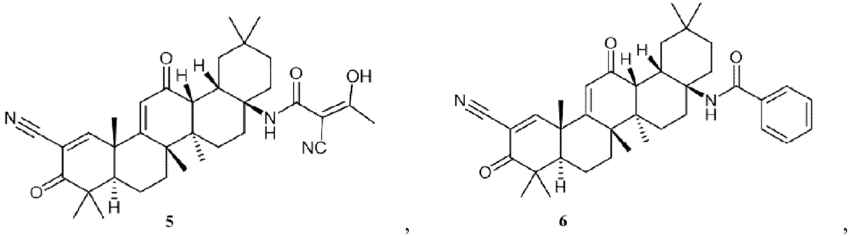

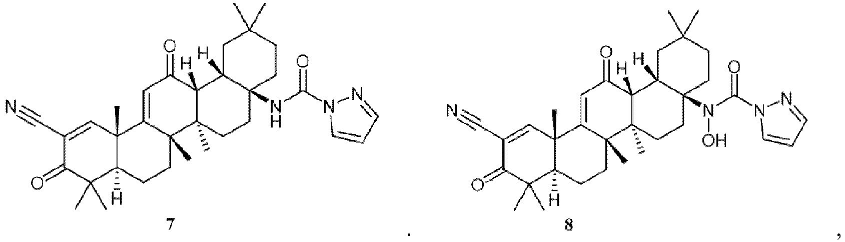

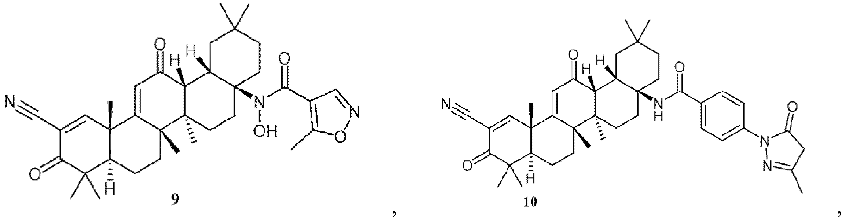

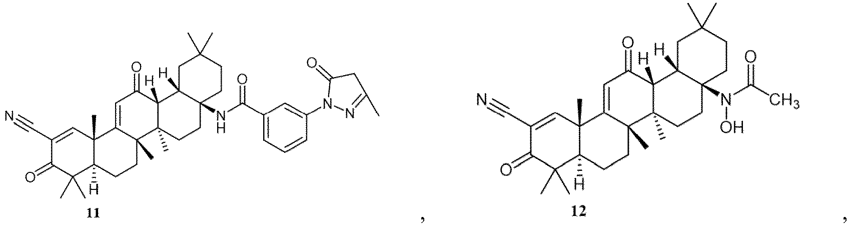

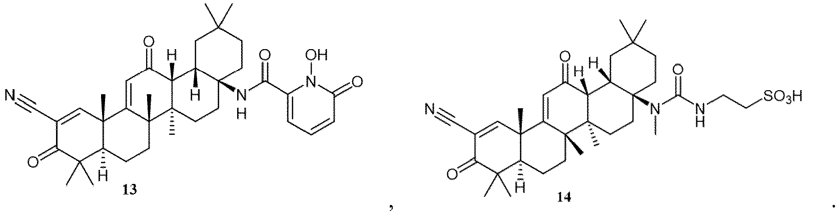

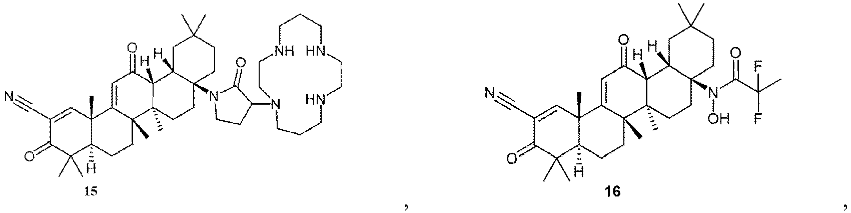

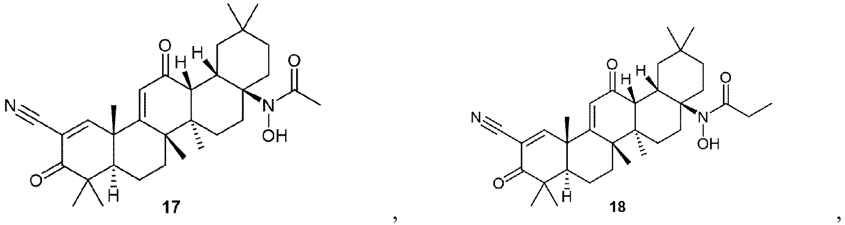

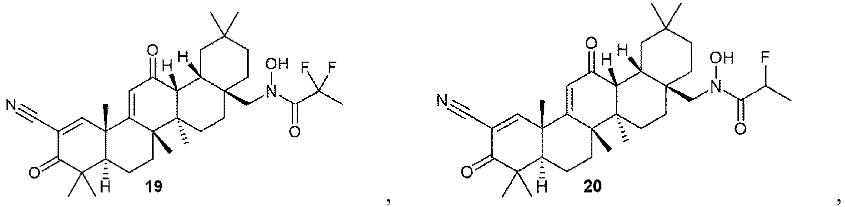

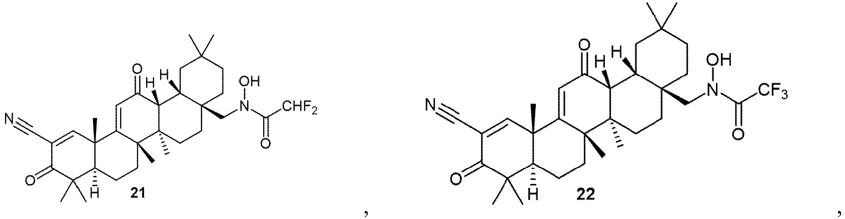

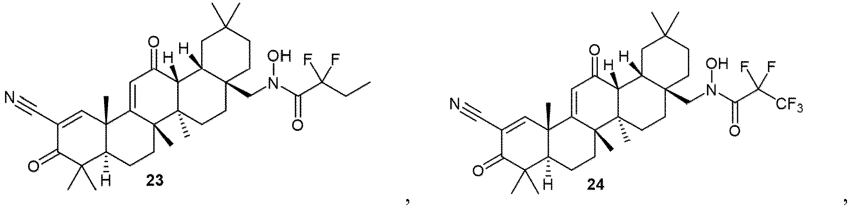

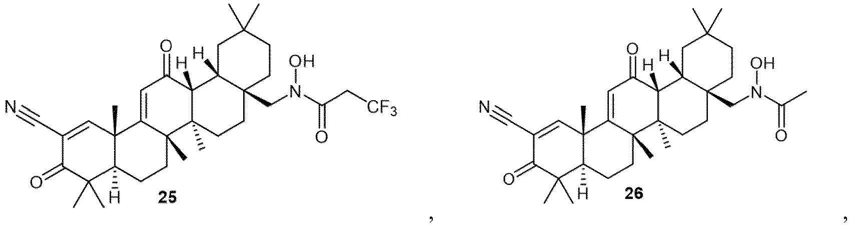

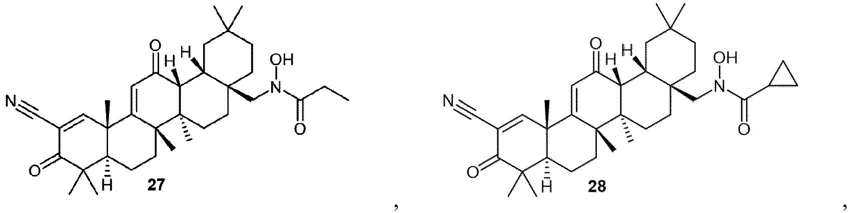

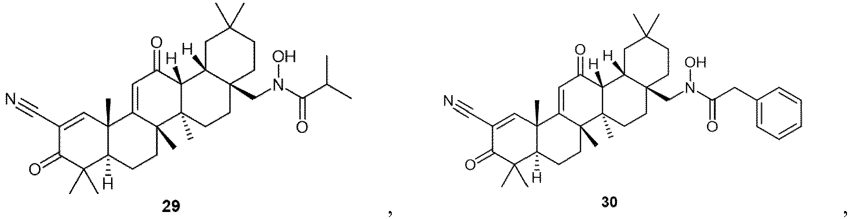

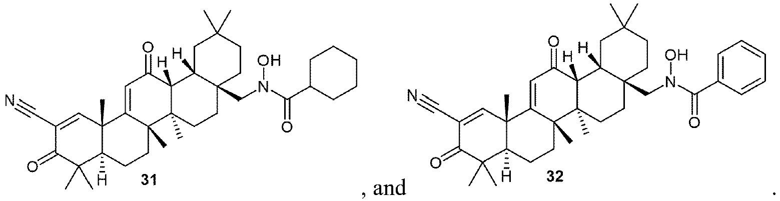

7. Use of the compound according to claim 6 in the preparation of an NRF2-Leap 1 uncoupler.

8. Use of the compound according to claim 6 in preparation of a medicament for preventing and/or treating diseases in a patient, wherein the prepared medicament is used to prevent and/or treat the diseases comprising cerebral small vessel disease, mitochondrial encephalomyopathy, autism spectrum disorder, Rett syndrome, Friedreich ataxia, stroke, hemorrhagic stroke, ischemic stroke, multiple sclerosis, amyotrophic lateral sclerosis, schizophrenia, cognitive impairment in schizophrenia, Parkinson's disease, cognitive impairment in Parkinson's disease, Alzheimer's disease, vascular dementia, epilepsy, Huntington'schorea, heart failure, myocardial infarction, renal failure, and renal ischemia.

9. The use according to claim 8, wherein the compound is used in preparation of a medicament for preventing and/or treating stroke, multiple sclerosis, and amyotrophic lateral sclerosis.
